(19) Europäisches Patentamt
European Patent Office
Office européen des brevets

(11) EP 4 455 284 A1

(12) EUROPEAN PATENT APPLICATION
published in accordance with Art. 153(4) EPC

(43) Date of publication:
**30.10.2024 Bulletin 2024/44**

(21) Application number: **22909920.5**

(22) Date of filing: **19.12.2022**

(51) International Patent Classification (IPC):
***C12N 15/113*** *(2010.01)* ***A61K 31/713*** *(2006.01)*

(52) Cooperative Patent Classification (CPC):
**A61K 31/713; C12N 15/113**

(86) International application number:
**PCT/CN2022/139942**

(87) International publication number:
**WO 2023/116607 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.12.2021 CN 202111575800**

(71) Applicant: **Suzhou Ribo Life Science Co., Ltd.**
**Kunshan, Jiangsu 215300 (CN)**

(72) Inventors:
- **LIANG, Zicai**
  **Kunshan, Jiangsu 215300 (CN)**
- **ZHANG, Hongyan**
  **Kunshan, Jiangsu 215300 (CN)**
- **GAO, Shan**
  **Kunshan, Jiangsu 215300 (CN)**
- **DING, Xuefeng**
  **Kunshan, Jiangsu 215300 (CN)**

(74) Representative: **Kraus & Lederer PartGmbB**
**Thomas-Wimmer-Ring 15**
**80539 München (DE)**

Remarks:
The complete document including Reference Table(s) and the Sequence Listing(s) can be downloaded from the EPO website

# (54) NUCLEIC ACID, COMPOSITION AND CONJUGATE CONTAINING NUCLEIC ACID, PREPARATION METHOD THEREFOR AND USE THEREOF

(57) Provided are siRNA for inhibiting an expression level of a *RPTOR* gene and regulating and controlling the activity of mTORC1, and a pharmaceutical composition and conjugate containing the siRNA. The siRNA contains a sense strand and an antisense strand; each nucleotide in the siRNA is independently a modified or unmodified nucleotide, wherein the sense strand contains a segment of nucleotide sequence I, the length of the nucleotide sequence I is equal to the length of a nucleotide sequence as shown in SEQ ID NO: 1, and a difference therebetween is not more than three nucleotides; the antisense strand contains a nucleotide sequence II, the length of the nucleotide sequence II is equal to the length of a nucleotide sequence as shown in SEQ ID NO: 2, and a difference therebetween is not more than three nucleotide. The provided siRNA, pharmaceutical composition and conjugate can be used as a mTORC1 inhibitor, and can effectively treat related diseases caused by mTORC 1 activation and can be used as an autophagy inducer to treat autophagy-related diseases or symptoms.

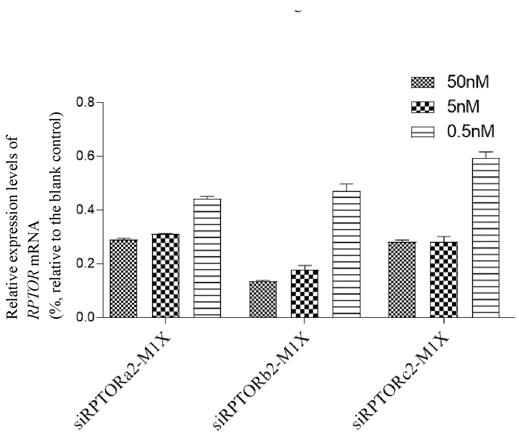

Figure 2

EP 4 455 284 A1

## Description

## TECHNICAL FIELD

The present disclosure relates to a nucleic acid capable of inhibiting mTORC1 activity, and a pharmaceutical composition and a siRNA conjugate comprising the nucleic acid. The present disclosure also relates to a method for preparing these nucleic acids, pharmaceutical compositions and siRNA conjugates, and use thereof.

## BACKGROUND OF THE INVENTION

Neurodegenerative diseases are the gradual loss of neuronal structure and function, including the death of neurons and the imbalance of glial cells, causing cognitive disorders such as dementia. Such diseases include Parkinson's disease (PD), Alzheimer's disease (AD), Frontotemporal dementia, Huntington's disease (HD), Amyotrophic lateral sclerosis (ALS, also known as Lou Gehrig's disease) and Spinal muscular atrophy (SMA). AD and PD mainly occur in middle-aged and elderly people. As the population is aging, the incidences of AD and PD are increasing. HD, ALS and SMA may occur in people of all ages.

At present, neurodegenerative diseases are treated predominantly by using Western medicine; however, this medicine treatment regimen requires long-term medication and has obvious side effects, such as, easily affecting central nervous system, digestive system, or respiratory systems, or even resulting in endocrine disturbance, mood swings, etc.

The mammalian target of rapamycin (mTOR) signaling pathway is a signaling pathway that regulates protein synthesis, cell growth, proliferation and the like. There are two different complexes mTORC1 and mTORC2 in cells. The mTORC1 is a complex consisted of *RPTOR,* Ras homolog enriched in brain (RHEB), DEP domain-containing mTOR-interacting protein (DEPTOR), mammalian lethal with SEC13 protein 8 (mLST8), and Proline rich AKT substrate of 40kDa (PRAS40), and the complex is a critical negative regulator of autophagy modulation. Previous studies have shown that the activation of mTORC1 and inhibition of mitophagy in Alzheimer's Disease (AD) patients and animal models are key factors that cause senile plaques, neurofibrillary tangles and cognitive decline in AD patients. Literatures have reported that inhibiting *RPTOR* can inhibit mTORC1 activity, thereby promoting cellular autophagy and mitophagy to decrease misfolded proteins in the brains of patients with neurodegenerative diseases.

There is a significant need in the art to develop new drugs that can regulate the expression levels of *RPTOR* gene and control mTORC1 activity, thereby treating diseases or symptoms associated with mTORC1 activation and autophagy dysfunction, especially neurodegenerative diseases.

## SUMMARY OF THE INVENTION

Surprisingly, the inventors of the present disclosure have found that the following siRNAs and their modified sequences of the present disclosure can specifically inhibit the expression of *RPTOR* gene in cells, and the pharmaceutical compositions or siRNA conjugates comprising such siRNAs can effectively deliver the siRNAs of the present disclosure to the target tissues and/or cells, thereby showing high drug-developing potential in the treatment or prevention of neurodegenerative diseases, especially Alzheimer's disease.

In one aspect, the present disclosure provides a siRNA capable of inhibiting the expression of *RPTOR* gene, comprising a sense strand and an antisense strand, each nucleotide in the siRNA being independently a modified or unmodified nucleotide, wherein the sense strand comprises a nucleotide sequence I, and the antisense strand comprises a nucleotide sequence II; the nucleotide sequence I and the nucleotide sequence II are at least partly reverse complementary to form a double-stranded region; wherein the nucleotide sequence I and the nucleotide sequence II are selected from a group of the sequences as shown in any of i) to iii):

i) the nucleotide sequence I has an equal length to the nucleotide sequence shown in SEQ ID NO: 1 with no more than 3 nucleotide differences; and the nucleotide sequence II has an equal length to the nucleotide sequence shown in SEQ ID NO: 2 with no more than 3 nucleotide differences:

5'-CUGCCAUGGAGUAUCUGA$Z_{a1}$-3' (SEQ ID NO: 1);
5'-$Z_{a2}$UCAGAUACUCCAUGGCAG-3' (SEQ ID NO: 2),

wherein $Z_{a1}$ is A and $Z_{a2}$ is U; the nucleotide sequence I comprises a nucleotide $Z_{a3}$ at the position corresponding to $Z_{a1}$; the nucleotide sequence II comprises a nucleotide $Z_{a4}$ at the position corresponding to $Z_{a2}$; the nucleotide $Z_{a4}$ is the first nucleotide at 5' terminal of the antisense strand;

ii) the nucleotide sequence I has an equal length to the nucleotide sequence shown in SEQ ID NO: 123 with no

more than 3 nucleotide differences; and the nucleotide sequence II has an equal length to the nucleotide sequence shown in SEQ ID NO: 124 with no more than 3 nucleotide differences:

5'-ACAACAUCAAGUACUACG$Z_{b1}$-3' (SEQ ID NO: 123);
5'-$Z_{b2}$CGUAGUACUUGAUGUUGU-3' (SEQ ID NO: 124),

wherein $Z_{b1}$ is A and $Z_{b2}$ is U; the nucleotide sequence I comprises a nucleotide $Z_{b3}$ at the position corresponding to $Z_{b1}$; the nucleotide sequence II comprises a nucleotide $Z_{b4}$ at the position corresponding to $Z_{b2}$; the nucleotide $Z_{b4}$ is the first nucleotide at 5' terminal of the antisense strand; and

iii) the nucleotide sequence I has an equal length to the nucleotide sequence shown in SEQ ID NO: 245 with no more than 3 nucleotide differences; and the nucleotide sequence II has an equal length to the nucleotide sequence shown in SEQ ID NO: 246 with no more than 3 nucleotide differences:

5'-CGACUACUACAUCUCCGU$Z_{c1}$-3' (SEQ ID NO: 245);
5'-$Z_{c2}$ACGGAGAUGUAGUAGUCG-3' (SEQ ID NO: 246),

wherein $Z_{c1}$ is G and $Z_{c2}$ is C; the nucleotide sequence I comprises a nucleotide $Z_{c3}$ at the position corresponding to $Z_{c1}$; the nucleotide sequence II comprises a nucleotide $Z_{c4}$ at the position corresponding to $Z_{c2}$; the nucleotide $Z_{c4}$ is the first nucleotide at 5' terminal of the antisense strand.

In another aspect, the present disclosure provides a pharmaceutical composition, comprising the siRNA of the present disclosure and a pharmaceutically acceptable carrier.

In a further aspect, the present disclosure provides a siRNA conjugate, comprising the siRNA of the present disclosure and a conjugation group conjugated to the siRNA.

In a further aspect, the present disclosure provides use of the siRNA and/or the pharmaceutical composition and/or the siRNA conjugate of the present disclosure in the manufacture of a medicament for treating and/or preventing diseases associated with the regulation of *RPTOR* function.

In a further aspect, the present disclosure provides a method for treating a disease or symptom associated with the regulation of *RPTOR* function, e.g., a neurodegenerative disease or a non-alcoholic steatohepatitis, in particular Alzheimer's disease, wherein the method comprises administering an effective amount of the siRNA and/or the pharmaceutical composition and/or the siRNA conjugate of the present disclosure to a subject in need thereof.

In a further aspect, the present disclosure provides a method for inhibiting the expression of *RPTOR* gene in cells, comprising contacting an effective amount of the siRNA and/or the pharmaceutical composition and/or the siRNA conjugate of the present disclosure with the cells.

Furthermore, the present disclosure also provides a kit, comprising the siRNA, and/or the pharmaceutical composition, and/or the siRNA conjugate of the present disclosure.

## INCORPORATION BY REFERENCE

All publications, patents, and patent applications mentioned in this specification are incorporated herein by reference to the same extent as if each individual publication, patent, or patent application is specifically and individually incorporated herein by reference.

## BENEFICIAL EFFECTS

The siRNAs, the pharmaceutical compositions and the siRNA conjugates of the present disclosure have good stability, higher inhibitory activity against *RPTOR* mRNA, lower off-target effects, and/or could effectively treat and relieve diseases or symptoms associated with the regulation of *RPTOR* function.

For example, the siRNA, the pharmaceutical composition or the siRNA conjugate of the present disclosure exhibits excellent inhibitory activity against the expression of a target gene in *in vitro* cell experiments. For example, in HepG2 human hepatoma cells *in vitro,* the siRNA of the present disclosure shows an inhibition rate of at least 40.5% against *RPTOR* mRNA at a low concentration of 0.5 nM; the siRNA of the present disclosure shows an inhibition rate of at least 68.3% against *RPTOR* mRNA at a concentration of 5 nM; and the siRNA of the present disclosure shows an inhibition rate of at least 71.1% or even up to 86.8% against *RPTOR* mRNA at a concentration of 50 nM, exhibiting excellent inhibitory effect against the expression of *RPTOR* gene. Moreover, all the siRNAs of the present disclosure of different lengths and different modifications have higher inhibition rates.

For another example, the siRNA, the pharmaceutical composition or the siRNA conjugate of the present disclosure has good stability and inhibitory activity against the target mRNA *in vivo.* For example, the siRNA conjugate of the present disclosure of different modifications shows an inhibition rate of 51.3% or 52.9% against *RPTOR* mRNA expression at a concentration of 3 mg/kg in C57BL/6j mice, exhibiting excellent inhibitory activity against *RPTOR* mRNA.

Therefore, the siRNA, the pharmaceutical composition and the siRNA conjugate of the present disclosure could inhibit the expression of *RPTOR* gene, effectively treat diseases caused by mTORC1 activation and diseases associated with cellular autophagy dysfunction, and thus show a promising prospect of application.

Additional features and advantages of the present disclosure will be illustrated in detail in the following part "DETAILED DESCRIPTION OF THE INVENTION".

## BRIEF DESCRIPTION OF THE DRAWINGS

Figure 1 is a histogram showing relative expression levels of *RPTOR* mRNA in HepG2 human hepatoma cells *in vitro* after transfection with the siRNA of the present disclosure at a concentration of 50 nM and with the comparative siRNA.

Figure 2 is a histogram showing relative expression levels of *RPTOR* mRNA in HepG2 human hepatoma cells *in vitro* after transfection with the siRNAs of the present disclosure at different concentrations.

## DETAILED DESCRIPTION OF THE INVENTION

The specific embodiments of the present disclosure are described in detail as below. It should be understood that the specific embodiments described herein are only for the purpose of illustration and explanation of the present disclosure and are not intended to limit the present disclosure in any manner.

### Definitions

In the context of the present disclosure, *RPTOR* mRNA refers to the sequence as shown in Genbank Accession No. NM_020761.3. Furthermore, unless otherwise specified, as used in the present disclosure, the term "target gene" refers to a gene that encodes the above *RPTOR* mRNA, and the term "target mRNA" refers to the above *RPTOR* mRNA.

In the context of the present disclosure, unless otherwise specified, C, G, U, and A represent the base composition of a nucleotide; m represents that the nucleotide adjacent to the left side of the letter m is a methoxy modified nucleotide; f represents that the nucleotide adjacent to the left side of the letter f is a fluoro modified nucleotide; s represents the two nucleotides adjacent to both sides of the letter s are linked by a phosphorothioate linkage; P1 represents that the nucleotide adjacent to the right side of P1 is a 5'-phosphate nucleotide or a 5'-phosphate analogue modified nucleotide; in some embodiments, P1 represents a specifically modified VP, Ps or P, wherein the letter combination VP represents that the nucleotide adjacent to the right side of the letter combination VP is a vinylphosphonate (5'-(E)-vinylphosphonate, E-VP) modified nucleotide; the letter combination Ps represents that the nucleotide adjacent to the right side of the letter combination Ps is a phosphorothioate modified nucleotide; and P represents that the nucleotide adjacent to the right side of the letter P is a 5'-phosphate nucleotide.

In the context of the present disclosure, the "fluoro modified nucleotide" refers to a nucleotide formed by substituting 2'-hydroxy of the ribose group of the nucleotide with a fluorine atom, and the "non-fluoro modified nucleotide" refers to a nucleotide formed by substituting 2'-hydroxy of the ribose group of the nucleotide with a non-fluoro group, or a nucleotide analogue. The "nucleotide analogue" refers to a group that can replace a nucleotide in the nucleic acid, while structurally differs from an adenine ribonucleotide, a guanine ribonucleotide, a cytosine ribonucleotide, a uracil ribonucleotide, or thymine deoxyribonucleotide. For example, the nucleotide analogue is an isonucleotide, a bridged nucleic acid (BNA) nucleotide or an acyclic nucleotide. The "methoxy modified nucleotide" refers to a nucleotide formed by substituting 2'-hydroxy of the ribose group with a methoxy group.

In the context of the present disclosure, the expressions "complementary" and "reverse complementary" can be interchangeably used, and have a well-known meaning in the art; namely, the bases in one strand are each complementarily paired with those in the other strand of a double-stranded nucleic acid molecule. In DNA, a purine base adenine (A) is always paired with a pyrimidine base thymine (T) (or uracil (U) in RNAs); and a purine base guanine (G) is always paired with a pyrimidine base cytosine (C). Each base pair comprises a purine and a pyrimidine. While adenines in one strand are always paired with thymines (or uracils) in another strand, and guanines are always paired with cytosines, these two strands are considered as being complementary with each other; and the sequence of a strand may be deduced from the sequence of its complementary strand. Correspondingly, a "mispairing" means that in a double-stranded nucleic acid, the bases at corresponding positions are not presented in a manner of being complementarily

paired.

In the context of the present disclosure, unless otherwise specified, "basically reverse complementary" means that there are no more than 3 base mispairings between two nucleotide sequences. "Substantially reverse complementary" means that there is no more than 1 base mispairing between two nucleotide sequences. "Completely reverse complementary" means that there is no base mispairing between two nucleotide sequences.

In the context of the present disclosure, when a nucleotide sequence has a "nucleotide difference" from another nucleotide sequence, the bases of the nucleotides at the same position therebetween are changed. For example, if a nucleotide base in the second sequence is A and the nucleotide base at the same position in the first sequence is U, C, G or T, these two nucleotide sequences are considered as having a nucleotide difference at this position. In some embodiments, if a nucleotide at a position is replaced with an abasic nucleotide or a nucleotide analogue, it is also considered that there is a nucleotide difference at the position. The "abasic nucleotide" refers to a monomeric compound formed by substituting the nucleic acid base in a nucleotide with other groups or a hydrogen atom, wherein said other groups include, but are not limited to, substituted or unsubstituted aryl or heteroaryl groups.

In the context of the present disclosure, particularly in the description of the method for preparing the siRNA, the pharmaceutical composition, or the siRNA conjugate of the present disclosure, unless otherwise specified, the "nucleoside monomer" refers to, according to the type and sequence of the nucleotides in the siRNA or siRNA conjugate to be prepared, unmodified or modified nucleoside phosphoramidite monomer (unmodified or modified RNA phosphoramidites; sometimes RNA phosphoramidites are referred to as nucleoside phosphoramidites) used in a solid phase phosphoramidite synthesis. Solid phase phosphoramidite synthesis is a well-known method for RNA synthesis to those skilled in the art. Nucleoside monomers used in the present disclosure are all commercially available.

In the context of the present disclosure, unless otherwise specified, "conjugation" means that two or more chemical moieties each with specific function are linked to each other via a covalent linkage. Correspondingly, a "conjugate" refers to a compound formed by covalent linkage of individual chemical moieties. Further, a "siRNA conjugate" represents a compound formed by covalently attaching a siRNA and one or more chemical moieties each with specific functions. According to the context of the present disclosure, the siRNA conjugate should be understood as the generic term of many siRNA conjugates, or a siRNA conjugate shown by a chemical formula. In the context of the present disclosure, a "conjugation molecule" should be interpreted as specific compounds capable of being conjugated to a siRNA via reactions, thereby finally forming the siRNA conjugate of the present disclosure.

Those skilled in the art would understand, with respect to any group comprising one or more substituents, that such groups are not intended to introduce any substitution or substitution patterns that are sterically impractical, synthetically infeasible and/or inherently unstable.

As used herein, "alkyl" refers to straight chain and branched chain having the indicated number of carbon atoms, usually 1 to 20 carbon atoms, for example 1 to 10 carbon atoms, such as 1 to 8 or 1 to 6 carbon atoms. For example, $C_1$-$C_6$ alkyl encompasses both straight and branched chain alkyl of 1 to 6 carbon atoms. When an alkyl residue having a specific number of carbon atoms is mentioned, all branched and straight chain forms having that number of carbon atoms are intended to be encompassed; thus, for example, "butyl" is meant to encompass n-butyl, sec-butyl, isobutyl, and t-butyl; "propyl" includes n-propyl and isopropyl. Alkylene is a subset of alkyl, referring to a bivalent group that is the same as alkyl, but has two attachment points.

As used herein, "alkenyl" refers to an unsaturated branched or straight-chain alkyl group having at least one carbon-carbon double bond which is obtained by removing one hydrogen molecule from two adjacent carbon atoms of the parent alkyl. The group may be in either cis or trans configuration of the double bond. Typical alkenyl groups include, but not limited to, ethenyl; propenyls such as prop-1-en-1-yl, prop-1-en-2-yl, prop-2-en-1-yl (allyl), prop-2-en-2-yl; butenyls such as but-1-en-1-yl, but-1-en-2-yl, 2-methyl-prop-1-en-1-yl, but-2-en-1-yl, but-2-en-1-yl, but-2-en-2-yl, buta-1, 3-dien-1-yl, buta-1, 3-dien-2-yl; and the like. In certain embodiments, an alkenyl group has 2 to 20 carbon atoms, and in other embodiments, 2 to 10, 2 to 8, or 2 to 6 carbon atoms. Alkenylene is a subset of alkenyl, referring to the same residues as alkenyl, but having two attachment positions.

As used herein, "alkoxy" refers to an alkyl group of the indicated number of carbon atoms attached through an oxygen bridge, such as, for example, methoxy, ethoxy, propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy, 3-methylpentyloxy, and the like. Alkoxy groups will usually have 1 to 10, 1 to 8, 1 to 6, or 1 to 4 carbon atoms attached through oxygen bridge.

Various hydroxyl protecting groups can be used in the present disclosure. In general, protecting groups render chemical functional groups inert to specific reaction conditions, and can be attached to and removed from such functional groups in a molecule without substantially damaging the remainder of the molecule. Representative hydroxylprotecting groups are disclosed in Beaucage, et al., Tetrahedron 1992, 48, 2223-2311, and also in Greene and Wuts, Protective Groups in Organic Synthesis, Chapter 2, 2d ed, John Wiley & Sons, New York, 1991, each of which is hereby incorporated by reference in their entirety. In some embodiments, the protecting group is stable under basic conditions but can be removed under acidic conditions. In some embodiments, non-exclusive examples of hydroxyl protecting groups used herein include dimethoxytrityl (DMT), monomethoxytrityl, 9-phenylxanthen-9-yl (Pixyl), and 9-(p-methoxyphenyl)xanthen-9-yl (Mox). In some embodiments, non-exclusive examples of hydroxyl protecting groups used herein comprise Tr (trityl), MMTr (4-methoxytrityl), DMTr (4, 4'-dimethoxytrityl), and TMTr (4, 4', 4"-trimethoxytrityl).

The term "subject", as used herein, refers to any animal, e.g., mammal or marsupial. The subject of the present disclosure includes, but is not limited to, human, non-human primate (e.g., rhesus or other kinds of macaque), mouse, pig, horse, donkey, cow, rabbit, sheep, rat and any kind of poultry.

As used herein, "treatment" refers to an approach for obtaining beneficial or desirable results, including but not limited to therapeutic benefit. "Therapeutic benefit" means eradication or improvement of potential disorder to be treated. Also, a therapeutic benefit is achieved by eradicating or ameliorating one or more of physiological symptoms associated with the potential disorder such that an improvement is observed in the subject, notwithstanding that the subject may still be afflicted with the potential disorder.

As used herein, "prevention" refers to an approach for obtaining beneficial or desirable results, including but not limited to prophylactic benefit. In order to obtain "prophylactic benefit", the siRNA, the pharmaceutical composition or the siRNA conjugate may be administered to a subject at risk of developing a particular disease, or to a subject at a risk of developing a particular disease, even though the diagnosis of this disease may not have been made yet.

## The siRNA of the present disclosure

In one aspect, the present disclosure provides a siRNA capable of inhibiting the expression of *RPTOR* gene.

The siRNA of the present disclosure comprises nucleotide groups as basic structural units. It is well known to those skilled in the art that the nucleotide group comprises a phosphate group, a ribose group and a base. Detailed illustrations of these groups are omitted herein.

The siRNA of the present disclosure comprises a sense strand and an antisense strand. The sense strand and antisense strand have the same or different lengths. The sense strand has a length of 19-23 nucleotides, and the antisense strand has a length of 19-26 nucleotides. As such, the length ratio of the sense strand to the antisense strand in the siRNA of the present disclosure may be 19/19, 19/20, 19/21, 19/22, 19/23, 19/24, 19/25, 19/26, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25, or 23/26. In some embodiments, the length ratio of the sense strand to the antisense strand in the siRNA of the present disclosure is 19/21, 21/21, 21/23, or 23/25.

According to the present disclosure, the siRNA comprises a sense strand and an antisense strand, each nucleotide in the siRNA being independently a modified or unmodified nucleotide, wherein the sense strand comprises a nucleotide sequence I, and the antisense strand comprises a nucleotide sequence II; the nucleotide sequence I and the nucleotide sequence II are at least partly reverse complementary to form a double-stranded region.

In some embodiments, the siRNA of the present disclosure may be the following first siRNA, second siRNA or third siRNA, each of which is described respectively below.

## First siRNA

In some embodiments, the siRNA of the present disclosure is the first siRNA, wherein the nucleotide sequence I has an equal length to the nucleotide sequence shown in SEQ ID NO: 1 with no more than 3 nucleotide differences; and the nucleotide sequence II has an equal length to the nucleotide sequence shown in SEQ ID NO: 2 with no more than 3 nucleotide differences:

5'-CUGCCAUGGAGUAUCUGA$Z_{a1}$-3' (SEQ ID NO: 1);
5'-$Z_{a2}$UCAGAUACUCCAUGGCAG-3' (SEQ ID NO: 2),

wherein $Z_{a1}$ is A, and $Z_{a2}$ is U; the nucleotide sequence I comprises a nucleotide $Z_{a3}$ at the position corresponding to $Z_{a1}$; the nucleotide sequence II comprises a nucleotide $Z_{a4}$ at the position corresponding to $Z_{a2}$; the nucleotide $Z_{a4}$ is the first nucleotide at 5' terminal of the antisense strand.

In this context, the term "position corresponding" means being at the same position in the nucleotide sequences when counting from the same terminal of the nucleotide sequences. For example, the first nucleotide at 3' terminal of the nucleotide sequence I is a nucleotide at the position corresponding to the first nucleotide at 3' terminal of SEQ ID NO: 1.

In some embodiments, the sense strand comprises only the nucleotide sequence I, and the antisense strand comprises only the nucleotide sequence II. In some embodiments, the nucleotide sequence I and the nucleotide sequence shown in SEQ ID NO: 1 have no more than 1 nucleotide difference, and/or the nucleotide sequence II and the nucleotide sequence shown in SEQ ID NO: 2 have no more than 1 nucleotide difference.

In some embodiments, the nucleotide difference between the nucleotide sequence II and the nucleotide sequence shown in SEQ ID NO: 2 includes a difference at the position $Z_{a4}$, where $Z_{a4}$ is selected from A, C or G. In some embodiments, $Z_{a3}$ is a nucleotide complementary to $Z_{a4}$. The siRNAs with the above-mentioned nucleotide differences

have higher inhibitory ability against the target mRNA, and these siRNAs comprising the nucleotide differences are within the protection scope of the present disclosure.

In some embodiments, the nucleotide sequence I and the nucleotide sequence II are basically reverse complementary, substantially reverse complementary, or completely reverse complementary to each other; the "basically reverse complementary" means that there are no more than 3 base mispairings between two nucleotide sequences; the "substantially reverse complementary" means that there is no more than 1 base mispairing between two nucleotide sequences; and the "completely reverse complementary" means that there is no mispairing between two nucleotide sequences.

In some embodiments, the sense strand and the antisense strand have the same or different length, wherein the sense strand has a length of 19 to 23 nucleotides, and the antisense strand has a length of 19 to 26 nucleotides; and the nucleotide sequence I is the nucleotide sequence shown in SEQ ID NO: 3, and the nucleotide sequence II is the nucleotide sequence shown in SEQ ID NO: 4:

5'-CUGCCAUGGAGUAUCUGA$Z_{a3}$-3' (SEQ ID NO: 3);
5'-$Z_{a4}$UCAGAUACUCCAUGGCAG-3' (SEQ ID NO:4),

wherein $Z_{a3}$ is selected from A, U, G or C, and $Z_{a4}$ is a nucleotide complementary to $Z_{a3}$; in some embodiments, $Z_{a3}$ is A, and $Z_{a4}$ is U.

In some embodiments, the sense strand further comprises a nucleotide sequence III, the antisense strand further comprises a nucleotide sequence IV, and the nucleotide sequence III and the nucleotide sequence IV independently of one another have a length of 1 to 4 nucleotides; the nucleotide sequence III and the nucleotide sequence IV have an equal length, and are substantially reverse complementary or completely reverse complementary to each other; the nucleotide sequence III is linked to 5' terminal of the nucleotide sequence I, the nucleotide sequence IV is linked to 3' terminal of the nucleotide sequence II.

In some embodiments, the nucleotide sequence IV and a second nucleotide sequence are substantially reverse complementary or completely reverse complementary to each other; the second nucleotide sequence refers to a nucleotide sequence in the target mRNA that is adjacent to 5' terminal of the nucleotide sequence shown in SEQ ID NO: 1 and has an equal length to the nucleotide sequence IV In some embodiments, in the direction from 5' terminal to 3' terminal, the nucleotide sequence III and the nucleotide sequence IV both have a length of 1 nucleotide; the base of the nucleotide sequence III is A, and the base of the nucleotide sequence IV is U; in this case, the length ratio of the sense strand to the antisense strand is 20/20. Alternatively, the nucleotide sequence III and the nucleotide sequence IV both have a length of 2 nucleotides; in the direction from 5' terminal to 3' terminal, the base composition of the nucleotide sequence III is CA, and the base composition of the nucleotide sequence IV is UG; in this case, the length ratio of the sense strand to the antisense strand is 21/21. Alternatively, the nucleotide sequence III and the nucleotide sequence IV both have a length of 3 nucleotides; in the direction from 5' terminal to 3' terminal, the base composition of the nucleotide sequence III is UCA, and the base composition of the nucleotide sequence IV is UGA; in this case, the length ratio of the sense strand to the antisense strand is 22/22. Alternatively, the nucleotide sequence III and the nucleotide sequence IV both have a length of 4 nucleotides; in the direction from 5' terminal to 3' terminal, the base composition of the nucleotide sequence III is GUCA, and the base composition of the nucleotide sequence IV is UGAC; in this case, the length ratio of the sense strand to the antisense strand is 23/23.

In some embodiments, the nucleotide sequence III and the nucleotide sequence IV are completely reverse complementary to each other. Thus, once the base(s) of the nucleotide sequence III is(are) provided, the base(s) of nucleotide sequence IV is(are) also determined.

Second siRNA

In some embodiments, the siRNA of the present disclosure is the second siRNA, wherein the nucleotide sequence I has an equal length to the nucleotide sequence shown in SEQ ID NO: 123 with no more than 3 nucleotide differences; and the nucleotide sequence II has an equal length to the nucleotide sequence shown in SEQ ID NO: 124 with no more than 3 nucleotide differences:

5'-ACAACAUCAAGUACUACG$Z_{b1}$-3' (SEQ ID NO: 123);
5'-$Z_{b2}$CGUAGUACUUGAUGUUGU-3' (SEQ ID NO: 124),

wherein $Z_{b1}$ is A, and $Z_{b2}$ is U; the nucleotide sequence I comprises a nucleotide $Z_{b3}$ at the position corresponding to $Z_{b1}$; the nucleotide sequence II comprises a nucleotide $Z_{b4}$ at the position corresponding to $Z_{b2}$; the nucleotide $Z_{b4}$ is the first nucleotide at 5' terminal of the antisense strand.

In some embodiments, the sense strand comprises only the nucleotide sequence I, and the antisense strand

comprises only the nucleotide sequence II. In some embodiments, the nucleotide sequence I and the nucleotide sequence shown in SEQ ID NO: 123 have no more than 1 nucleotide difference, and/or the nucleotide sequence II and the nucleotide sequence shown in SEQ ID NO: 124 have no more than 1 nucleotide difference.

In some embodiments, the nucleotide difference between the nucleotide sequence II and the nucleotide sequence shown in SEQ ID NO: 124 includes a difference at the position $Z_{b4}$, where $Z_{b4}$ is selected from A, C or G. In some embodiments, $Z_{b3}$ is a nucleotide complementary to $Z_{b4}$. The siRNAs with the above-mentioned nucleotide differences have higher inhibitory ability against the target mRNA, and these siRNAs comprising the nucleotide differences are within the protection scope of the present disclosure.

In some embodiments, the nucleotide sequence I and the nucleotide sequence II are basically reverse complementary, substantially reverse complementary, or completely reverse complementary to each other; the "basically reverse complementary" means that there are no more than 3 base mispairings between two nucleotide sequences; the "substantially reverse complementary" means that there is no more than 1 base mispairing between two nucleotide sequences; and the "completely reverse complementary" means that there is no mispairing between two nucleotide sequences.

In some embodiments, the nucleotide sequence I is the nucleotide sequence shown in SEQ ID NO: 125, and the nucleotide sequence II is the nucleotide sequence shown in SEQ ID NO: 126:

5'-ACAACAUCAAGUACUACGZ$_{b3}$-3' (SEQ ID NO: 125);
5'-Z$_{b4}$CGUAGUACUUGAUGUUGU-3' (SEQ ID NO: 126),

wherein $Z_{b3}$ is selected from A, U, G or C, and $Z_{b4}$ is a nucleotide complementary to $Z_{b3}$; in some embodiments, $Z_{b3}$ is A, and $Z_{b4}$ is U.

In some embodiments, the sense strand further comprises a nucleotide sequence III, the antisense strand further comprises a nucleotide sequence IV, and the nucleotide sequence III and the nucleotide sequence IV independently of one another have a length of 1 to 4 nucleotides; the nucleotide sequence III and the nucleotide sequence IV have an equal length, and are substantially reverse complementary or completely reverse complementary to each other; the nucleotide sequence III is linked to 5' terminal of the nucleotide sequence I, the nucleotide sequence IV is linked to 3' terminal of the nucleotide sequence II.

In some embodiments, the nucleotide sequence IV and a second nucleotide sequence are substantially reverse complementary or completely reverse complementary to each other; the second nucleotide sequence refers to a nucleotide sequence in the target mRNA that is adjacent to 5' terminal of the nucleotide sequence shown in SEQ ID NO: 123 and has an equal length to the nucleotide sequence IV. In some embodiments, in the direction from 5' terminal to 3' terminal, the nucleotide sequence III and the nucleotide sequence IV both have a length of 1 nucleotide; the base of the nucleotide sequence III is A, and the base of the nucleotide sequence IV is U; in this case, the length ratio of the sense strand to the antisense strand is 20/20. Alternatively, the nucleotide sequence III and the nucleotide sequence IV both have a length of 2 nucleotides; in the direction from 5' terminal to 3' terminal, the base composition of the nucleotide sequence III is CA, and the base composition of the nucleotide sequence IV is UG; in this case, the length ratio of the sense strand to the antisense strand is 21/21. Alternatively, the nucleotide sequence III and the nucleotide sequence IV both have a length of 3 nucleotides; in the direction from 5' terminal to 3' terminal, the base composition of the nucleotide sequence III is UCA, and the base composition of the nucleotide sequence IV is UGA; in this case, the length ratio of the sense strand to the antisense strand is 22/22. Alternatively, the nucleotide sequence III and the nucleotide sequence IV both have a length of 4 nucleotides; in the direction from 5' terminal to 3' terminal, the base composition of the nucleotide sequence III is AUCA, and the base composition of the nucleotide sequence IV is UGAU; in this case, the length ratio of the sense strand to the antisense strand is 23/23.

In some embodiments, the nucleotide sequence III and the nucleotide sequence IV are completely reverse complementary to each other. Thus, once the base(s) of the nucleotide sequence III is(are) provided, the base(s) of nucleotide sequence IV is(are) also determined.

Third siRNA

In some embodiments, the siRNA of the present disclosure is the third siRNA, wherein the nucleotide sequence I has an equal length to the nucleotide sequence shown in SEQ ID NO: 245 with no more than 3 nucleotide differences; and the nucleotide sequence II has an equal length to the nucleotide sequence shown in SEQ ID NO: 246 with no more than 3 nucleotide differences:

5'-CGACUACUACAUCUCCGUZ$_{c1}$-3' (SEQ ID NO: 245);
5'-Z$_{c2}$ACGGAGAUGUAGUAGUCG-3' (SEQ ID NO: 246),

wherein $Z_{c1}$ is G and $Z_{c2}$ is C; and the nucleotide sequence I comprises a nucleotide $Z_{c3}$ at the position corresponding to $Z_{c1}$; the nucleotide sequence II comprises a nucleotide $Z_{c4}$ at the position corresponding to $Z_{c2}$; the nucleotide $Z_{c4}$ is the first nucleotide at 5' terminal of the antisense strand.

In some embodiments, the sense strand comprises only the nucleotide sequence I, and the antisense strand comprises only the nucleotide sequence II. In some embodiments, the nucleotide sequence I and the nucleotide sequence as shown by SEQ ID NO: 245 have no more than 1 nucleotide difference, and/or the nucleotide sequence II and the nucleotide sequence as shown by SEQ ID NO: 246 have no more than 1 nucleotide difference.

In some embodiments, the nucleotide difference between the nucleotide sequence II and the nucleotide sequence shown in SEQ ID NO: 246 includes a difference at the position $Z_{c4}$, where $Z_{c4}$ is selected from A, C or G. In some embodiments, $Z_{c3}$ is a nucleotide complementary to $Z_{c4}$. The siRNAs with the above-mentioned nucleotide differences have higher inhibitory ability against the target mRNA, and these siRNAs comprising the nucleotide differences are within the protection scope of the present disclosure.

In some embodiments, the nucleotide sequence I and the nucleotide sequence II are basically reverse complementary, substantially reverse complementary, or completely reverse complementary to each other; the "basically reverse complementary" means that there are no more than 3 base mispairings between two nucleotide sequences; the "substantially reverse complementary" means that there is no more than 1 base mispairing between two nucleotide sequences; and the "completely reverse complementary" means that there is no mispairing between two nucleotide sequences.

In some embodiments, the nucleotide sequence I is the nucleotide sequence shown in SEQ ID NO: 247, and the nucleotide sequence II is the nucleotide sequence shown in SEQ ID NO: 248:

5'-CGACUACUACAUCUCCGU$Z_{c3}$-3' (SEQ ID NO: 247);
5'-$Z_{c4}$ACGGAGAUGUAGUAGUCG-3' (SEQ ID NO: 248),

wherein $Z_{c3}$ is selected from A, U, G or C, and $Z_{c4}$ is a nucleotide complementary to $Z_{c3}$; in some embodiments, $Z_{c3}$ is G, and $Z_{c4}$ is C.

In some embodiments, the sense strand further comprises a nucleotide sequence III, the antisense strand further comprises a nucleotide sequence IV, and the nucleotide sequence III and the nucleotide sequence IV independently of one another have a length of 1 to 4 nucleotides; the nucleotide sequence III and the nucleotide sequence IV have an equal length, and are substantially reverse complementary or completely reverse complementary to each other; the nucleotide sequence III is linked to 5' terminal of the nucleotide sequence I, the nucleotide sequence IV is linked to 3' terminal of the nucleotide sequence II.

In some embodiments, the nucleotide sequence IV and a second nucleotide sequence are substantially reverse complementary or completely reverse complementary to each other; the second nucleotide sequence refers to a nucleotide sequence in the target mRNA that is adjacent to 5' terminal of the nucleotide sequence shown in SEQ ID NO: 245 and has an equal length to the nucleotide sequence IV In some embodiments, in the direction from 5' terminal to 3' terminal, the nucleotide sequence III and the nucleotide sequence IV both have a length of 1 nucleotide; the base of the nucleotide sequence III is A, and the base of the nucleotide sequence IV is U; in this case, the length ratio of the sense strand to the antisense strand is 20/20 Alternatively, the nucleotide sequence III and the nucleotide sequence IV both have a length of 2 nucleotides; in the direction from 5' terminal to 3' terminal, the base composition of the nucleotide sequence III is AA, and the base composition of the nucleotide sequence IV is UU; in this case, the length ratio of the sense strand to the antisense strand is 21/21. Alternatively, the nucleotide sequence III and the nucleotide sequence IV both have a length of 3 nucleotides; in the direction from 5' terminal to 3' terminal, the base composition of the nucleotide sequence III is CAA, and the base composition of the nucleotide sequence IV is UUG; in this case, the length ratio of the sense strand to the antisense strand is 22/22. Alternatively, the nucleotide sequence III and the nucleotide sequence IV both have a length of 4 nucleotides; in the direction from 5' terminal to 3' terminal, the base composition of the nucleotide sequence III is GCAA, and the base composition of the nucleotide sequence IV is UUGC; in this case, the length ratio of the sense strand to the antisense strand is 23/23.

In some embodiments, the nucleotide sequence III and the nucleotide sequence IV are completely reverse complementary to each other. Thus, once the base(s) of the nucleotide sequence III is(are) provided, the base(s) of nucleotide sequence IV is(are) also determined.

The following description regarding the nucleotide sequence V, the nucleotide sequence VI, the nucleic acid sequence, and the nucleotide modification and the modified sequence of the siRNA is applicable to the above-mentioned first siRNA, second siRNA or third siRNA. That is, unless stated otherwise, the following description of the siRNA should be regarded as the description of each of the first siRNA, second siRNA and third siRNA. For example, if no particular siRNA is specifically indicated, the expression "the siRNA further comprises a nucleotide sequence V" means that "the first siRNA, the second siRNA or the third siRNA further comprises a nucleotide sequence V".

In some embodiments, the sense strand and the antisense strand have different lengths. The antisense strand

further comprises a nucleotide sequence V, which has a length of 1-3 nucleotides and is linked to 3' terminal of the antisense strand, thereby constituting a 3' overhang of the antisense strand.

In some embodiments, the sense strand further comprises a nucleotide sequence VI, which has a length of 1-3 nucleotides and is linked to 3' terminal of the sense strand, thereby constituting a 3' overhang of the sense strand.

In some embodiments, the siRNA of the present disclosure comprises a nucleotide sequence V, but does not comprise a nucleotide sequence VI. As such, the length ratio of the sense strand to the antisense strand in the siRNA of the present disclosure may be 19/20, 19/21, 19/22, 20/21, 20/22, 20/23, 21/22, 21/23, 21/24, 22/23, 22/24, 22/25, 23/24, 23/25, or 23/26. In some embodiments, the siRNA of the present disclosure comprises nucleotide sequences V and VI. In some embodiments, the nucleotide sequence V and the nucleotide sequence VI have the same or different length. As such, the length ratio of the sense strand to the antisense strand in the siRNA of the present disclosure may be (19-26): (19-26). In some embodiments, the nucleotide sequence V and/or the nucleotide sequence VI have a length of 2 nucleotides. As such, the length ratio of the sense strand to the antisense strand in the siRNA of the present disclosure may be 19/21, 21/21, 21/23, 23/23, 23/25, or 25/25.

Each nucleotide in the nucleotide sequence V may be any nucleotide. In order to facilitate the synthesis and to save cost, in some embodiments, the nucleotide sequence V is 2 consecutive thymine deoxyribonucleotides (dTdT) or 2 consecutive uracil ribonucleotides (UU). Alternatively, in order to enhance the affinity between the antisense strand of the siRNA and the target mRNA, the nucleotides at the corresponding positions of the nucleotide sequence V and the target mRNAare complementary to each otherof the target mRNA. Thus, in some embodiments, the length ratio of the sense strand and the antisense strand of the siRNA of the present disclosure is 19/21 or 21/23. In this case, the siRNA of the present disclosure exhibits better mRNA silencing activity.

Each nucleotide in the nucleotide sequence VI may be any nucleotide. In order to facilitate the synthesis and to save synthesis cost, in some embodiments, the nucleotide sequence VI is 2 consecutive thymine deoxyribonucleotides (dTdT) or 2 consecutive uracil ribonucleotides (UU). Alternatively, in order to enhance the affinity between the sense strand and the antisense strand of the siRNA, the nucleotides at the corresponding positions of the nucleotide sequence VI and the target mRNA are identical. Thus, in some embodiments, the siRNA of the present disclosure comprises nucleotide sequences V and VI, and the length ratio of the sense strand and the antisense strand of the siRNA of the present disclosure is 21/21 or 23/23. In this case, the siRNA of the present disclosure exhibits better mRNA silencing activity.

The nucleotides at the corresponding positions of the target mRNA refer to the nucleotides or nucleotide sequences adjacent to 5' terminal of a segment of nucleotide sequence of the target mRNA. This segment of nucleotide sequence of the target mRNA refers to the segment of nucleotide sequence which is substantially reverse complementary or completely reverse complementary to the nucleotide sequence II, or is substantially reverse complementary or completely reverse complementary to the nucleotide sequence consisting of the nucleotide sequence II and the nucleotide sequence IV

In some embodiments, the sense strand of the siRNA comprises the nucleotide sequence shown in SEQ ID NO: 5, and the antisense strand comprises the nucleotide sequence shown in SEQ ID NO: 6:

5'-CUGCCAUGGAGUAUCUGA$Z_{a3}$-3' (SEQ ID NO: 5);

5'-$Z_{a4}$UCAGAUACUCCAUGGCAGUG-3' (SEQ ID NO: 6),

wherein $Z_{a4}$ is the first nucleotide at 5' terminal of the antisense strand; $Z_{a3}$ is selected from A, U, G or C, and $Z_{a4}$ is a nucleotide complementary to $Z_{a3}$; or

the sense strand of the siRNA comprises the nucleotide sequence shown in SEQ ID NO: 7, and the antisense strand of the siRNA comprises the nucleotide sequence shown in SEQ ID NO: 8:

5'-CACUGCCAUGGAGUAUCUGA$Z_{a3}$-3' (SEQ ID NO: 7);
5'-$Z_{a4}$UCAGAUACUCCAUGGCAGUGAC-3' (SEQ ID NO: 8),

wherein $Z_{a4}$ is the first nucleotide at 5' terminal of the antisense strand; $Z_{a3}$ is selected from A, U, G or C, and $Z_{a4}$ is a nucleotide complementary to $Z_{a3}$.

In some embodiments, the sense strand of the siRNA comprises the nucleotide sequence shown in SEQ ID NO: 127, and the antisense strand of the siRNA comprises the nucleotide sequence shown in SEQ ID NO: 128:

5'-ACAACAUCAAGUACUACG$Z_{b3}$-3' (SEQ ID NO: 127);
5'-$Z_{b4}$CGUAGUACUUGAUGUUGUUG-3' (SEQ ID NO: 128), or

the sense strand of the siRNA comprises the nucleotide sequence shown in SEQ ID NO: 129, and the antisense strand of the siRNA comprises the nucleotide sequence shown in SEQ ID NO: 130:

5'-CAACAACAUCAAGUACUACG$Z_{b3}$-3'(SEQ ID NO: 129);
5'-$Z_{b4}$CGUAGUACUUGAUGUUGUUGAU-3' (SEQ ID NO: 130),

wherein $Z_{b4}$ is the first nucleotide at 5' terminal of the antisense strand; $Z_{b3}$ is selected from A, U, G or C, and $Z_{b4}$ is a nucleotide complementary to $Z_{b3}$.

In some embodiments, the sense strand of the siRNA comprises the nucleotide sequence shown in SEQ ID NO: 249, and the antisense strand of the siRNA comprises the nucleotide sequence shown in SEQ ID NO: 250:

5'-CGACUACUACAUCUCCGU$Z_{c3}$-3'(SEQ ID NO: 249);
5'-$Z_{c4}$ACGGAGAUGUAGUAGUCGUU-3'(SEQ ID NO:250), or
the sense strand of the siRNA comprises the nucleotide sequence shown in SEQ ID NO: 251, and the antisense strand of the siRNA comprises the nucleotide sequence shown in SEQ ID NO: 252:

5'-AACGACUACUACAUCUCCGU$Z_{c3}$-3'(SEQ ID NO: 251);
5'-$Z_{c4}$ACGGAGAUGUAGUAGUCGUUGC-3' (SEQ ID NO: 252),

wherein $Z_{c4}$ is the first nucleotide at 5' terminal of the antisense strand; $Z_{c3}$ is selected from A, U, G or C, and $Z_{c4}$ is a nucleotide complementary to $Z_{c3}$.

In some embodiment, the siRNAs of the present disclosure are siRPTORa1, siRPTORa2, siRPTORa3, siRPTORb1, siRPTORb2, siRPTORb3, siRPTORc1, siRPTORc2, and siRPTORc3 listed in Table 1.

As described above, each nucleotide in the siRNA of the present disclosure is independently a modified or unmodified nucleotide. In some embodiments, the nucleotides in the siRNA of the present disclosure are unmodified nucleotides; in some embodiments, some or all nucleotides in the siRNA of the present disclosure are modified nucleotides, while the modifications on the nucleotide groups would not cause significant decrease or loss of the function of the siRNA of the present disclosure to inhibit the expression of *RPTOR* gene.

In some embodiments, the siRNA of the present disclosure comprises at least one modified nucleotide. In the context of the present disclosure, the term "modified nucleotide" used herein refers to a nucleotide formed by substituting the 2'-hydroxy of the ribose group with other groups, a nucleotide analogue, or a nucleotide with modified base. Said modified nucleotides would not cause significant decrease or loss of the function of the siRNA to inhibit the expression of genes. For example, the modified nucleotides disclosed by J.K. Watts, G. F. Deleavey and M. J.Damha, Chemically Modified siRNA: tools and applications. Drug Discov Today, 2008.13(19-20): p.842-55 may be selected.

In some embodiments, at least one nucleotide in the sense strand or the antisense strand of the siRNA of the present disclosure is a modified nucleotide, and/or at least one phosphate group is a phosphate group with modification group(s). In other words, at least a portion of the phosphate groups and/or ribose groups in the phosphate-ribose backbone of at least one single strand in the sense strand and the antisense strand are phosphate groups with modification groups and/or ribose groups with modification groups.

In some embodiments, all nucleotides in the sense strand and/or the antisense strand are modified nucleotides. In some embodiments, each nucleotide in the sense strand and the antisense strand of the siRNA of the present disclosure is independently a fluoro modified nucleotide or a non-fluoro modified nucleotide.

The inventors of the present disclosure have surprisingly found that the siRNA of the present disclosure has achieved a high degree of balance between plasma stability and gene silencing efficiency in animal experiments.

In some embodiments, the fluoro modified nucleotides are located in the nucleotide sequence I and the nucleotide sequence II; and the nucleotide sequence I comprises no more than 5 fluoro modified nucleotides; and in the direction from 5' terminal to 3' terminal, the nucleotides at positions 7, 8 and 9 of the nucleotide sequence I are fluoro modified nucleotides; the nucleotide sequence II comprises no more than 7 fluoro modified nucleotides; and the nucleotides at positions 2, 6, 14, and 16 of the nucleotide sequence II are fluoro modified nucleotides.

In some embodiments, in the direction from 5' terminal to 3' terminal, the nucleotides at positions 7, 8 and 9 or at positions 5, 7, 8 and 9 of the nucleotide sequence I in the sense strand are fluoro modified nucleotides, and the nucleotides at the other positions in the sense strand are non-fluoro modified nucleotides; in the direction from 5' terminal to 3' terminal, the nucleotides at positions 2, 6, 14, and 16 or at positions 2, 6, 8, 9, 14, and 16 of the nucleotide sequence II in the antisense strand are fluoro modified nucleotides, and the nucleotides at the other positions in the antisense strand are non-fluoro modified nucleotides.

In the context of the present disclosure, the "fluoro modified nucleotide" refers to a nucleotide formed by

substituting the 2'-hydroxy of the ribose group of the nucleotide with a fluoro group, which has a structure as shown by Formula (7). The "non-fluoro modified nucleotide" refers to a nucleotide formed by substituting the 2'-hydroxy of the ribose group of the nucleotide with a non-fluoro group, or a nucleotide analogue. In some embodiments, each non-fluoro modified nucleotide is independently selected from the group consisting of a nucleotide formed by substituting the 2'-hydroxy of the ribose group of the nucleotide with a non-fluoro group, and a nucleotide analogue.

The nucleotides formed by substituting 2'-hydroxy of the ribose group with a non-fluoro group are well-known to those skilled in the art, and can be one selected from the group consisting of 2'-alkoxy modified nucleotide, 2'-substituted alkoxy modified nucleotide, 2'-alkyl modified nucleotide, 2'-substituted alkyl modified nucleotide, 2'-amino modified nucleotide, 2'-substituted amino modified nucleotide, and 2'-deoxy nucleotide.

In some embodiments, the 2'-alkoxy modified nucleotide is a methoxy modified nucleotide (2'-OMe), as shown by Formula (8). In some embodiments, the 2'-substituted alkoxy modified nucleotide is, for example, a 2'-O-methoxyethyl modified nucleotide (2'-MOE) as shown by Formula (9). In some embodiments, the 2'-amino modified nucleotide (2'-$NH_2$) is as shown by Formula (10). In some embodiments, the 2'-deoxy nucleotide (DNA) is as shown by Formula (11):

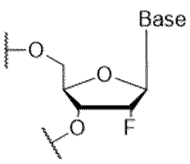 , 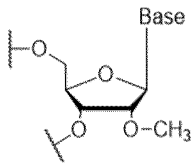 , 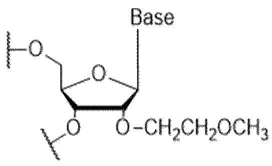 , 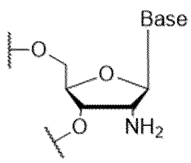 , 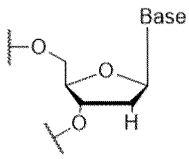

Formula (7) Formula (8) Formula (9) Formula (10) Formula (11).

The "nucleotide analogue" refers to a group that can replace a nucleotide in the nucleic acid, while structurally differs from an adenine ribonucleotide, a guanine ribonucleotide, a cytosine ribonucleotide, a uracil ribonucleotide, or thymine deoxyribonucleotide. In some embodiments, the nucleotide analogue may be an isonucleotide, a bridged nucleic acid (BNA) nucleotide or an acyclic nucleotide.

A BNA is a nucleotide that is constrained or is not accessible. BNA can contain a 5- membered ring, a 6-membered ring or a 7-membered ring bridged structure with a "fixed" C3'-endo sugar puckering. The bridge is typically incorporated at the 2'and 4'-position of the ribose to afford a 2', 4'-BNA nucleotide. In some embodiments, the BNA may be LNA, ENA and cET BNA, which are as shown by Formulae (12), (13) and (14), respectively:

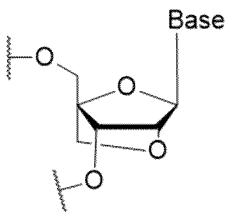 , 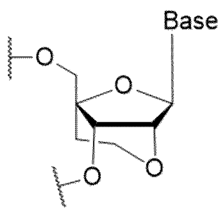 , 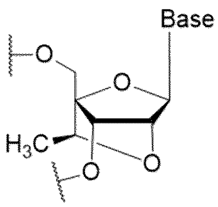

Formula (12) Formula (13) Formula (14).

An acyclic nucleotide is a nucleotide in which the ribose ring is opened. In some embodiments, the acyclic nucleotide may be an unlocked nucleic acid (UNA) nucleotide or a glycerol nucleic acid (GNA) nucleotide, which are as shown by Formulae (15) and (16), respectively:

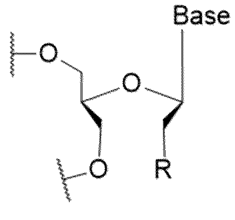 , 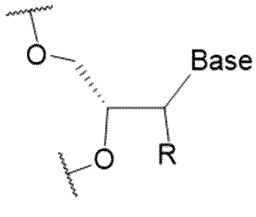

Formula (15) Formula (16).

In the above Formulae (15) and (16), R is selected from H, OH, or alkoxy (O-alkyl).

An isonucleotide is a compound in which the position of the base on the ribose ring changes. In some embodiments, the isonucleotide may be a compound in which the base is transposed from position-1' to position-2' or -3' on the ribose ring, as shown by Formula (17) or (18):

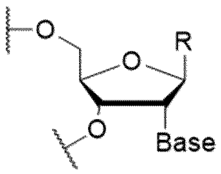

Formula (17)

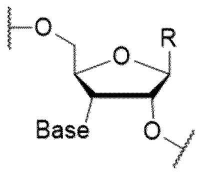

Formula (18).

In the above compounds of Formulae (17)-(18), "Base" represents a base of a nucleic acid, such as A, U, G, C, or T; R is selected from H, OH, F, or the above non-fluoro group.

In some embodiments, a nucleotide analogue is one selected from the group consisting of an isonucleotide, LNA, ENA, cET, UNA, and GNA. In some embodiments, each non-fluoro modified nucleotide is a methoxy modified nucleotide. In the context of the present disclosure, the methoxy modified nucleotide refers to a nucleotide formed by substituting the 2'-hydroxy of the ribose group with a methoxy group. In some embodiments, the non-fluoro modified nucleotide is independently a 2'-O-methoxy modified nucleotide or a 2'-O-methoxyethyl modified nucleotide.

In the context of the present disclosure, the "fluoro modified nucleotide" refers to a compound in which 2'-hydroxy of the nucleotide is substituted with a fluoro group, which has a structure as shown by Formula (7). The "methoxy modified nucleotide" refers to a compound in which 2'-hydroxy of the ribose group in the nucleotide is substituted with a methoxy, which has a structure as shown by Formula (8). The "2'-O-methoxyethyl modified nucleotide" refers to a compound in which 2'-hydroxy of the ribose group in the nucleotide is substituted with a methoxyethyl, which has a structure as shown by Formula (9).

In some embodiments, the siRNA of the present disclosure is a siRNA with the following modifications: in the direction from 5' terminal to 3' terminal, the nucleotides at positions 7, 8 and 9 or at positions 5, 7, 8 and 9 of the nucleotide sequence I in the sense strand are fluoro modified nucleotides, and the nucleotides at the other positions in the sense strand are methoxy modified nucleotides; the nucleotides at positions 2, 6, 14, and 16 or at positions 2, 6, 8, 9, 14, and 16 of the nucleotide sequence II in the antisense strand are fluoro modified nucleotides, and the nucleotides at the other positions in the antisense strand are methoxy modified nucleotides.

In some embodiments, the siRNA of the present disclosure is a siRNA with the following modifications: in the direction from 5' terminal to 3' terminal, the nucleotides at positions 5, 7, 8, and 9 of the nucleotide sequence I in the sense strand of the siRNA are fluoro modified nucleotides, and the nucleotides at the other positions of the sense strand of the siRNA are methoxy modified nucleotides; and in the direction from 5' terminal to 3' terminal, the nucleotides at positions 2, 6, 14 and 16 or at positions 2, 6, 8, 9, 14, and 16 of the nucleotide sequence II in the antisense strand of the siRNA are fluoro modified nucleotides, and the nucleotides at the other positions in the antisense strand of the siRNA are methoxy modified nucleotides.

Alternatively, in the direction from 5' terminal to 3' terminal, the nucleotides at positions 5, 7, 8 and 9 of the nucleotide sequence I in the sense strand of the siRNA are fluoro modified nucleotides, and the nucleotides at the other positions in the sense strand of the siRNA are methoxy modified nucleotides; and, in the direction from 5' terminal to 3' terminal, the nucleotides at positions 2, 6, 14 and 16 of the nucleotide sequence II in the antisense strand of the siRNA are fluoro modified nucleotides, and the nucleotides at the other positions in the antisense strand of the siRNA are methoxy modified nucleotides.

Alternatively, in the direction from 5' terminal to 3' terminal, the nucleotides at positions 7, 8 and 9 of the nucleotide sequence I in the sense strand of the siRNA are fluoro modified nucleotides, and the nucleotides at the other positions in the sense strand of the siRNA are methoxy modified nucleotides; and in the direction from 5' terminal to 3' terminal, the nucleotides at positions 2, 6, 14, and 16 of the nucleotide sequence II in the antisense strand of the siRNA are fluoro modified nucleotides, and the nucleotides at the other positions in the antisense strand of the siRNA are methoxy modified nucleotides.

In some embodiments, the siRNA of the present disclosure is a siRNA with the following modifications: in the direction from 5' terminal to 3' terminal, the nucleotides at positions 7, 8, and 9 or at positions 5, 7, 8, and 9 of the nucleotide sequence I in the sense strand of the siRNA are fluoro modified nucleotides, and the nucleotides at the other positions of the sense strand of the siRNA are methoxy modified nucleotides; and in the direction from 5' terminal to 3' terminal, the nucleotides at positions 2, 6, 14, and 16 or at positions 2, 6, 8, 9, 14, and 16 of the nucleotide sequence II in the antisense strand of the siRNA are fluoro modified nucleotides; and in the direction from 5' terminal to 3' terminal, at least one nucleotide of the nucleotides at positions 3-6 of the nucleotide sequence II is a 2'-O-methoxyethyl modified

nucleotide. In some embodiments, the nucleotide at position 3 or 5 of the nucleotide sequence II in the antisense strand is a 2'-O-methoxyethyl modified nucleotide, and the nucleotides at the other positions in the antisense strand are methoxy modified nucleotides. In some embodiments, in the direction from 5' terminal to 3' terminal, no more than 2 nucleotides of the nucleotides at positions 3-9 of the nucleotide sequence II are 2'-O-methoxyethyl modified nucleotides.

In some embodiments, the siRNA of the present disclosure is any one of the following siRNAs: siRPTORa1-M1, siRPTORa1-M2, siRPTORa1-M3, siRPTORa2-M1, siRPTORa2-M2, siRPTORa2-M3, siRPTORa3-M1, siRPTORa3-M2, siS*RPTOR*a3-M3, siRPTORb1-M1, siRPTORb1-M2, siRPTORb1-M3, siRPTORb2-M1, siRPTORb2-M2, siRPTORb2-M3, siRPTORb3-M1, siRPTORb3-M2, siS*RPTOR*b3-M3, siRPTORc1-M1, siRPTORc1-M2, siRPTORc1-M3, siRPTORc2-M1, siRPTORc2-M2, siRPTORc2-M3, siRPTORc3-M1, siRPTORc3-M2, and siS*RPTO*Rc3-M3.

The siRNAs with the above modifications not only have lower costs, but also make it difficult for the ribonucleases in blood to cleave the nucleic acid, thereby increasing the stability of the nucleic acid and rendering the nucleic acid to have stronger resistance against nuclease hydrolysis. Meanwhile, the siRNAs with the above modifications also exhibit higher inhibitory activity against the target mRNA.

In some embodiments, at least a portion of the phosphate groups in the phosphate-ribose backbone of at least one single strand in the sense strand and the antisense strand of the siRNA of the present disclosure are phosphate groups with modification groups. In some embodiments, the phosphate group with modification group(s) is a phosphorothioate group formed by substituting at least one oxygen atom in a phosphodiester bond in the phosphate group with a sulfur atom; and in some embodiments the phosphate group with modification group(s) is a phosphorothioate group having a structure as shown by Formula (1):

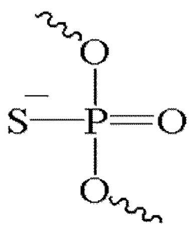

Formula (1).

This modification can stabilize the double-stranded structure of the siRNA, while maintaining high specificity and high affinity of base pairing.

In some embodiments, in the siRNAs of the present disclosure, a phosphorothioate linkage exists in at least one of the following positions: the position between the first and the second nucleotides at either terminal of the sense strand or antisense strand, the position between the second and the third nucleotides at either terminal of the sense strand or antisense strand, or any combination thereof. In some embodiments, a phosphorothioate linkage exists at all the above positions except for 5' terminal of the sense strand. In some embodiments, a phosphorothioate linkage exists at all the above positions except for 3' terminal of the sense strand. In some embodiments, a phosphorothioate linkage exists in at least one of the following positions:

the position between the first and second nucleotides at 5' terminal of the sense strand;
the position between the second and third nucleotides at 5' terminal of the sense strand;
the position between the first and second nucleotides at 3' terminal of the sense strand;
the position between the second and third nucleotides at 3' terminal of the sense strand;
the position between the first and second nucleotides at 5' terminal of the antisense strand;
the position between the second and third nucleotides at 5' terminal of the antisense strand;
the position between the first and second nucleotides at 3' terminal of the antisense strand; and
the position between the second and third nucleotides at 3' terminal of the antisense strand.

In some embodiments, the siRNA of the present disclosure is any one of the following siRNAs: siRPTORa1-M1S, siRPTORal-MIX, siRPTORa1-M2S, siRPTORa1-M2X, siRPTORa1-M3S, siRPTORa1-M3X, siRPTORa2-M1S, siRPTORa2-M1X, siRPTORa2-M2S, siRPTORa2-M2X, siRPTORa2-M3S, siRPTORa2-M3X, siRPTORa3-M1S, siRPTORa3-M1X, siRPTORa3-M2S, siRPTORa3-M2X, siRPTORa3-M3S, siRPTORa3-M3X, siRPTORa1-T1S, siRPTORa1-T2S, siRPTORb1-M1S, siRPTORb1-M1X, siRPTORb1-M2S, siRPTORb1-M2X, siRPTORb1-M3S, siRPTORb1-M3X, siRPTORb2-M1S, siRPTORb2-M1X, siRPTORb2-M2S, siRPTORb2-M2X, siRPTORb2-M3S, siRPTORb2-M3X, siRPTORb3-MIS, siRPTORb3-M1X, siRPTORb3-M2S, siRPTORb3-M2X, siRPTORb3-M3S,

siRPTORb3-M3X, siRPTORb1-T1S, siRPTORb1-T2S, siRPTORc1-M1S, siRPTORc1-M1X, siRPTORc1-M2S, siRPTORc1-M2X, siRPTORc1-M3S, siRPTORc1-M3X, siRPTORc2-M1S, siRPTORc2-M1X, siRPTORc2-M2S, siRPTORc2-M2X, siRPTORc2-M3S, siRPTORc2-M3X, siRPTORc3-M1S, siRPTORc3-M1X, siRPTORc3-M2S, siRPTORc3-M2X, siRPTORc3-M3S, siRPTORc3-M3X, siRPTORc1-T1S, and siRPTORc1-T2S.

In some embodiments, the nucleotide at 5'-terminal in the antisense strand of the siRNA is a 5'-phosphate nucleotide or a 5'-phosphate analogue modified nucleotide.

Typical 5'-phosphate nucleotides or 5'-phosphate analogue modified nucleotides are well known to those skilled in the art. For example, the 5'-phosphate nucleotides may have the following structure:

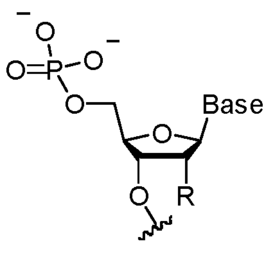

Formula (2);

as another example, Anastasia Khvorova and Jonathan K. Watts, The chemical evolution of oligonucleotide therapies of clinical utility. Nature Biotechnology, 2017, 35(3): 238-48 discloses the following four 5'-phosphate analogue modified nucleotides:

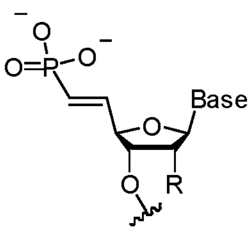

Formula (3)

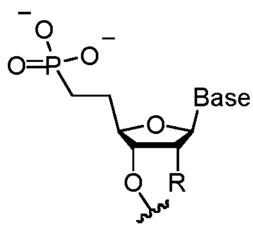

Formula (4)

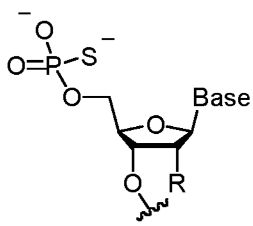

Formula (5)

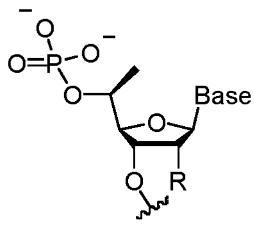

Formula (6)

wherein R is selected from H, OH, methoxy, and F; "Base" represents a nucleic acid base selected from A, U, C, G, or T.

In some embodiments, the 5'-phosphate nucleotide is a nucleotide with 5'-phosphate modification as shown in Formula (2), the 5'-phosphate analogue modified nucleotide is a nucleotide with 5'-(E)-vinylphosphonate (E-VP) modification as shown in Formula (3) or phosphorothioate modified nucleotide as shown in Formula (5).

In some embodiments, the siRNA of the present disclosure is any one of the following siRNAs: siRPTORa1-M1P1, siRPTORa1-M2P1, siRPTORa1-M3P1, siRPTORa2-M1P1, siRPTORa2-M2P1, siRPTORa2-M3P1, siRPTORa3-M1P1, siRPTORa3-M2P1, siRPTORa3-M3P1, siRPTORa1-M1SP1, siRPTORa1-M2SP1, siRPTORa1-M3SP1, siRPTORa2-M1SP1, siRPTORa2-M2SP1, siRPTORa2-M3SP1, siRPTORa3-M1SP1, siRPTORa3-M2SP1, siRPTORa3-M3SP1, siRPTORa1-M1XP1, siRPTORa1-M2XP1, siRPTORa1-M3XP1, siRPTORa2-M1XP1, siRPTORa2-M2XP1, siRPTORa2-M3XP1, siRPTORa3-M1XP1, siRPTORa3-M2XP1, siRPTORa3-M3XP1, siRPTORb1-M1P1, siRPTORb1-M2P1, siRPTORb1-M3P1, siRPTORb2-M1P1, siRPTORb2-M2P1, siRPTORb2-M3P1, siRPTORb3-M1P1, siRPTORb3-M2P1, siRPTORb3-M3P1, siRPTORb1-M1SP1, siRPTORb1-M2SP1, siRPTORb1-M3SP1, siRPTORb2-M1SP1, siRPTORb2-M2SP1, siRPTORb2-M3SP1, siRPTORb3-M1SP1, siRPTORb3-M2SP1, siRPTORb3-M3SP1, siRPTORb1-M1XP1, siRPTORb1-M2XP1, siRPTORb1-M3XP1, siRPTORb2-M1XP1, siRPTORb2-M2XP1, siRPTORb2-M3XP1, siRPTORb3-M1XP1, siRPTORb3-M2XP1, siRPTORb3-M3XP1, siRPTORc1-M1P1, siRPTORc1-M2P1, siRPTORc1-M3P1, siRPTORc2-M1P1, siRPTORc2-M2P1, siRPTORc2-M3P1, siRPTORc3-M1P1, siRPTORc3-M2P1, siRPTORc3-M3P1, siRPTORc1-M1SP1, siRPTORc1-M2SP1, siRPTORc1-M3SP1, siRPTORc2-M1SP1, siRPTORc2-M2SP1, siRPTORc2-M3SP1, siRPTORc3-M1SP1, siRPTORc3-M2SP1, siRPTORc3-M3SP1, siRPTORc1-M1XP1, siRPTORc1-M2XP1, siRPTORc1-M3XP1, siRPTORc2-M1XP1, siRPTORc2-M2XP1, siRPTORc2-M3XP1, siRPTORc3-M1XP1, siRPTORc3-M2XP1, and siRPTORc3-M3XP1.

The inventors of the present disclosure have surprisingly found that the siRNAs of the present disclosure have significantly enhanced plasma and lysosomal stability, while maintaining higher gene-suppressing activity.

The siRNAs of the present disclosure can be obtained by conventional methods for preparing siRNAs in the

art, e.g., solid phase synthesis and liquid phase synthesis methods. Therein, commercial customization services have already been available for solid phase synthesis. A modified nucleotides can be introduced into the siRNAs of the present disclosure by using a nucleotide monomer having a corresponding modification, wherein the methods for preparing a nucleotide monomer having a corresponding modification and the methods for introducing a modified nucleotide into a siRNA are also well-known to those skilled in the art.

Pharmaceutical Composition

In one aspect, the present disclosure provides a pharmaceutical composition, comprising the above siRNA as an active ingredient and a pharmaceutically acceptable carrier.

The pharmaceutically acceptable carrier may be a carrier conventionally used in the field of siRNA administration, for example, but not limited to, one or more of magnetic nanoparticles (such as $Fe_3O_4$ and $Fe_2O_3$-based nanoparticle), carbon nanotubes, mesoporous silicon, calcium phosphate nanoparticles, polyethylenimine (PEI), polyamidoamine (PAMAM) dendrimer, poly(L-lysine) (PLL), chitosan, 1, 2-dioleoyl-3-trimethylammonium-propane (DOTAP), poly(D&L-lactic/glycolic acid) copolymer (PLGA), poly(2-aminoethyl ethylene phosphate) (PPEEA), poly(2-dimethylaminoethyl methacrylate) (PDMAEMA), and derivatives thereof.

In some embodiments, in the pharmaceutical composition of the present invention, there are no special requirements for the contents of the siRNA and the pharmaceutically acceptable carrier. In some embodiments, the weight ratio of the siRNA to the pharmaceutically acceptable carrier is 1: (1-500); and in some embodiments, the weight ratio is 1: (1-50).

In some embodiments, the pharmaceutical composition of the present invention may also comprise other pharmaceutically acceptable excipients, which may be one or more of various conventional formulations or compounds in the art. For example, said other pharmaceutically acceptable excipients may comprise at least one of a pH buffer, a protective agent and an osmotic pressure regulator.

The pH buffer may be a tris(hydroxymethyl) aminomethane hydrochloride buffer solution with a pH of 7.5-8.5, and/or a phosphate buffer solution with a pH of 5.5-8.5, such as a phosphate buffer solution with a pH of 5.5-8.5.

The protective agent may be at least one of inositol, sorbitol, sucrose, trehalose, mannose, maltose, lactose, and glucose. The content of the protective agent may be from 0.01 wt % to 30 wt % based on the total weight of the pharmaceutical composition.

The osmotic pressure regulator may, for example, be sodium chloride and/or potassium chloride. The content of the osmotic pressure regulator allows the osmotic pressure of the pharmaceutical composition to be 200-700 mOsm/kg. Depending on the desired osmotic pressure, those skilled in the art could readily determine the content of the osmotic pressure regulator. In some embodiments, the dose of the formulation prepared from the pharmaceutical composition will be adjusted due to different administration manners during administration.

In some embodiments, the pharmaceutical composition may be a liquid formulation, for example, an injection solution; or a lyophilized powder for injection, which is mixed with a liquid excipient to form a liquid formulation upon administration. The liquid formulation may be used for, but not limited to, administration by subcutaneous, intramuscular, intracerebroventricular, or intrathecal injection, and the pharmaceutical composition may also be delivered by, but not limited to, nasal administration, oropharyngeal inhalation, spray administration, or other routes. In some embodiments, the pharmaceutical composition is delivered by intrathecal injection. In some embodiments, intrathecal injection of the pharmaceutical composition into spinal fluid can be performed as a bolus injection or via minipumps, wherein the mini pumps can be implanted beneath the skin, providing a regular and constant delivery of siRNA into spinal fluid. In some embodiments, intrathecal administration is performed via a surgically-implanted osmotic pump. In some embodiments, the osmotic pump is implanted into subarachnoid space of spinal canal to facilitate intrathecal administration. More details about this intrathecal delivery system may be found in PCT/US2015/013253 filed on Jan. 28, 2015, which is incorporated by reference in its entirety.

In some embodiments, the pharmaceutical composition may be in the form of a liposome formulation. In some embodiments, the pharmaceutically acceptable carrier used in the liposome formulation comprises an amine-containing transfection compound (hereinafter also referred to as an organic amine), a helper lipid and/or a PEGylated lipid. Therein, the organic amine, the helper lipid and the PEGylated lipid may be respectively selected from one or more of the amine-containing transfection compounds or the pharmaceutically acceptable salts or derivatives thereof, the helper lipids and the PEGylated lipids as described in the Chinese patent application CN103380113A, which is incorporated herein by reference in its entirety.

In some embodiments, the organic amine may be a compound as shown by Formula (201) as described in the Chinese patent application CN103380113A or a pharmaceutically acceptable salt thereof:

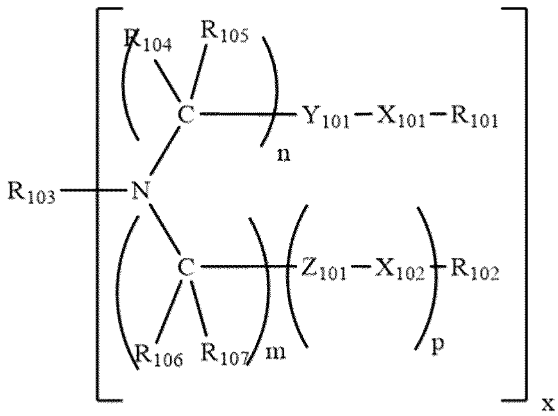

Formula (201),

wherein:

$X_{101}$ and $X_{102}$ independently of one another are selected from O, S, N-A and C-A, wherein A is hydrogen or a $C_1$-$C_{20}$ hydrocarbon chain;

$Y_{101}$ and $Z_{101}$ independently of one another are selected from C=O, C=S, S=O, CH-OH and $SO_2$;

$R_{101}$, $R_{102}$, $R_{103}$, $R_{104}$, $R_{105}$, $R_{106}$, and $R_{107}$ independently of one another are selected from hydrogen; a cyclic or an acyclic, substituted or unsubstituted, branched or linear aliphatic group; a cyclic or an acyclic, substituted or unsubstituted, branched or linear heteroaliphatic group; a substituted or unsubstituted, branched or linear acyl group; a substituted or unsubstituted, branched or linear aryl group; and a substituted or unsubstituted, branched or linear heteroaryl group;

x is an integer of 1-10;

n is an integer of 1-3, m is an integer of 0-20, p is 0 or 1, wherein if m=p=0, then $R_{102}$ is hydrogen; and

if at least one of n and m is 2, then $R_{103}$ and nitrogen in Formula (201) form a structure as shown by Formula (202) or (203):

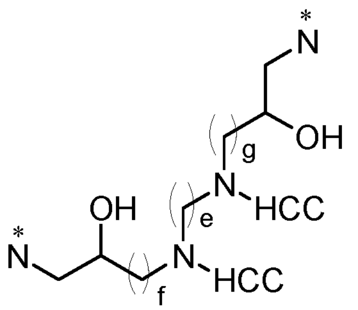

Formula (202)

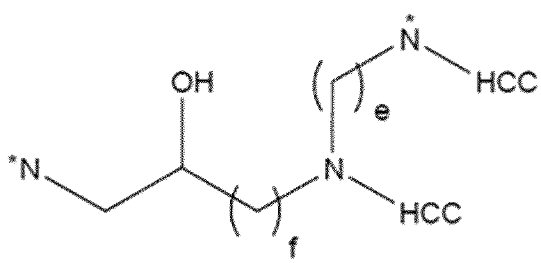

Formula (203);

wherein g, e and f independently of one another are an integer of 1-6; "HCC" represents a hydrocarbon chain, and each *N represents a nitrogen atom shown in Formula (201).

In some embodiments, $R_{103}$ is a polyamine. In other embodiments, $R_{103}$ is a ketal. In some embodiments, $R_{101}$ and $R_{102}$ in the Formula (201) independently of one another are any of substituted or unsubstituted, branched or linear alkyl or alkenyl groups which have 3-20 carbon atoms (such as 8-18 carbon atoms) and 0-4 double bonds (such as 0-2 double bonds).

In some embodiments, if n and m independently of one another are 1 or 3, $R_{103}$ represents any of the following

Formulae (204)-(213):

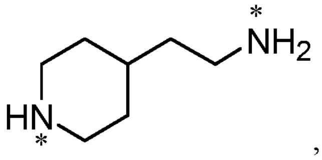

,

Formula (204)

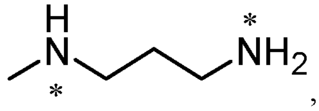

,

Formula (205)

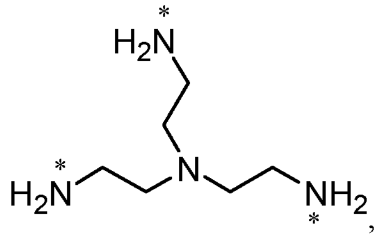

,

Formula (206)

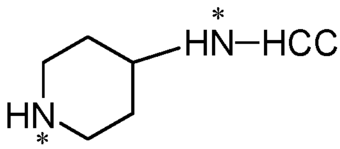

,

Formula (207)

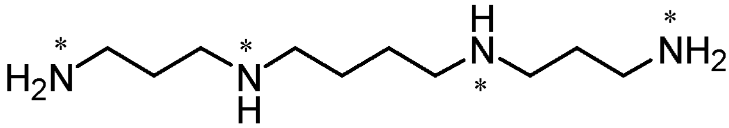

,

Formula (208)

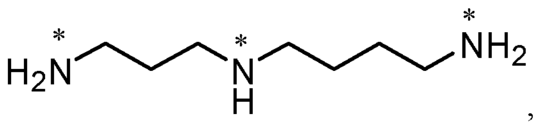

,

Formula (209)

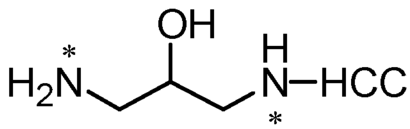

,

Formula (210)

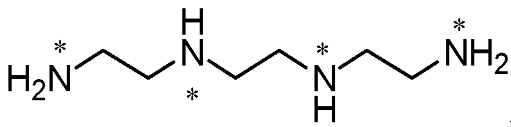

,

Formula (211)

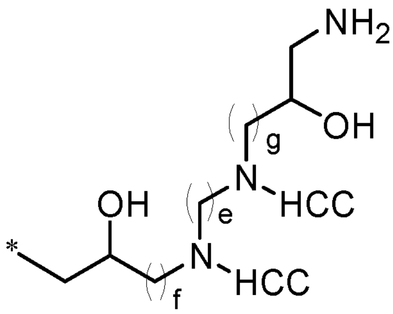

, and

Formula (212)

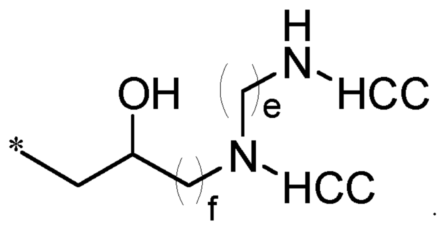

;

Formula (213)

wherein in Formulae (204)-(213), g, e and f independently of one another are an integer of 1-6; each "HCC" represents a hydrocarbon chain; and each * represents a potential attachment point of $R_{103}$ to the nitrogen atom in Formula (201), wherein each H at any * position can be replaced to achieve the attachment to the nitrogen atom in Formula (201).

The compound as shown by Formula (201) may be prepared according to the description of the Chinese patent application CN103380113A.

In some embodiments, the organic amine may be an organic amine as shown by Formula (214) and/or an organic amine as shown by Formula (215):

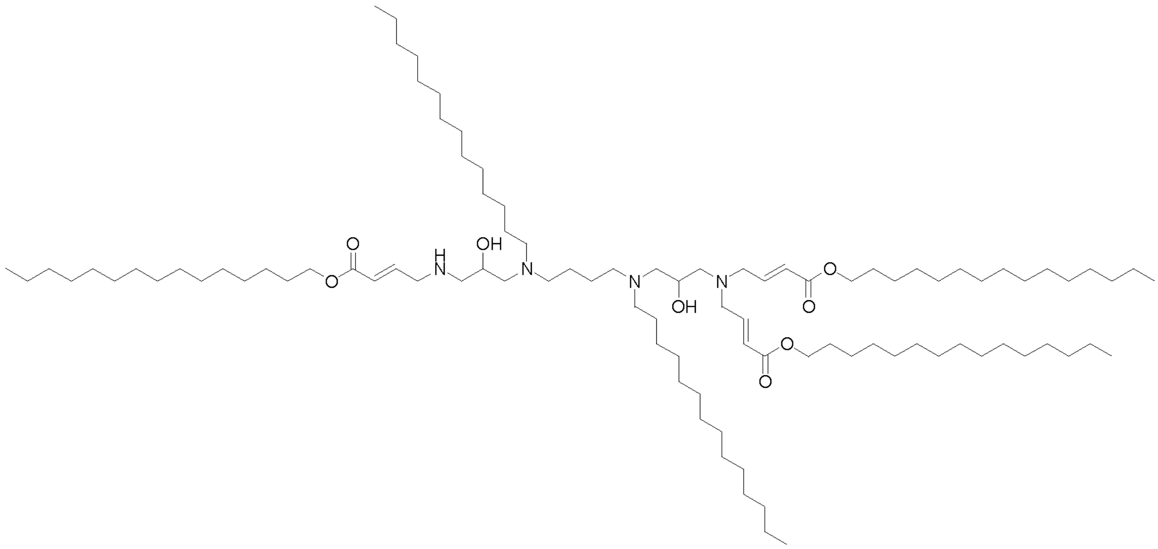

,

Formula (214)

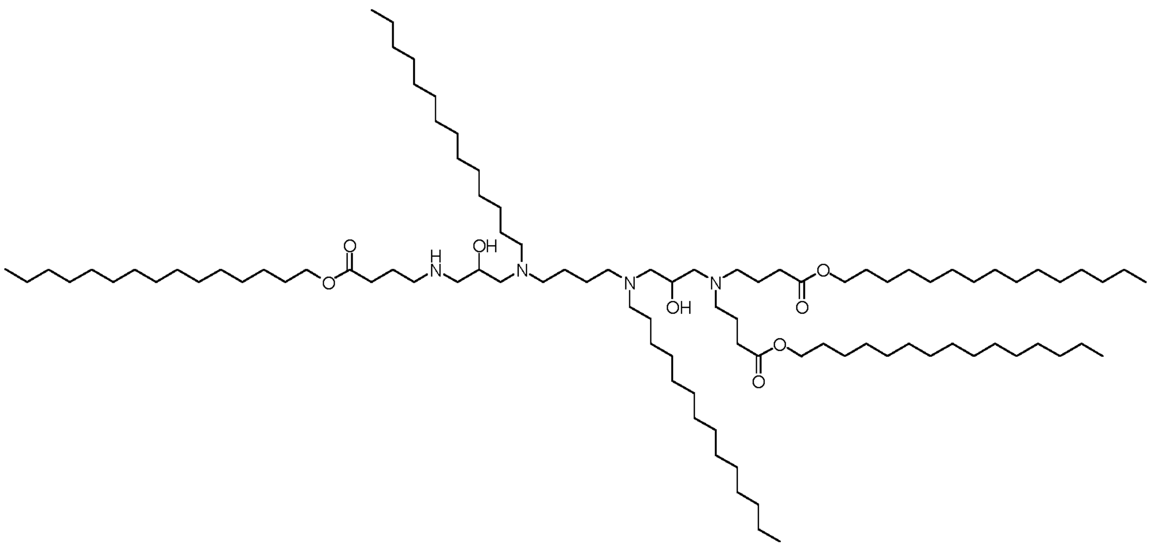

;

Formula (215)

the helper lipid is cholesterol, cholesterol analogs and/or cholesterol derivatives; and the PEGylated lipid is 1, 2-dipalmitoyl-sn-glycero-3-phosphatidylethanolamine-N-[methoxy(polyethylene glycol)]-2000.

In some embodiments, the molar ratio among the organic amine, the helper lipid, and the PEGylated lipid in the pharmaceutical composition is (19.7-80): (19.7-80): (0.3-50); for example, the molar ratio may be (50-70): (20-40): (3-20).

In some embodiments, the pharmaceutical compositions formed by the siRNA of the present disclosure and the above amine-containing transfection agents have an average diameter from about 30 nm to about 200 nm, typically from about 40 nm to about 135 nm, and more typically, the average diameter of the liposome particles is from about 50 nm to about 120 nm, from about 50 nm to about 100 nm, from about 60 nm to about 90 nm, or from about 70 nm to about 90 nm, for example, the average diameter of the liposome particles is about 30, 40, 50, 60, 70, 75, 80, 85, 90, 100, 110, 120, 130, 140, 150 or 160 nm.

In some embodiments, in the pharmaceutical composition formed by the siRNA of the present disclosure and the above amine-containing transfection agents, the weight ratio (weight/weight ratio) of the siRNA to total lipids, e.g., the organic amines, the helper lipids and/or the PEGylated lipids, ranges from about 1:1 to about 1:50, from about 1:1 to about 1:30, from about 1:3 to about 1:20, from about 1:4 to about 1:18, from about 1:5 to about 1:17, from about 1:5 to about 1:15, from about 1:5 to about 1:12, from about 1:6 to about 1:12, or from about 1:6 to about 1:10. For example,

the weight ratio of the siRNA of the present disclosure to total lipids is about 1:5, 1:6, 1:7, 1:8, 1:9, 1:10, 1:11, 1:12, 1:13, 1:14, 1:15, 1:16, 1:17 or 1:18.

In some embodiments, the pharmaceutical composition may be marketed with each component being separate, and used in the form of a liquid formulation. In some embodiments, the pharmaceutical composition formed by the siRNA of the present disclosure and the above pharmaceutically acceptable carrier may be prepared by various known processes, except replacing the existing siRNA with the siRNA of the present disclosure. In some embodiments, the pharmaceutical composition may be prepared according to the following process.

The organic amines, helper lipids and PEGylated lipids are suspended in alcohol at a molar ratio as described above and mixed homogeneously to yield a lipid solution; the alcohol is used in an amount such that the resultant lipid solution is present at a total mass concentration of 2 to 25 mg/mL (e.g., 8 to 18 mg/mL). The alcohol is a pharmaceutically acceptable alcohol, such as an alcohol that is in liquid form at about room temperature, for example, one or more of ethanol, propylene glycol, benzyl alcohol, glycerol, PEG 200, PEG 300, PEG 400; such as, being ethanol.

The siRNA of the present disclosure is dissolved in a buffered salt solution to produce an aqueous solution of the siRNA. The buffered salt solution has a concentration of 0.05 to 0.5 M, such as 0.1 to 0.2 M. The pH of the buffered salt solution is adjusted to 4.0 to 5.5, such as 5.0 to 5.2. The buffered salt solution is used in an amount such that the siRNA is present at a concentration of less than 0.6 mg/ml, such as 0.2 to 0.4 mg/mL. The buffered salt may be one or more selected from the group consisting of soluble acetate and soluble citrate, such as sodium acetate and/or potassium acetate.

The lipid solution and the aqueous solution of the siRNA are mixed. The product obtained after mixing is incubated at a temperature of 40 to 60°C for at least 2 minutes (e.g., 5 to 30 minutes) to produce an incubated lipid formulation. The volume ratio of the lipid solution to the aqueous solution of the siRNA is 1: (2-5), such as 1:4.

The incubated liposome formulation is concentrated or diluted, and then subjected to impurity removal and sterilization to afford the pharmaceutical composition of the present disclosure, which has the following physicochemical parameters: a pH of 6.5 to 8, an encapsulation percentage of not lower than 80%, a particle size of 40 to 200 nm, a polydispersity index of no greater than 0.30, and an osmotic pressure of 250 to 400 mOsm/kg. For example, the physicochemical parameters may be as follows: a pH of 7.2 to 7.6, an encapsulation percentage of not lower than 90%, a particle size of 60 to 100 nm, a polydispersity index of no greater than 0.20, and an osmotic pressure of 300 to 400 mOsm/kg.

Therein, the concentration or dilution step may be performed before, after or simultaneously with the step of impurity removal. The method for removing impurities may be any of various existing methods, for example, ultrafiltration under 100 kDa using a tangential flow system and a hollow fiber column, with a phosphate buffer (PBS) at pH 7.4 as ultrafiltration exchange solution, and. The method for sterilization may be any of various existing methods, such as filtration sterilization on a 0.22 μm filter.

<u>siRNA conjugate</u>

In another aspect, the present disclosure provides a siRNA conjugate comprising the above siRNA and a conjugation group conjugated to the siRNA.

In general, the conjugation group comprises at least one pharmaceutically acceptable targeting group and/or a delivery auxiliary group. In some embodiments, the conjugation group further comprises a linker, and the linker and/or the targeting group or the delivery auxiliary group are sequentially linked. In some embodiments, there are 1 to 6 targeting groups. In some embodiments, there are 2 to 4 targeting groups. The siRNA molecule may be non-covalently or covalently conjugated to the conjugation group, for example, the siRNA molecule is covalently conjugated to the conjugation group. The conjugation site between the siRNA and the conjugation group can be at 3'-terminal or 5'-terminal of the sense strand of the siRNA, or at 5'-terminal of the antisense strand of the siRNA, or within the internal sequence of the siRNA. In some embodiments, the conjugation site between the siRNA and the conjugation group can be at 3'-terminal of the sense strand of the siRNA. In some embodiments, the conjugation group is linked to the phosphate group, the 2'-hydroxy group or the base of a nucleotide. In some embodiments, the conjugation group may be linked to a 3'-hydroxy group when the nucleotides are linked via a 2'-5'-phosphodiester bond. When the conjugation group is linked to a terminal of the siRNA, the conjugation group is typically linked to a phosphate group of a nucleotide; when the conjugation group is linked to an internal sequence of the siRNA, the conjugation group is typically linked to a ribose ring or a base. For specific linking manners, reference may be made to the literature: Muthiah Manoharan et.al. siRNA conjugates carrying sequentially assembled trivalent N-acetylgalactosamine linked through nucleosides elicit robust gene silencing in vivo in hepatocytes. ACS Chemical biology, 2015, 10(5):1181-7.

In some embodiments, the siRNA and the conjugation group can be linked by an acid-labile or reducible chemical bond, and these chemical bonds can be degraded under the acidic environment of cell endosomes, thereby rendering the siRNA to be in free state. For nondegradable conjugation modes, the conjugation group can be linked to the sense strand of the siRNA, thereby minimizing the effect of conjugation on the activity of the siRNA.

In some embodiments, the pharmaceutically acceptable targeting group may be a conventional ligand in the field of siRNA administration. In some embodiments, each ligand is independently selected from a ligand capable of binding to a cell surface receptor. In some embodiments, at least one targeting ligand targets a receptor which mediates delivery to a central nervous system (CNS) tissue. The types of these ligands are well-known to those skilled in the art and they serve the function of binding to specific receptors on the surface of the target cell, thereby mediating delivery of the siRNA linked to the ligand into the target cell. In some embodiments, at least one of the targeting groups is selected from a ligand capable of binding to a cell surface receptor that expresses *RPTOR.* In some embodiments, at least one of the targeting groups is a ligand that targets a receptor on the surface of hepatic parenchymal cells. In some embodiments, at least one of or each of the targeting groups is a ligand that targets an asialoglycoprotein receptor (ASGPR) on the surface of a hepatocyte. In some embodiments, at least one of or each of the targeting groups is N-acetylgalactosamine (GalNAc). In some embodiments, the conjugates of the present disclosure have the structures of the various siRNA conjugates as disclosed in CN110959011A, which is incorporated herein by reference in its entirety.

In some embodiments, the conjugate of the present disclosure has a structure as shown by Formula (301):

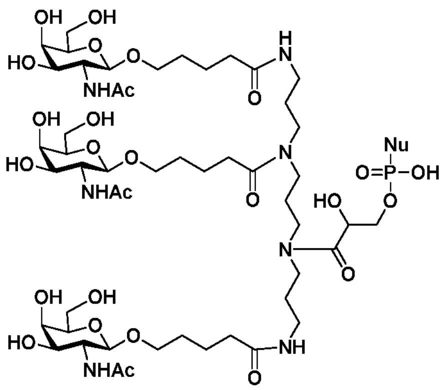

Formula (301),

wherein Nu represents a siRNA group formed by the siRNA of the present disclosure. In some embodiments, the siRNA conjugate has a structure shown in formula (301), wherein Nu has a sequence corresponding to any one of siRPTORa2-M1S, siRPTORb2-M1S, siRPTORc2-M1S, siRPTORc1-T1S, and siRPTORc1-T2S; and the conjugation group is linked to the 3'-position on the ribose ring of the nucleotide at 3' terminal of the sense strand of the siRNA in the Nu, and the siRNA conjugate is in the form of a sodium salt.

In some embodiments, each of the targeting groups is selected from a ligand capable of binding to a cell surface receptor that expresses *RPTOR.* In some embodiments, at least one of the targeting groups is a ligand that targets a receptor on the surface of the target cell in CNS. In some embodiments, each of the targeting groups is a ligand that targets a receptor on the surface of the target cell surface in CNS. In some embodiments, the ligand can be conjugated to the siRNA to enable specific delivery to a CNS tissue. In some embodiments, at least one of the targeting groups is a peptide ligand. In some embodiments, each of the targeting groups is a peptide ligand. In some embodiments, the targeting ligand is selected from the group consisting of Angiopep-2, lipoprotein receptor related protein (LRP) ligand, bEnd.3 cell binding ligand, transferrin receptor (TfR) ligand, mannose receptor ligand, glucose transporter protein, and LDL receptor ligand. For example, various targeting groups as described in CN112400018A (e.g., the ligands as described at paragraph [0856]), which is incorporated herein by reference in its entirety.

In some embodiments, the pharmaceutically acceptable delivery auxiliary group may be a lipophilic group, including an aliphatic or alicyclic compound. In some embodiments, the lipophilic group comprises a linear aliphatic hydrocarbon, a branched aliphatic hydrocarbon or a steroid. In some embodiments, the lipophilic group comprises a saturated or unsaturated C4-C30 hydrocarbon chain. In some embodiments, the lipophilic group comprises a saturated or unsaturated C6-C18 hydrocarbon chain (e.g., a linear C6-C18 alkyl or alkenyl). In some embodiments, the lipophilic group comprises a saturated or unsaturated C16 hydrocarbon chain (e.g., a linear C16 alkyl or alkenyl). In some embodiments, the lipophilic group is a C6-C30 aliphatic acyl group which refers to the remaining radical after removal of the hydroxy group in a C6-C30 fatty acid. In some embodiments, the lipophilic group is non-covalently or covalently conjugated to the siRNA. In some embodiments, the conjugation site between the lipophilic group and the siRNA is at 3'-terminal or 5'-terminal of the sense strand of the siRNA. In some embodiments, the conjugation site between the lipophilic group and the siRNA is at 5'-terminal of the antisense strand. In some embodiments, the conjugation site between the lipophilic group and the siRNA can also be within the internal sequence of the siRNA. In some embodiments, the lipophilic group is linked to the phosphate group, the ribose ring or the base of a nucleotide. In some embodiments, when the lipophilic group is conjugated to the base of a nucleotide, the preferred site is one that does not interfere with

the hydrogen bonding interaction needed for base pairing. In some embodiments, the lipophilic group is linked to the phosphate group, the 2'-hydroxy group or the base of a nucleotide. In some embodiments, the lipophilic group can be conjugated to the ribose ring via the 2'-hydroxy group. Various methods of linking the delivery auxiliary group to the siRNA are well-known to those skilled in the art; for example, the various preparation methods as described in CN112400018A (e.g., the preparation methods as described at paragraphs [1053]-[1065]), which is incorporated herein by reference in its entirety.

In some embodiments, the targeting group is conjugated to the siRNA via one or more linkers. The inventors of the present disclosure have surprisingly found that the siRNA conjugate of the present disclosure exhibits a significantly improved plasma stability, and further shows higher silencing activity against *RPTOR* mRNA. In some embodiments, the siRNA of the present disclosure may be any one of the siRNAs as listed in Tables 1a, 1b and 1c. By using these siRNAs, the siRNA conjugate of the present disclosure shows much higher silencing activity against *RPTOR* mRNA.

Table 1a. The first siRNAs of the present disclosure

| siRNA No. | SEQ ID NO: | Sequence Direction 5' - 3' |
|---|---|---|
| siRPTORal | 9 | CUGCCAUGGAGUAUCUGAA |
| | 10 | UUCAGAUACUCCAUGGCAGUG |
| siRPTORa2 | 11 | CUGCCAUGGAGUAUCUGAACG |
| | 12 | UUCAGAUACUCCAUGGCAGUG |
| siRPTORa3 | 13 | CACUGCCAUGGAGUAUCUGAA |
| | 14 | UUCAGAUACUCCAUGGCAGUGAC |
| siRPTORal -M1 | 15 | CmUmGmCmCmAmUfGfGfAmGmUmAmUmCmUmGmAmAm |
| | 16 | UmUfCmAmGmAfUmAmCmUmCmCmAmUfGmGfCmAmGmUmGm |
| siRPTORa1 -M2 | 17 | CmUmGmCmCfAmUfGfGfAmGmUmAmUmCmUmGmAmAm |
| | 18 | UmUfCmAmGmAfUmAfCfUmCmCmAmUfGmGfCmAmGmUmGm |
| siRPTORa1 -M3 | 19 | CmUmGmCmCfAmUfGfGfAmGmUmAmUmCmUmGmAmAm |
| | 20 | UmUfCmAmGmAfUmAmCmUmCmCmAmUfGmGfCmAmGmUmGm |
| siRPTORa2 -M1 | 21 | CmUmGmCmCmAmUfGfGfAmGmUmAmUmCmUmGmAmAmCmGm |
| | 22 | UmUfCmAmGmAfUmAmCmUmCmCmAmUfGmGfCmAmGmUmGm |
| siRPTORa2 -M2 | 23 | CmUmGmCmCfAmUfGfGfAmGmUmAmUmCmUmGmAmAmCmGm |
| | 24 | UmUfCmAmGmAfUmAfCfUmCmCmAmUfGmGfCmAmGmUmGm |
| siRPTORa2 -M3 | 25 | CmUmGmCmCfAmUfGfGfAmGmUmAmUmCmUmGmAmAmCmGm |
| | 26 | UmUfCmAmGmAfUmAmCmUmCmCmAmUfGmGfCmAmGmUmGm |
| siRPTORa3 M1 | 27 | CmAmCmUmGmCmCmAmUfGfGfAmGmUmAmUmCmUmGmAmAm |
| | 28 | UmUfCmAmGmAfUmAmCmUmCmCmAmUfGmGfCmAmGmUmGmAmCm |
| siRPTORa3 -M2 | 29 | CmAmCmUmGmCmCfAmUfGfGfAmGmUmAmUmCmUmGmAmAm |
| | 30 | UmUfCmAmGmAfUmAfCfUmCmCmAmUfGmGfCmAmGmUmGmAmCm |
| siRPTORa3 -M3 | 31 | CmAmCmUmGmCmCfAmUfGfGfAmGmUmAmUmCmUmGmAmAm |
| | 32 | UmUfCmAmGmAfUmAmCmUmCmCmAmUfGmGfCmAmGmUmGmAmCm |
| siRPTORa1 | 33 | CmsUmsGmCmCmAmUfGfGfAmGmUmAmUmCmUmGmAmAm |
| -M1S | 34 | UmsUfsCmAmGmAfUmAmCmUmCmCmAmUfGmGfCmAmGmsUmsGm |
| siRPTORa1 -M1X | 35 | CmsUmsGmCmCmAmUfGfGfAmGmUmAmUmCmUmGmsAmsAm |
| | 36 | UmsUfsCmAmGmAfUmAmCmUmCmCmAmUfGmGfCmAmGmsUmsGm |
| siRPTORa1 -M2S | 37 | CmsUmsGmCmCfAmUfGfGfAmGmUmAmUmCmUmGmAmAm |
| | 38 | UmsUfsCmAmGmAfUmAfCfUmCmCmAmUfGmGfCmAmGmsUmsGm |

(continued)

| siRNA No. | SEQ ID NO: | Sequence Direction 5' - 3' |
|---|---|---|
| siRPTORa1-M2X | 39 | CmsUmsGmCmCfAmUfGfGfAmGmUmAmUmCmUmGmsAmsAm |
| | 40 | UmsUfsCmAmGmAfUmAfCfUmCmCmAmUfGmGfCmAmGmsUmsGm |
| siRPTORa1-M3S | 41 | CmsUmsGmCmCfAmUfGfGfAmGmUmAmUmCmUmGmAmAm |
| | 42 | UmsUfsCmAmGmAfUmAmCmUmCmCmAmUfGmGfCmAmGmsUmsGm |
| siRPTORa1-M3X | 43 | CmsUmsGmCmCfAmUfGfGfAmGmUmAmUmCmUmGmsAmsAm |
| | 44 | UmsUfsCmAmGmAfUmAmCmUmCmCmAmUfGmGfCmAmGmsUmsGm |
| siRPTORa2-M1S | 45 | CmsUmsGmCmCmAmUfGfGfAmGmUmAmUmCmUmGmAmAmCmGm |
| | 46 | UmsUfsCmAmGmAfUmAmCmUmCmCmAmUfGmGfCmAmGmsUmsGm |
| siRPTORa2-M1X | 47 | CmsUmsGmCmCmAmUfGfGfAmGmUmAmUmCmUmGmAmAmsCmsGm |
| | 48 | UmsUfsCmAmGmAfUmAmCmUmCmCmAmUfGmGfCmAmGmsUmsGm |
| siRPTORa2-M2S | 49 | CmsUmsGmCmCfAmUfGfGfAmGmUmAmUmCmUmGmAmAmCmGm |
| | 50 | UmsUfsCmAmGmAfUmAfCfUmCmCmAmUfGmGfCmAmGmsUmsGm |
| siRPTORa2-M2X | 51 | CmsUmsGmCmCfAmUfGfGfAmGmUmAmUmCmUmGmAmAmsCmsGm |
| | 52 | UmsUfsCmAmGmAfUmAfCfUmCmCmAmUfGmGfCmAmGmsUmsGm |
| siRPTORa2-M3S | 53 | CmsUmsGmCmCfAmUfGfGfAmGmUmAmUmCmUmGmAmAmCmGm |
| | 54 | UmsUfsCmAmGmAfUmAmCmUmCmCmAmUfGmGfCmAmGmsUmsGm |
| siRPTORa2-M3X | 55 | CmsUmsGmCmCfAmUfGfGfAmGmUmAmUmCmUmGmAmAmsCmsGm |
| | 56 | UmsUfsCmAmGmAfUmAmCmUmCmCmAmUfGmGfCmAmGmsUmsGm |
| siRPTORa3-M1S | 57 | CmsAmsCmUmGmCmCmAmUfGfGfAmGmUmAmUmCmUmGmAmAm |
| | 58 | UmsUfsCmAmGmAfUmAmCmUmCmCmAmUfGmGfCmAmGmUmGmsAmsCm |
| siRPTORa3-M1X | 59 | CmsAmsCmUmGmCmCmAmUfGfGfAmGmUmAmUmCmUmGmsAmsAm |
| | 60 | UmsUfsCmAmGmAfUmAmCmUmCmCmAmUfGmGfCmAmGmUmGmsAmsCm |
| siRPTORa3-M2S | 61 | CmsAmsCmUmGmCmCfAmUfGfGfAmGmUmAmUmCmUmGmAmAm |
| | 62 | UmsUfsCmAmGmAfUmAfCfUmCmCmAmUfGmGfCmAmGmUmGmsAmsCm |
| siRPTORa3-M2X | 63 | CmsAmsCmUmGmCmCfAmUfGfGfAmGmUmAmUmCmUmGmsAmsAm |
| | 64 | UmsUfsCmAmGmAfUmAfCfUmCmCmAmUfGmGfCmAmGmUmGmsAmsCm |
| siRPTORa3-M3S | 65 | CmsAmsCmUmGmCmCfAmUfGfGfAmGmUmAmUmCmUmGmAmAm |
| | 66 | UmsUfsCmAmGmAfUmAmCmUmCmCmAmUfGmGfCmAmGmUmGmsAmsCm |
| siRPTORa3-M3X | 67 | CmsAmsCmUmGmCmCfAmUfGfGfAmGmUmAmUmCmUmGmsAmsAm |
| | 68 | UmsUfsCmAmGmAfUmAmCmUmCmCmAmUfGmGfCmAmGmUmGmsAmsCm |
| siRPTORa1-M1P1 | 69 | CmUmGmCmCmAmUfGfGfAmGmUmAmUmCmUmGmAmAm |
| | 70 | P1UmUfCmAmGmAfUmAmCmUmCmCmAmUfGmGfCmAmGmUmGm |

(continued)

| siRNA No. | SEQ ID NO: | Sequence Direction 5' - 3' |
|---|---|---|
| siRPTORa1 -M2P1 | 71 | CmUmGmCmCfAmUfGfGfAmGmUmAmUmCmUmGmAmAm |
| | 72 | P1UmUfCmAmGmAfUmAfCfUmCmCmAmUfGmGfCmAmGmUmGm |
| siRPTORa1 -M3P1 | 73 | CmUmGmCmCfAmUfGfGfAmGmUmAmUmCmUmGmAmAm |
| | 74 | P1UmUfCmAmGmAfUmAmCmUmCmCmAmUfGmGfCmAmGmUmGm |
| siRPTORa2 -M1P1 | 75 | CmUmGmCmCmAmUfGfGfAmGmUmAmUmCmUmGmAmAmCmGm |
| | 76 | P1UmUfCmAmGmAfUmAmCmUmCmCmAmUfGmGfCmAmGmUmGm |
| siRPTORa2 -M2P1 | 77 | CmUmGmCmCfAmUfGfGfAmGmUmAmUmCmUmGmAmAmCmGm |
| | 78 | P1UmUfCmAmGmAfUmAfCfUmCmCmAmUfGmGfCmAmGmUmGm |
| siRPTORa2 -M3P1 | 79 | CmUmGmCmCfAmUfGfGfAmGmUmAmUmCmUmGmAmAmCmGm |
| | 80 | P1UmUfCmAmGmAfUmAmCmUmCmCmAmUfGmGfCmAmGmUmGm |
| siRPTORa3 -M1P1 | 81 | CmAmCmUmGmCmCmAmUfGfGfAmGmUmAmUmCmUmGmAmAm |
| | 82 | P1UmUfCmAmGmAfUmAmCmUmCmCmAmUfGmGfCmAmGmUmGmAm Cm |
| siRPTORa3 -M2P1 | 83 | CmAmCmUmGmCmCfAmUfGfGfAmGmUmAmUmCmUmGmAmAm |
| | 84 | P1UmUfCmAmGmAfUmAfCfUmCmCmAmUfGmGfCmAmGmUmGmAmCm |
| siRPTORa3 -M3P1 | 85 | CmAmCmUmGmCmCfAmUfGfGfAmGmUmAmUmCmUmGmAmAm |
| | 86 | P1UmUfCmAmGmAfUmAmCmUmCmCmAmUfGmGfCmAmGmUmGmAm Cm |
| siRPTORa1 -M1SP1 | 87 | CmsUmsGmCmCmAmUfGfGfAmGmUmAmUmCmUmGmAmAm |
| | 88 | P1UmsUfsCmAmGmAfUmAmCmUmCmCmAmUfGmGfCmAmGmsUmsGm |
| siRPTORa1 -M1XP1 | 89 | CmsUmsGmCmCmAmUfGfGfAmGmUmAmUmCmUmGmsAmsAm |
| | 90 | P1UmsUfsCmAmGmAfUmAmCmUmCmCmAmUfGmGfCmAmGmsUmsGm |
| siRPTORa1 -M2SP1 | 91 | CmsUmsGmCmCfAmUfGfGfAmGmUmAmUmCmUmGmAmAm |
| | 92 | P1UmsUfsCmAmGmAfUmAfCfUmCmCmAmUfGmGfCmAmGmsUmsGm |
| siRPTORa1 -M2XP1 | 93 | CmsUmsGmCmCfAmUfGfGfAmGmUmAmUmCmUmGmsAmsAm |
| | 94 | P1UmsUfsCmAmGmAfUmAfCfUmCmCmAmUfGmGfCmAmGmsUmsGm |
| siRPTORa1 -M3SP1 | 95 | CmsUmsGmCmCfAmUfGfGfAmGmUmAmUmCmUmGmAmAm |
| | 96 | P1UmsUfsCmAmGmAfUmAmCmUmCmCmAmUfGmGfCmAmGmsUmsGm |
| siRPTORa1 -M3XP1 | 97 | CmsUmsGmCmCfAmUfGfGfAmGmUmAmUmCmUmGmsAmsAm |
| | 98 | P1UmsUfsCmAmGmAfUmAmCmUmCmCmAmUfGmGfCmAmGmsUmsGm |
| siRPTORa2 -M1SP1 | 99 | CmsUmsGmCmCmAmUfGfGfAmGmUmAmUmCmUmGmAmAmCmGm |
| | 100 | P1UmsUfsCmAmGmAfUmAmCmUmCmCmAmUfGmGfCmAmGmsUmsGm |
| siRPTORa2 -M1XP1 | 101 | CmsUmsGmCmCmAmUfGfGfAmGmUmAmUmCmUmGmAmAmsCmsGm |
| | 102 | P1UmsUfsCmAmGmAfUmAmCmUmCmCmAmUfGmGfCmAmGmsUmsGm |
| siRPTORa2 -M2SP1 | 103 | CmsUmsGmCmCfAmUfGfGfAmGmUmAmUmCmUmGmAmAmCmGm |
| | 104 | P1UmsUfsCmAmGmAfUmAfCfUmCmCmAmUfGmGfCmAmGmsUmsGm |
| siRPTORa2 -M2XP1 | 105 | CmsUmsGmCmCfAmUfGfGfAmGmUmAmUmCmUmGmAmAmsCmsGm |
| | 106 | P1UmsUfsCmAmGmAfUmAfCfUmCmCmAmUfGmGfCmAmGmsUmsGm |

(continued)

| siRNA No. | SEQ ID NO: | Sequence Direction 5' - 3' |
|---|---|---|
| siRPTORa2 -M3SP1 | 107 | CmsUmsGmCmCfAmUfGfGfAmGmUmAmUmCmUmGmAmAmCmGm |
| | 108 | P1UmsUfsCmAmGmAfUmAmCmUmCmCmAmUfGmGfCmAmGmsUmsGm |
| siRPTORa2 -M3XP1 | 109 | CmsUmsGmCmCfAmUfGfGfAmGmUmAmUmCmUmGmAmAmsCmsGm |
| | 110 | P1UmsUfsCmAmGmAfUmAmCmUmCmCmAmUfGmGfCmAmGmsUmsGm |
| siRPTORa3 -M1SP1 | 111 | CmsAmsCmUmGmCmCmAmUfGfGfAmGmUmAmUmCmUmGmAmAm |
| | 112 | P1UmsUfsCmAmGmAfUmAmCmUmCmCmAmUfGmGfCmAmGmUmGmsAmsCm |
| siRPTORa3 -M1XP1 | 113 | CmsAmsCmUmGmCmCmAmUfGfGfAmGmUmAmUmCmUmGmsAmsAm |
| | 114 | P1UmsUfsCmAmGmAfUmAmCmUmCmCmAmUfGmGfCmAmGmUmGmsAmsCm |
| siRPTORa3 -M2SP1 | 115 | CmsAmsCmUmGmCmCfAmUfGfGfAmGmUmAmUmCmUmGmAmAm |
| | 116 | P1UmsUfsCmAmGmAfUmAfCfUmCmCmAmUfGmGfCmAmGmUmGmsAmsCm |
| siRPTORa3 -M2XP1 | 117 | CmsAmsCmUmGmCmCfAmUfGfGfAmGmUmAmUmCmUmGmsAmsAm |
| | 118 | P1UmsUfsCmAmGmAfUmAfCfUmCmCmAmUfGmGfCmAmGmUmGmsAmsCm |
| siRPTORa3 -M3SP1 | 119 | CmsAmsCmUmGmCmCfAmUfGfGfAmGmUmAmUmCmUmGmAmAm |
| | 120 | P1UmsUfsCmAmGmAfUmAmCmUmCmCmAmUfGmGfCmAmGmUmGmsAmsCm |
| siRPTORa3 -M3XP1 | 121 | CmsAmsCmUmGmCmCfAmUfGfGfAmGmUmAmUmCmUmGmsAmsAm |
| | 122 | P1UmsUfsCmAmGmAfUmAmCmUmCmCmAmUfGmGfCmAmGmUmGmsAmsCm |
| siRPTORa1 -T1S | 373 | CmsUmsGmCmCmAmUfGfGfAmGmUmAmUmCmUmGmAmAm |
| | 374 | UmsUfsCmoeAmGmAfUmAmCmUmCmCmAmUfGmGfCmAmGmsUmsGm |
| siRPTORa1 -T2S | 375 | CmsUmsGmCmCmAmUfGfGfAmGmUmAmUmCmUmGmAmAm |
| | 376 | UmsUfsCmAmGmoeAfUmAmCmUmCmCmAmUfGmGfCmAmGmsUmsGm |

Table 1b. The second siRNAs of the present disclosure

| siRNA No. | SEQ ID NO: | Sequence Direction 5' - 3' |
|---|---|---|
| siRPTORb1 | 131 | ACAACAUCAAGUACUACGA |
| | 132 | UCGUAGUACUUGAUGUUGUUG |
| siRPTORb2 | 133 | ACAACAUCAAGUACUACGACG |
| | 134 | UCGUAGUACUUGAUGUUGUUG |
| siRPTORb3 | 135 | GUACAACAUCAAGUACUACGA |
| | 136 | UCGUAGUACUUGAUGUUGUUGAU |
| siRPTORb1 -mi | 137 | AmCmAmAmCmAmUfCfAfAmGmUmAmCmUmAmCmGmAm |
| | 138 | UmCfGmUmAmGfUmAmCmUmUmGmAmUfGmUfUmGmUmUmGm |
| siRPTORb1 -M2 | 139 | AmCmAmAmCfAmUfCfAfAmGmUmAmCmUmAmCmGmAm |
| | 140 | UmCfGmUmAmGfUmAfCfUmUmGmAmUfGmUfUmGmUmUmGm |

(continued)

| siRNA No. | SEQ ID NO: | Sequence Direction 5' - 3' |
|---|---|---|
| siRPTORb1-M3 | 141 | AmCmAmAmCfAmUfCfAfAmGmUmAmCmUmAmCmGmAm |
| | 142 | UmCfGmUmAmGfUmAmCmUmUmGmAmUfGmUfUmGmUmUmGm |
| siRPTORb2-mi | 143 | AmCmAmAmCmAmUfCfAfAmGmUmAmCmUmAmCmGmAmCmGm |
| | 144 | UmCfGmUmAmGfUmAmCmUmUmGmAmUfGmUfUmGmUmUmGm |
| siRPTORb2-M2 | 145 | AmCmAmAmCfAmUfCfAfAmGmUmAmCmUmAmCmGmAmCmGm |
| | 146 | UmCfGmUmAmGfUmAfCfUmUmGmAmUfGmUfUmGmUmUmGm |
| siRPTORb2-M3 | 147 | AmCmAmAmCfAmUfCfAfAmGmUmAmCmUmAmCmGmAmCmGm |
| | 148 | UmCfGmUmAmGfUmAmCmUmUmGmAmUfGmUfUmGmUmUmGm |
| siRPTORb3-mi | 149 | CmAmAmCmAmAmCmAmUfCfAfAmGmUmAmCmUmAmCmGmAm |
| | 150 | UmCfGmUmAmGfUmAmCmUmUmGmAmUfGmUfUmGmUmUmGmAmUm |
| siRPTORb3-M2 | 151 | CmAmAmCmAmAmCfAmUfCfAfAmGmUmAmCmUmAmCmGmAm |
| | 152 | UmCfGmUmAmGfUmAfCfUmUmGmAmUfGmUfUmGmUmUmGmAmUm |
| siRPTORb3-M3 | 153 | CmAmAmCmAmAmCfAmUfCfAfAmGmUmAmCmUmAmCmGmAm |
| | 154 | UmCfGmUmAmGfUmAmCmUmUmGmAmUfGmUfUmGmUmUmGmAmUm |
| siRPTORb1-M1S | 155 | AmsCmsAmAmCmAmUfCfAfAmGmUmAmCmUmAmCmGmAm |
| | 156 | UmsCfsGmUmAmGfUmAmCmUmUmGmAmUfGmUfUmGmUmsUmsGm |
| siRPTORb1-M1X | 157 | AmsCmsAmAmCmAmUfCfAfAmGmUmAmCmUmAmCmsGmsAm |
| | 158 | UmsCfsGmUmAmGfUmAmCmUmUmGmAmUfGmUfUmGmUmsUmsGm |
| siRPTORb1-M2S | 159 | AmsCmsAmAmCfAmUfCfAfAmGmUmAmCmUmAmCmGmAm |
| | 160 | UmsCfsGmUmAmGfUmAfCfUmUmGmAmUfGmUfUmGmUmsUmsGm |
| siRPTORb1-M2X | 161 | AmsCmsAmAmCfAmUfCfAfAmGmUmAmCmUmAmCmsGmsAm |
| | 162 | UmsCfsGmUmAmGfUmAfCfUmUmGmAmUfGmUfUmGmUmsUmsGm |
| siRPTORb1-M3S | 163 | AmsCmsAmAmCfAmUfCfAfAmGmUmAmCmUmAmCmGmAm |
| | 164 | UmsCfsGmUmAmGfUmAmCmUmUmGmAmUfGmUfUmGmUmsUmsGm |
| siRPTORb1-M3X | 165 | AmsCmsAmAmCfAmUfCfAfAmGmUmAmCmUmAmCmsGmsAm |
| | 166 | UmsCfsGmUmAmGfUmAmCmUmUmGmAmUfGmUfUmGmUmsUmsGm |
| siRPTORb2-M1S | 167 | AmsCmsAmAmCmAmUfCfAfAmGmUmAmCmUmAmCmGmAmCmGm |
| | 168 | UmsCfsGmUmAmGfUmAmCmUmUmGmAmUfGmUfUmGmUmsUmsGm |
| siRPTORb2 | 169 | AmsCmsAmAmCmAmUfCfAfAmGmUmAmCmUmAmCmGmAmsCmsGm |
| -M1X | 170 | UmsCfsGmUmAmGfUmAmCmUmUmGmAmUfGmUfUmGmUmsUmsGm |
| siRPTORb2-M2S | 171 | AmsCmsAmAmCfAmUfCfAfAmGmUmAmCmUmAmCmGmAmCmGm |
| | 172 | UmsCfsGmUmAmGfUmAfCfUmUmGmAmUfGmUfUmGmUmsUmsGm |
| siRPTORb2-M2X | 173 | AmsCmsAmAmCfAmUfCfAfAmGmUmAmCmUmAmCmGmAmsCmsGm |
| | 174 | UmsCfsGmUmAmGfUmAfCfUmUmGmAmUfGmUfUmGmUmsUmsGm |
| siRPTORb2-M3S | 175 | AmsCmsAmAmCfAmUfCfAfAmGmUmAmCmUmAmCmGmAmCmGm |
| | 176 | UmsCfsGmUmAmGfUmAmCmUmUmGmAmUfGmUfUmGmUmsUmsGm |

(continued)

| siRNA No. | SEQ ID NO: | Sequence Direction 5' - 3' |
|---|---|---|
| siRPTORb2 -M3X | 177 | AmsCmsAmAmCfAmUfCfAfAmGmUmAmCmUmAmCmGmAmsCmsGm |
| | 178 | UmsCfsGmUmAmGfUmAmCmUmUmGmAmUfGmUfUmGmUmsUmsGm |
| siRPTORb3 -M1S | 179 | CmsAmsAmCmAmAmCmAmUfCfAfAmGmUmAmCmUmAmCmGmAm |
| | 180 | UmsCfsGmUmAmGfUmAmCmUmUmGmAmUfGmUfUmGmUmUmGmsAmsUm |
| siRPTORb3 -M1X | 181 | CmsAmsAmCmAmAmCmAmUfCfAfAmGmUmAmCmUmAmCmsGmsAm |
| | 182 | UmsCfsGmUmAmGfUmAmCmUmUmGmAmUfGmUfUmGmUmUmGmsAmsUm |
| siRPTORb3 -M2S | 183 | CmsAmsAmCmAmAmCfAmUfCfAfAmGmUmAmCmUmAmCmGmAm |
| | 184 | UmsCfsGmUmAmGfUmAfCfUmUmGmAmUfGmUfUmGmUmUmGmsAmsUm |
| siRPTORb3 -M2X | 185 | CmsAmsAmCmAmAmCfAmUfCfAfAmGmUmAmCmUmAmCmsGmsAm |
| | 186 | UmsCfsGmUmAmGfUmAfCfUmUmGmAmUfGmUfUmGmUmUmGmsAmsUm |
| siRPTORb3 -M3S | 187 | CmsAmsAmCmAmAmCfAmUfCfAfAmGmUmAmCmUmAmCmGmAm |
| | 188 | UmsCfsGmUmAmGfUmAmCmUmUmGmAmUfGmUfUmGmUmUmGmsAmsUm |
| siRPTORb3 -M3X | 189 | CmsAmsAmCmAmAmCfAmUfCfAfAmGmUmAmCmUmAmCmsGmsAm |
| | 190 | UmsCfsGmUmAmGfUmAmCmUmUmGmAmUfGmUfUmGmUmUmGmsAmsUm |
| siRPTORb1 -M1P1 | 191 | AmCmAmAmCmAmUfCfAfAmGmUmAmCmUmAmCmGmAm |
| | 192 | P1UmCfGmUmAmGfUmAmCmUmUmGmAmUfGmUfUmGmUmUmGm |
| siRPTORbl -M2P1 | 193 | AmCmAmAmCfAmUfCfAfAmGmUmAmCmUmAmCmGmAm |
| | 194 | P1UmCfGmUmAmGfUmAfCfUmUmGmAmUfGmUfUmGmUmUmGm |
| siRPTORbl -M3P1 | 195 | AmCmAmAmCfAmUfCfAfAmGmUmAmCmUmAmCmGmAm |
| | 196 | P1UmCfGmUmAmGfUmAmCmUmUmGmAmUfGmUfUmGmUmUmGm |
| siRPTORb2 -M1P1 | 197 | AmCmAmAmCmAmUfCfAfAmGmUmAmCmUmAmCmGmAmCmGm |
| | 198 | P1UmCfGmUmAmGfUmAmCmUmUmGmAmUfGmUfUmGmUmUmGm |
| siRPTORb2 -M2P1 | 199 | AmCmAmAmCfAmUfCfAfAmGmUmAmCmUmAmCmGmAmCmGm |
| | 200 | P1UmCfGmUmAmGfUmAfCfUmUmGmAmUfGmUfUmGmUmUmGm |
| siRPTORb2 -M3P1 | 201 | AmCmAmAmCfAmUfCfAfAmGmUmAmCmUmAmCmGmAmCmGm |
| | 202 | P1UmCfGmUmAmGfUmAmCmUmUmGmAmUfGmUfUmGmUmUmGm |
| siRPTORb3 -M1P1 | 203 | CmAmAmCmAmAmCmAmUfCfAfAmGmUmAmCmUmAmCmGmAm |
| | 204 | P1UmCfGmUmAmGfUmAmCmUmUmGmAmUfGmUfUmGmUmUmGmAmUm |
| siRPTORb3 -M2P1 | 205 | CmAmAmCmAmAmCfAmUfCfAfAmGmUmAmCmUmAmCmGmAm |
| | 206 | P1UmCfGmUmAmGfUmAfCfUmUmGmAmUfGmUfUmGmUmUmGmAmUm |

(continued)

| siRNA No. | SEQ ID NO: | Sequence Direction 5' - 3' |
|---|---|---|
| siRPTORb3 -M3P1 | 207 | CmAmAmCmAmAmCfAmUfCfAfAmGmUmAmCmUmAmCmGmAm |
| | 208 | P1UmCfGmUmAmGfUmAmCmUmUmGmAmUfGmUfUmGmUmUmGmAmUm |
| siRPTORbl -M1SP1 | 209 | AmsCmsAmAmCmAmUfCfAfAmGmUmAmCmUmAmCmGmAm |
| | 210 | P1UmsCfsGmUmAmGfUmAmCmUmUmGmAmUfGmUfUmGmUmsUmsGm |
| siRPTORb1 -M1XP1 | 211 | AmsCmsAmAmCmAmUfCfAfAmGmUmAmCmUmAmCmsGmsAm |
| | 212 | P1UmsCfsGmUmAmGfUmAmCmUmUmGmAmUfGmUfUmGmUmsUmsGm |
| siRPTORb1 -M2SP1 | 213 | AmsCmsAmAmCfAmUfCfAfAmGmUmAmCmUmAmCmGmAm |
| | 214 | P1UmsCfsGmUmAmGfUmAfCfUmUmGmAmUfGmUfUmGmUmsUmsGm |
| siRPTORb1 -M2XP1 | 215 | AmsCmsAmAmCfAmUfCfAfAmGmUmAmCmUmAmCmsGmsAm |
| | 216 | P1UmsCfsGmUmAmGfUmAfCfUmUmGmAmUfGmUfUmGmUmsUmsGm |
| siRPTORb1 -M3SP1 | 217 | AmsCmsAmAmCfAmUfCfAfAmGmUmAmCmUmAmCmGmAm |
| | 218 | P1UmsCfsGmUmAmGfUmAmCmUmUmGmAmUfGmUfUmGmUmsUmsGm |
| siRPTORb1 -M3XP1 | 219 | AmsCmsAmAmCfAmUfCfAfAmGmUmAmCmUmAmCmsGmsAm |
| | 220 | P1UmsCfsGmUmAmGfUmAmCmUmUmGmAmUfGmUfUmGmUmsUmsGm |
| siRPTORb2 -M1SP1 | 221 | AmsCmsAmAmCmAmUfCfAfAmGmUmAmCmUmAmCmGmAmCmGm |
| | 222 | P1UmsCfsGmUmAmGfUmAmCmUmUmGmAmUfGmUfUmGmUmsUmsGm |
| siRPTORb2 -M1XP1 | 223 | AmsCmsAmAmCmAmUfCfAfAmGmUmAmCmUmAmCmGmAmsCmsGm |
| | 224 | P1UmsCfsGmUmAmGfUmAmCmUmUmGmAmUfGmUfUmGmUmsUmsGm |
| siRPTORb2 -M2SP1 | 225 | AmsCmsAmAmCfAmUfCfAfAmGmUmAmCmUmAmCmGmAmCmGm |
| | 226 | P1UmsCfsGmUmAmGfUmAfCfUmUmGmAmUfGmUfUmGmUmsUmsGm |
| siRPTORb2 -M2XP1 | 227 | AmsCmsAmAmCfAmUfCfAfAmGmUmAmCmUmAmCmGmAmsCmsGm |
| | 228 | P1UmsCfsGmUmAmGfUmAfCfUmUmGmAmUfGmUfUmGmUmsUmsGm |
| siRPTORb2 -M3SP1 | 229 | AmsCmsAmAmCfAmUfCfAfAmGmUmAmCmUmAmCmGmAmCmGm |
| | 230 | P1UmsCfsGmUmAmGfUmAmCmUmUmGmAmUfGmUfUmGmUmsUmsGm |
| siRPTORb2 -M3XP1 | 231 | AmsCmsAmAmCfAmUfCfAfAmGmUmAmCmUmAmCmGmAmsCmsGm |
| | 232 | P1UmsCfsGmUmAmGfUmAmCmUmUmGmAmUfGmUfUmGmUmsUmsGm |
| siRPTORb3 -M1SP1 | 233 | CmsAmsAmCmAmAmCmAmUfCfAfAmGmUmAmCmUmAmCmGmAm |
| | 234 | P1UmsCfsGmUmAmGfUmAmCmUmUmGmAmUfGmUfUmGmUmUmGmsAmsUm |
| siRPTORb3 -M1XP1 | 235 | CmsAmsAmCmAmAmCmAmUfCfAfAmGmUmAmCmUmAmCmsGmsAm |
| | 236 | P1UmsCfsGmUmAmGfUmAmCmUmUmGmAmUfGmUfUmGmUmUmGmsAmsUm |
| siRPTORb3 -M2SP1 | 237 | CmsAmsAmCmAmAmCfAmUfCfAfAmGmUmAmCmUmAmCmGmAm |
| | 238 | P1UmsCfsGmUmAmGfUmAfCfUmUmGmAmUfGmUfUmGmUmUmGmsAmsUm |
| siRPTORb3 -M2XP1 | 239 | CmsAmsAmCmAmAmCfAmUfCfAfAmGmUmAmCmUmAmCmsGmsAm |
| | 240 | P1UmsCfsGmUmAmGfUmAfCfUmUmGmAmUfGmUfUmGmUmUmGmsAmsUm |

(continued)

| siRNA No. | SEQ ID NO: | Sequence Direction 5' - 3' |
|---|---|---|
| siRPTORb3 -M3SP1 | 241 | CmsAmsAmCmAmAmCfAmUfCfAfAmGmUmAmCmUmAmCmGmAm |
| | 242 | P1UmsCfsGmUmAmGfUmAmCmUmUmGmAmUfGmUfUmGmUmUmGmsAmsUm |
| siRPTORb3 -M3XP1 | 243 | CmsAmsAmCmAmAmCfAmUfCfAfAmGmUmAmCmUmAmCmsGmsAm |
| | 244 | P1UmsCfsGmUmAmGfUmAmCmUmUmGmAmUfGmUfUmGmUmUmGmsAmsUm |
| siRPTORb1 -T1S | 377 | AmsCmsAmAmCmAmUfCfAfAmGmUmAmCmUmAmCmGmAm |
| | 378 | UmsCfsGmoeUmAmGfUmAmCmUmUmGmAmUfGmUfUmGmUmsUmsGm |
| siRPTORb1 -T2S | 379 | AmsCmsAmAmCmAmUfCfAfAmGmUmAmCmUmAmCmGmAm |
| | 380 | UmsCfsGmUmAmoeGfUmAmCmUmUmGmAmUfGmUfUmGmUmsUmsGm |

Table 1c. The third siRNAs of the present disclosure

| siRNA No. | SEQ ID NO: | Sequence Direction 5' - 3' |
|---|---|---|
| siRPTORc1 | 253 | CGACUACUACAUCUCCGUG |
| | 254 | CACGGAGAUGUAGUAGUCGUU |
| siRPTORc2 | 255 | CGACUACUACAUCUCCGUGUA |
| | 256 | CACGGAGAUGUAGUAGUCGUU |
| siRPTORc3 | 257 | CGCGACUACUACAUCUCCGUG |
| | 258 | CACGGAGAUGUAGUAGUCGUUGC |
| siRPTORc1 -M1 | 259 | CmGmAmCmUmAmCfUfAfCmAmUmCmUmCmCmGmUmGm |
| | 260 | CmAfCmGmGmAfGmAmUmGmUmAmGmUfAmGfUmCmGmUmUm |
| siRPTORc1 -M2 | 261 | CmGmAmCmUfAmCfUfAfCmAmUmCmUmCmCmGmUmGm |
| | 262 | CmAfCmGmGmAfGmAfUfGmUmAmGmUfAmGfUmCmGmUmUm |
| siRPTORc1 -M3 | 263 | CmGmAmCmUfAmCfUfAfCmAmUmCmUmCmCmGmUmGm |
| | 264 | CmAfCmGmGmAfGmAmUmGmUmAmGmUfAmGfUmCmGmUmUm |
| siRPTORc2 -M1 | 265 | CmGmAmCmUmAmCfUfAfCmAmUmCmUmCmCmGmUmGmUmAm |
| | 266 | CmAfCmGmGmAfGmAmUmGmUmAmGmUfAmGfUmCmGmUmUm |
| siRPTORc2 -M2 | 267 | CmGmAmCmUfAmCfUfAfCmAmUmCmUmCmCmGmUmGmUmAm |
| | 268 | CmAfCmGmGmAfGmAfUfGmUmAmGmUfAmGfUmCmGmUmUm |
| siRPTORc2 -M3 | 269 | CmGmAmCmUfAmCfUfAfCmAmUmCmUmCmCmGmUmGmUmAm |
| | 270 | CmAfCmGmGmAfGmAmUmGmUmAmGmUfAmGfUmCmGmUmUm |
| siRPTORc3 -M1 | 271 | AmAmCmGmAmCmUmAmCfUfAfCmAmUmCmUmCmCmGmUmGm |
| | 272 | CmAfCmGmGmAfGmAmUmGmUmAmGmUfAmGfUmCmGmUmUmGmCm |
| siRPTORc3 -M2 | 273 | AmAmCmGmAmCmUfAmCfUfAfCmAmUmCmUmCmCmGmUmGm |
| | 274 | CmAfCmGmGmAfGmAfUfGmUmAmGmUfAmGfUmCmGmUmUmGmCm |
| siRPTORc3 -M3 | 275 | AmAmCmGmAmCmUfAmCfUfAfCmAmUmCmUmCmCmGmUmGm |
| | 276 | CmAfCmGmGmAfGmAmUmGmUmAmGmUfAmGfUmCmGmUmUmGmCm |

(continued)

| siRNA No. | SEQ ID NO: | Sequence Direction 5' - 3' |
|---|---|---|
| siRPTORc1 -M1S | 277 | CmsGmsAmCmUmAmCfUfAfCmAmUmCmUmCmCmGmUmGm |
| | 278 | CmsAfsCmGmGmAfGmAmUmGmUmAmGmUfAmGfUmCmGmsUmsUm |
| siRPTORc1 -M1X | 279 | CmsGmsAmCmUmAmCfUfAfCmAmUmCmUmCmCmGmsUmsGm |
| | 280 | CmsAfsCmGmGmAfGmAmUmGmUmAmGmUfAmGfUmCmGmsUmsUm |
| siRPTORc1 -M2S | 281 | CmsGmsAmCmUfAmCfUfAfCmAmUmCmUmCmCmGmUmGm |
| | 282 | CmsAfsCmGmGmAfGmAfUfGmUmAmGmUfAmGfUmCmGmsUmsUm |
| siRPTORcl -M2X | 283 | CmsGmsAmCmUfAmCfUfAfCmAmUmCmUmCmCmGmsUmsGm |
| | 284 | CmsAfsCmGmGmAfGmAfUfGmUmAmGmUfAmGfUmCmGmsUmsUm |
| siRPTORcl -M3S | 285 | CmsGmsAmCmUfAmCfUfAfCmAmUmCmUmCmCmGmUmGm |
| | 286 | CmsAfsCmGmGmAfGmAmUmGmUmAmGmUfAmGfUmCmGmsUmsUm |
| siRPTORc1 -M3X | 287 | CmsGmsAmCmUfAmCfUfAfCmAmUmCmUmCmCmGmsUmsGm |
| | 288 | CmsAfsCmGmGmAfGmAmUmGmUmAmGmUfAmGfUmCmGmsUmsUm |
| siRPTORc2 -M1S | 289 | CmsGmsAmCmUmAmCfUfAfCmAmUmCmUmCmCmGmUmGmUmAm |
| | 290 | CmsAfsCmGmGmAfGmAmUmGmUmAmGmUfAmGfUmCmGmsUmsUm |
| siRPTORc2- M1X | 291 | CmsGmsAmCmUmAmCfUfAfCmAmUmCmUmCmCmGmUmGmsUmsAm |
| | 292 | CmsAfsCmGmGmAfGmAmUmGmUmAmGmUfAmGfUmCmGmsUmsUm |
| siRPTORc2 -M2S | 293 | CmsGmsAmCmUfAmCfUfAfCmAmUmCmUmCmCmGmUmGmUmAm |
| | 294 | CmsAfsCmGmGmAfGmAfUfGmUmAmGmUfAmGfUmCmGmsUmsUm |
| siRPTORc2 -M2X | 295 | CmsGmsAmCmUfAmCfUfAfCmAmUmCmUmCmCmGmUmGmsUmsAm |
| | 296 | CmsAfsCmGmGmAfGmAfUfGmUmAmGmUfAmGfUmCmGmsUmsUm |
| siRPTORc2 -M3S | 297 | CmsGmsAmCmUfAmCfUfAfCmAmUmCmUmCmCmGmUmGmUmAm |
| | 298 | CmsAfsCmGmGmAfGmAmUmGmUmAmGmUfAmGfUmCmGmsUmsUm |
| siRPTORc2 -M3X | 299 | CmsGmsAmCmUfAmCfUfAfCmAmUmCmUmCmCmGmUmGmsUmsAm |
| | 300 | CmsAfsCmGmGmAfGmAmUmGmUmAmGmUfAmGfUmCmGmsUmsUm |
| siRPTORc3 -M1S | 301 | AmsAmsCmGmAmCmUmAmCfUfAfCmAmUmCmUmCmCmGmUmGm |
| | 302 | CmsAfsCmGmGmAfGmAmUmGmUmAmGmUfAmGfUmCmGmUmUmsGmsCm |
| siRPTORc3 -M1X | 303 | AmsAmsCmGmAmCmUmAmCfUfAfCmAmUmCmUmCmCmGmsUmsGm |
| | 304 | CmsAfsCmGmGmAfGmAmUmGmUmAmGmUfAmGfUmCmGmUmUmsGmsCm |
| siRPTORc3 -M2S | 305 | AmsAmsCmGmAmCmUfAmCfUfAfCmAmUmCmUmCmCmGmUmGm |
| | 306 | CmsAfsCmGmGmAfGmAfUfGmUmAmGmUfAmGfUmCmGmUmUmsGmsCm |
| siRPTORc3 -M2X | 307 | AmsAmsCmGmAmCmUfAmCfUfAfCmAmUmCmUmCmCmGmsUmsGm |
| | 308 | CmsAfsCmGmGmAfGmAfUfGmUmAmGmUfAmGfUmCmGmUmUmsGmsCm |
| siRPTORc3 -M3S | 309 | AmsAmsCmGmAmCmUfAmCfUfAfCmAmUmCmUmCmCmGmUmGm |
| | 310 | CmsAfsCmGmGmAfGmAmUmGmUmAmGmUfAmGfUmCmGmUmUmsGmsCm |
| siRPTORc3 -M3X | 311 | AmsAmsCmGmAmCmUfAmCfUfAfCmAmUmCmUmCmCmGmsUmsGm |
| | 312 | CmsAfsCmGmGmAfGmAmUmGmUmAmGmUfAmGfUmCmGmUmUmsGmsCm |
| siRPTORc1 -M1P1 | 313 | CmGmAmCmUmAmCfUfAfCmAmUmCmUmCmCmGmUmGm |
| | 314 | P1CmAfCmGmGmAfGmAmUmGmUmAmGmUfAmGfUmCmGmUmUm |

(continued)

| siRNA No. | SEQ ID NO: | Sequence Direction 5' - 3' |
|---|---|---|
| siRPTORc1 -M2P1 | 315 | CmGmAmCmUfAmCfUfAfCmAmUmCmUmCmCmGmUmGm |
| | 316 | P1CmAfCmGmGmAfGmAfUfGmUmAmGmUfAmGfUmCmGmUmUm |
| siRPTORc1 -M3P1 | 317 | CmGmAmCmUfAmCfUfAfCmAmUmCmUmCmCmGmUmGm |
| | 318 | P1CmAfCmGmGmAfGmAmUmGmUmAmGmUfAmGfUmCmGmUmUm |
| siRPTORc2 -M1P1 | 319 | CmGmAmCmUmAmCfUfAfCmAmUmCmUmCmCmGmUmGmUmAm |
| | 320 | P1CmAfCmGmGmAfGmAmUmGmUmAmGmUfAmGfUmCmGmUmUm |
| siRPTORc2 -M2P1 | 321 | CmGmAmCmUfAmCfUfAfCmAmUmCmUmCmCmGmUmGmUmAm |
| | 322 | P1CmAfCmGmGmAfGmAfUfGmUmAmGmUfAmGfUmCmGmUmUm |
| siRPTORc2 -M3P1 | 323 | CmGmAmCmUfAmCfUfAfCmAmUmCmUmCmCmGmUmGmUmAm |
| | 324 | P1CmAfCmGmGmAfGmAmUmGmUmAmGmUfAmGfUmCmGmUmUm |
| siRPTORc3 -M1P1 | 325 | AmAmCmGmAmCmUmAmCfUfAfCmAmUmCmUmCmCmGmUmGm |
| | 326 | P1CmAfCmGmGmAfGmAmUmGmUmAmGmUfAmGfUmCmGmUmUmGmCm |
| siRPTORc3 -M2P1 | 327 | AmAmCmGmAmCmUfAmCfUfAfCmAmUmCmUmCmCmGmUmGm |
| | 328 | P1CmAfCmGmGmAfGmAfUfGmUmAmGmUfAmGfUmCmGmUmUmGmCm |
| siRPTORc3 -M3P1 | 329 | AmAmCmGmAmCmUfAmCfUfAfCmAmUmCmUmCmCmGmUmGm |
| | 330 | P1CmAfCmGmGmAfGmAmUmGmUmAmGmUfAmGfUmCmGmUmUmGmCm |
| siRPTORc1 -M1SP1 | 331 | CmsGmsAmCmUmAmCfUfAfCmAmUmCmUmCmCmGmUmGm |
| | 332 | P1CmsAfsCmGmGmAfGmAmUmGmUmAmGmUfAmGfUmCmGmsUmsUm |
| siRPTORc1 -M1XP1 | 333 | CmsGmsAmCmUmAmCfUfAfCmAmUmCmUmCmCmGmsUmsGm |
| | 334 | P1CmsAfsCmGmGmAfGmAmUmGmUmAmGmUfAmGfUmCmGmsUmsUm |
| siRPTORc1 -M2SP1 | 335 | CmsGmsAmCmUfAmCfUfAfCmAmUmCmUmCmCmGmUmGm |
| | 336 | P1CmsAfsCmGmGmAfGmAfUfGmUmAmGmUfAmGfUmCmGmsUmsUm |
| siRPTORcl -M2XP1 | 337 | CmsGmsAmCmUfAmCfUfAfCmAmUmCmUmCmCmGmsUmsGm |
| | 338 | P1CmsAfsCmGmGmAfGmAfUfGmUmAmGmUfAmGfUmCmGmsUmsUm |
| siRPTORcl -M3SP1 | 339 | CmsGmsAmCmUfAmCfUfAfCmAmUmCmUmCmCmGmUmGm |
| | 340 | P1CmsAfsCmGmGmAfGmAmUmGmUmAmGmUfAmGfUmCmGmsUmsUm |
| siRPTORc1 -M3XP1 | 341 | CmsGmsAmCmUfAmCfUfAfCmAmUmCmUmCmCmGmsUmsGm |
| | 342 | P1CmsAfsCmGmGmAfGmAmUmGmUmAmGmUfAmGfUmCmGmsUmsUm |
| siRPTORc2 -M1SP1 | 343 | CmsGmsAmCmUmAmCfUfAfCmAmUmCmUmCmCmGmUmGmUmAm |
| | 344 | P1CmsAfsCmGmGmAfGmAmUmGmUmAmGmUfAmGfUmCmGmsUmsUm |
| siRPTORc2 -M1XP1 | 345 | CmsGmsAmCmUmAmCfUfAfCmAmUmCmUmCmCmGmUmGmsUmsAm |
| | 346 | P1CmsAfsCmGmGmAfGmAmUmGmUmAmGmUfAmGfUmCmGmsUmsUm |
| siRPTORc2 -M2SP1 | 347 | CmsGmsAmCmUfAmCfUfAfCmAmUmCmUmCmCmGmUmGmUmAm |
| | 348 | P1CmsAfsCmGmGmAfGmAfUfGmUmAmGmUfAmGfUmCmGmsUmsUm |
| siRPTORc2 -M2XP1 | 349 | CmsGmsAmCmUfAmCfUfAfCmAmUmCmUmCmCmGmUmGmsUmsAm |
| | 350 | P1CmsAfsCmGmGmAfGmAfUfGmUmAmGmUfAmGfUmCmGmsUmsUm |
| siRPTORc2 -M3SP1 | 351 | CmsGmsAmCmUfAmCfUfAfCmAmUmCmUmCmCmGmUmGmUmAm |
| | 352 | P1CmsAfsCmGmGmAfGmAmUmGmUmAmGmUfAmGfUmCmGmsUmsUm |

(continued)

| siRNA No. | SEQ ID NO: | Sequence Direction 5' - 3' |
|---|---|---|
| siRPTORc2 | 353 | CmsGmsAmCmUfAmCfUfAfCmAmUmCmUmCmCmGmUmGmsUmsAm |
| -M3XP1 | 354 | P1CmsAfsCmGmGmAfGmAmUmGmUmAmGmUfAmGfUmCmGmsUmsUm |
| siRPTORc3 -M1SP1 | 355 | AmsAmsCmGmAmCmUmAmCfUfAfCmAmUmCmUmCmCmGmUmGm |
| | 356 | P1CmsAfsCmGmGmAfGmAmUmGmUmAmGmUfAmGfUmCmGmUmUmsGmsCm |
| siRPTORc3 -M1XP1 | 357 | AmsAmsCmGmAmCmUmAmCfUfAfCmAmUmCmUmCmCmGmsUmsGm |
| | 358 | P1CmsAfsCmGmGmAfGmAmUmGmUmAmGmUfAmGfUmCmGmUmUmsGmsCm |
| siRPTORc3 -M2SP1 | 359 | AmsAmsCmGmAmCmUfAmCfUfAfCmAmUmCmUmCmCmGmUmGm |
| | 360 | P1CmsAfsCmGmGmAfGmAfUfGmUmAmGmUfAmGfUmCmGmUmUmsGmsCm |
| siRPTORc3 -M2XP1 | 361 | AmsAmsCmGmAmCmUfAmCfUfAfCmAmUmCmUmCmCmGmsUmsGm |
| | 362 | P1CmsAfsCmGmGmAfGmAfUfGmUmAmGmUfAmGfUmCmGmUmUmsGmsCm |
| siRPTORc3 -M3SP1 | 363 | AmsAmsCmGmAmCmUfAmCfUfAfCmAmUmCmUmCmCmGmUmGm |
| | 364 | P1CmsAfsCmGmGmAfGmAmUmGmUmAmGmUfAmGfUmCmGmUmUmsGmsCm |
| siRPTORc3 -M3XP1 | 365 | AmsAmsCmGmAmCmUfAmCfUfAfCmAmUmCmUmCmCmGmsUmsGm |
| | 366 | P1CmsAfsCmGmGmAfGmAmUmGmUmAmGmUfAmGfUmCmGmUmUmsGmsCm |
| siRPTORc1 -T1S | 381 | CmsGmsAmCmUmAmCfUfAfCmAmUmCmUmCmCmGmUmGm |
| | 382 | CmsAfsCmoeGmGmAfGmAmUmGmUmAmGmUfAmGfUmCmGmsUmsUm |
| siRPTORc1 -T2S | 383 | CmsGmsAmCmUmAmCfUfAfCmAmUmCmUmCmCmGmUmGm |
| | 384 | CmsAfsCmGmGmoeAfGmAmUmGmUmAmGmUfAmGfUmCmGmsUmsUm |

wherein C, G, U, and A represent the base composition of a nucleotides; m represents that the nucleotide adjacent to the left side of the letter m is a methoxy modified nucleotide; f represents that the nucleotide adjacent to the left side of the letter f is a fluoro modified nucleotide; s represents that the two nucleotides adjacent to both sides of the letter s are linked by a phosphorothioate linkage; P1 represents that the nucleotide adjacent to the right side of P1 is a 5'-phosphate nucleotide or a 5'-phosphate analog modified nucleotide. In some embodiments, P1 represents a specifically modified VP, Ps or P, wherein the letter combination VP represents that the nucleotide adjacent to the right side of the letter combination VP is a vinylphosphonate (5'-(E)-vinylphosphonate, E-VP) modified nucleotide; the letter combination Ps represents that the nucleotide adjacent to the right side of the letter combination Ps is a phosphorothioate modified nucleotide; and P represents that the nucleotide adjacent to the right side of the letter P is a 5'-phosphate nucleotide. The letter combination moe represents that the nucleotide adjacent to the left side of the letter combination moe is a 2'-O-methoxyethyl modified nucleotide. For example, the letter combination Gmoe represents that the base of the nucleotide adjacent to the left side of the letter combination moe is guanine, and is a 2'-O-methoxyethyl modified nucleotide. Furthermore, the letter combination Cmoe represents that the base of the nucleotide adjacent to the left side of the letter combination moe is 5-methyl-cytosine, and is a 2'-O-methoxyethyl modified nucleotide. In addition, each U in the sequences as listed in the above tables may be arbitrarily replaced with T, without obviously affecting the activity or off-target effect of the siRNA.

In the siRNA, the pharmaceutical composition or the siRNA conjugate of the present disclosure, adjacent nucleotides are linked via a phosphodiester bond or phosphorothioate diester bond. The non-bridging oxygen or sulfur atom in the phosphodiester bond or phosphorothioate diester bond is negatively charged, and may be present in the form of hydroxy or sulfhydryl. Moreover, the hydrogen ion in the hydroxy or sulfhydryl may be partially or completely substituted with a cation. The cation may be any cation, such as a metal cation, an ammonium cation $NH_4^+$ or an organic ammonium cation. In order to increase solubility, in one embodiment, the cation is selected from one or more of an alkali metal cation, an ammonium cation formed by a tertiary amine and a quaternary ammonium cation. The alkali metal ion may be $K^+$ and/or $Na^+$, and the cation formed by a tertiary amine may be an ammonium cation formed by triethylamine and/or an ammonium cation formed by N, N-diisopropylethylamine. Thus, the siRNA or siRNA conjugate of the present disclosure may be at least partially present in the form of salt. In one embodiment, non-bridging oxygen atom or sulfur atom in the phosphodiester bond or phosphorothioate diester bond at least partly binds to a sodium ion. The siRNA or

siRNA conjugate of the present disclosure is present or partially present in the form of sodium salt.

Those skilled in the art clearly know that a modified nucleotide may be introduced into the siRNA of the present disclosure by a nucleoside monomer with a corresponding modification. The methods for preparing a nucleoside monomer having the corresponding modification and the methods for introducing a modified nucleotide into a siRNA are also well-known to those skilled in the art. All modified nucleoside monomers may be either commercially available or may be prepared by known methods.

The siRNA conjugate of the present disclosure may be prepared by any appropriate synthesis routes. For example, for the conjugation molecule that comprises a targeting group and an active reaction group that can react with phosphoramidite to form a covalent linkage, the siRNA conjugate of the present disclosure can be prepared by the following method comprising: protecting the active group in the conjugation molecule with a protective agent; linking the conjugation molecule to a solid phase support; then sequentially linking nucleoside monomers in 3' to 5' direction according to the type and sequence of the nucleotides in the sense strand and antisense strands of the siRNA by the phosphoramidite solid phase synthesis method, wherein the step of linking each nucleoside monomer includes a four-step reaction of deprotection, coupling, capping, and oxidation or sulfurization; isolating the sense strand and the antisense strand of the siRNA; and annealing.

Further, the siRNA conjugate can also be prepared according to the disclosure of any known literature. For example, a method of sequentially linking the linking group having a specific structure and the targeting ligand to the siRNA via reaction is described in Example 1 of WO2019010274A1, which is incorporated herein by reference in its entirety.

Use of the siRNA, and the pharmaceutical composition and the siRNA conjugate comprising the siRNA of the present disclosure

In some embodiments, the present disclosure provides use of the siRNA and/or the pharmaceutical composition and/or the siRNA conjugate of the present disclosure in the manufacture of a medicament for treating and preventing diseases or symptoms associated with the regulation of *RPTOR* function. In some embodiments, the disease or symptom associated with the regulation of *RPTOR* function is a disease caused by mTORC1 activation or a disease associated with cellular autophagy dysfunction. In some embodiments, the neurodegenerative disease or symptom is Alzheimer's disease and/or Parkinson's disease. Preferably, the neurodegenerative disease is Alzheimer's disease. In some embodiments, the disease associated with cellular autophagy dysfunction is non-alcoholic steatohepatitis.

In some embodiments, the present disclosure provides a method for preventing and/or treating a disease or symptom associated with the regulation of *RPTOR* function, comprising administering an effective amount of the siRNA and/or the pharmaceutical composition and/or the siRNA conjugate of the present disclosure to a subject in need thereof. The purpose of preventing and/or treating the disease may be achieved through the mechanism of RNA interference by administering the siRNA active ingredient of the present disclosure to a subject in need thereof. Thus, the siRNA and/or the pharmaceutical composition and/or the siRNA conjugates of the present disclosure may be used for preventing and/or treating a disease or symptom associated with the regulation of *RPTOR* function, or for preparing a medicament for preventing and/or treating a disease or symptom associated with the regulation of *RPTOR* function.

As used herein, the term "administration/administer" refers to placing the siRNA, the pharmaceutical composition and/or the siRNA conjugate of the present disclosure into a subject's body by a method or route where at least, in part, the siRNA, the pharmaceutical composition and/or the siRNA conjugate of the present disclosure is located at a desired site to achieve a desired effect. The administration routes suitable for the methods of the present disclosure include topical administration and systemic administration. In general, topical administration results in the delivery of more siRNA conjugates to a particular site as compared with the systemic circulation of the subject; while systemic administration results in the delivery of the siRNA, the pharmaceutical composition and/or the siRNA conjugate of the present disclosure to the basic systemic circulation of the subject. Considering that the present disclosure is intended to provide a means for preventing and/or treating a neurodegenerative disease, in some embodiments, an administration manner capable of delivering a medicament to a central nervous system (CNS) tissue is used; in some embodiments, an administration manner capable of delivering a medicament to an intrathecal site is used; in some embodiments, an administration manner capable of injecting a medicament to spinal fluid is used.

Any suitable routes known in the art can be used for administration to a subject, including but not limited to, oral or parenteral route, such as intravenous administration, intramuscular administration, subcutaneous administration, transdermal administration, intratracheal administration (aerosol), intracerebroventricular administration, intrathecal administration, nasal administration, rectal administration, and topical administration (including buccal administration and sublingual administration). The administration frequency may be once or more times daily, weekly, biweekly, triweekly, monthly, or yearly. The dose of the siRNA, the pharmaceutical composition or the siRNA conjugate of the present disclosure may be a conventional dose in the art, which may be determined according to various parameters, especially age, weight and gender of a subject. Toxicity and efficacy may be measured in cell cultures or experimental animals by

standard pharmaceutical procedures, for example, by determining $LD_{50}$ (the lethal dose that can cause 50% population death) and $ED_{50}$ (the dose that can cause 50% of the maximum response intensity in a quantitative response, and that can cause 50% of the experimental subjects to have a positive response in a qualitative response). The dose range for human may be derived based on the data obtained from cell culture assays and animal studies. In some embodiments, the dose of the formulation prepared from the siRNA, the pharmaceutical composition or the siRNA conjugate will be adjusted according to different administration manners during administration.

When the siRNA, the pharmaceutical composition and/or the siRNA conjugate of the present disclosure is administered, for example, to male or female C57BL/6J mice with an age of 6 to 12 weeks old and a body weight of 18 to 25 g or ob/ob mice with a body weight of 30-45g body weight, calculated based on the amount of the siRNA: (i) for the siRNA conjugate, the dosage of the siRNA thereof may be 0.001 to 100 mg/kg body weight, in some embodiments is 0.01 to 50 mg/kg body weight, in some embodiments is 0.05 to 20 mg/kg body weight, in some further embodiments is 0.1 to 15 mg/kg body weight, and in some further embodiments is 0.1 to 10 mg/kg body weight; (ii) for a pharmaceutical composition formed by the siRNA and a pharmaceutically acceptable carrier, the dosage of the siRNA thereof may be 0.001 to 50 mg/kg body weight, in some embodiments is 0.01 to 10 mg/kg body weight, in some embodiments is 0.05 to 5 mg/kg body weight, and in some embodiments is 0.1 to 3 mg/kg body weight.

In some embodiments, the present disclosure provides a method of inhibiting the expression of *RPTOR* gene in cells, comprising contacting an effective amount of the siRNA and/or the pharmaceutical composition and/or the siRNA conjugate of the present disclosure with the cells, and introducing the siRNA and/or the pharmaceutical composition and/or the siRNA conjugate of the present disclosure into the cells, so as to realize the purpose of inhibiting the expression of *RPTOR* gene in the cells through the mechanism of RNA interference.

In the case where the expression of *RPTOR* gene in a cell is inhibited by using the method of the present disclosure, the amount of siRNA in the provided modified siRNA, the pharmaceutical composition and/or the siRNA conjugate is generally an amount sufficient to reduce the expression of the target gene and result in an extracellular concentration of 1 pM to 1 μM, or 0.01 nM to 100 nM, or 0.05 nM to 50 nM, or 0.05 nM to about 5 nM on the surface of the target cells. The amount required to achieve this local concentration will vary with various factors, including the delivery method, the delivery site, the number of cell layers between the delivery site and the target cells or tissue, the delivery route (topical or systemic), etc. The concentration at the delivery site may be significantly higher than that on the surface of the target cells or tissues.

## Kit

In one aspect, the present disclosure provides a kit comprising an effective amount of at least one of the siRNA, the pharmaceutical composition, and the siRNA conjugate of the present disclosure.

In some embodiments, the kit of the present disclosure provides the siRNA in one container. In some embodiments, the kit of the present disclosure comprises a container comprising pharmaceutically acceptable excipients. In some embodiments, the kis of the present disclosure further comprises additional ingredients, such as stabilizers or preservatives. In some embodiments, the kit comprises at least one additional therapeutic agent in other container than the container comprising the siRNA of the present disclosure. In some embodiments, the kit comprises an instruction for mixing the siRNA with pharmaceutically acceptable carriers and/or adjuvants or other ingredients (if any).

In the kit of the present disclosure, the siRNA and pharmaceutically acceptable carriers and/or adjuvants as well as the modified siRNA, pharmaceutical composition and/or siRNA conjugate, and/or pharmaceutically acceptable carriers and/or adjuvants may be provided in any form, e.g., in a liquid form, a dry form, or a lyophilized form. In some embodiments, the siRNA and pharmaceutically acceptable carriers and/or adjuvants as well as the pharmaceutical composition and/or siRNA conjugate and/or pharmaceutically acceptable carriers and/or adjuvants are substantially pure and/or sterile. In some embodiments, sterile water may be provided in the kit of the present disclosure.

Hereinafter, the present disclosure will be further illustrated with reference to the examples, but is not limited thereto in any respect.

## Examples

Unless otherwise specified, the reagents and the culture media used in following examples are all commercially available, and the procedures used such as nucleic acid electrophoresis and real-time PCR are all performed according to the methods described in Molecular Cloning (Cold Spring Harbor Laboratory Press (1989)).

Unless otherwise specified, ratios of reagents provided below are all calculated by volume ratio (v/v). The data are analyzed with Graphpad prism 8.0 statistical analysis software.

Unless otherwise specified, the reagents and culture media used in following examples are all commercially available, and the procedures used such as nucleic acid electrophoresis and real-time PCR are all performed according to the methods described in Molecular Cloning (Cold Spring Harbor Laboratory Press (1989)).

Preparation Examples 1 to 5: Synthesis of the siRNAs of the present disclosure

The siRNA sequences as listed in Table 2 were synthesized by the solid phase synthesis method, respectively. The sense strands and antisense strands complementary to one another as shown in Table 2 in an equimolar ratio were dissolved in DEPC water, and then annealed to afford siRPTORa2-M1X, siRPTORb2-M1X, siRPTORc2-M1X, siRPTORc1-T2S and siRPTORc1-M1S of the present disclosure. The siRNA was diluted to a concentration of 0.2 g/mL (calculated based on the siRNA) by using ultra-pure water (prepared by Milli-Q ultra-pure water instrument, with resistivity of 18.2MS2*cm (25°C)). The molecular weight was measured by Liquid Chromatography-Mass Spectrometry (LC-MS) (purchased from Waters Corp., model: LCT Premier). Since the measured values were in conformity with the calculated values, it was confirmed that the synthesized siRNA was the designed double stranded nucleic acid sequence of interest.

Comparative Preparation Example 1: Synthesis of comparative siRNA

The sense strand and antisense strand of the siRNA with siRNA NO. NC in Table 2 were synthesized by the solid phase synthesis method, respectively. The sense strand and antisense strand in an equimolar ratio were dissolved in DEPC water and then annealed to afford the comparative siRNA with siRNA NO. NC, i.e., the siRNA as the negative control, which does not have sequence homology to *RPTOR* mRNA.

Table 2. siRNA sequences

| Preparation Example No. | siRNA NO. | Sequence direction 5'-3' | | SEQ ID NO: |
|---|---|---|---|---|
| Preparation Example 1 | siRPTORa2-M1X | Sense strand | CmsUmsGmCmCmAmUfGfGfAmGmUmA mUmCmUmGmAmAmsCmsGm | 47 |
| | | Antisense strand | UmsUfsCmAmGmAfUmAmCmUmCmCmA mUfGmGfCmAmGmsUmsGm | 48 |
| Preparation Example 2 | siRPTORb2-M1X | Sense strand | AmsCmsAmAmCmAmUfCfAfAmGmUmA mCmUmAmCmGmAmsCmsGm | 169 |
| | | Antisense strand | UmsCfsGmUmAmGfUmAmCmUmUmGmA mUfGmUfUmGmUmsUmsGm | 170 |
| Preparation Example 3 | siRPTORc2-M1X | Sense strand | CmsGmsAmCmUmAmCfUfAfCmAmUmCm UmCmCmGmUmGmsUmsAm | 291 |
| | | Antisense strand | CmsAfsCmGmGmAfGmAmUmGmUmAmG mUfAmGfUmCmGmsUmsUm | 292 |
| Preparation Example 4 | siRPTORc1 -T2S | Sense strand | CmsGmsAmCmUmAmCfUfAfCmAmUmCm UmCmCmGmUmGm | 383 |
| | | Antisense strand | CmsAfsCmGmGmoeAfGmAmUmGmUmA mGmUfAmGfUmCmGmsUmsUm | 384 |
| Preparation Example 5 | siRPTORc1-M1S | Sense strand | CmsGmsAmCmUmAmCfUfAfCmAmUmCm UmCmCmGmUmGm | 277 |
| | | Antisense strand | CmsAfsCmGmGmAfGmAmUmGmUmAmG mUfAmGfUmCmGmsUmsUm | 278 |
| ComparativePre paration Example 1 | NC | Sense strand | UmsUmsCmUmCmCmGfAfAfCmGmUmG mUmCmAmCmGmUm | 367 |
| | | Antisense strand | PAmsCfsGmUmGmAfCmAmCmGmUmUm CmGfGmAfGmAmAmsCmsUm | 368 |

wherein C, G, U, and A represent the base composition of a nucleotide; m represents that the nucleotide adjacent to the left side of the letter m is a methoxy modified nucleotide; f represents that the nucleotide adjacent to the left side of the letter f is a fluoro modified nucleotide; s represents the two nucleotides adjacent to both sides of the letter s are linked by a phosphorothioate linkage; P represents that the nucleotide adjacent to the right side of the letter P is a 5'

-phosphate nucleotide; and the letter combination moe represents that the nucleotide adjacent to the left side of the letter combination moe is a 2'-O-methoxyethyl modified nucleotide.

After the preparation of the above siRNAs or the comparative siRNA of the present disclosure, they were lyophilized to solid powder and stored until used.

Preparation Examples 6-7: Synthesis of Conjugates 1-2

Conjugate 1 of the present disclosure was prepared by using the method for preparing the "Conjugate 1" in Preparation Example 1 of WO2019/105437A1, except that when preparing the single strands of the sense strand and the antisense strand, the nucleoside monomers at the corresponding positions were respectively linked one by one according to the sequences in the sense strand and antisense strand of siRPTORc1-T2S listed in Table 2. The molecular weight was measured by Liquid Chromatography-Mass Spectrometry (LC-MS). The results showed that the calculated value of the sense strand was 7468.3, and the measured value of the sense strand was 7467.2; and the calculated value of the antisense strand was 7107.7, and the measured value of the antisense strand was 7106.7. Since the measured values were in conformity with the calculated values, it was confirmed that the synthesized Conjugate 1 was the designed double stranded nucleic acid sequence of interest with the group as shown by Formula (301). Conjugate 1 has a structure as shown by Formula (301):

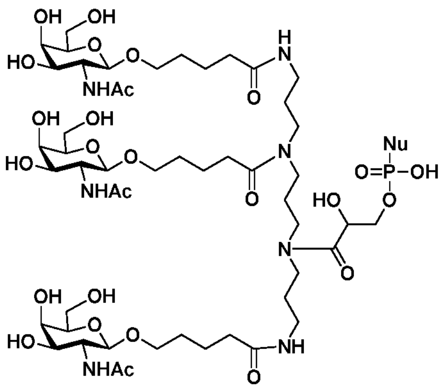
,

Formula (301)

wherein Nu in Formula (301) is siRPTORc1-T2S as listed in Table 1 of the present disclosure; the conjugation group is linked to the 3'-position on the ribose ring at 3' terminal of the sense strand in siRPTORc1-T2S; and the conjugate is in the form of a sodium salt.

Conjugate 2 of the present disclosure was prepared by the method above, except that when preparing the single strands of the sense strand and the antisense strand, the nucleoside monomers at the corresponding positions were respectively linked one by one according to the sequences in the sense strand and antisense strand of siRPTORc1-T2S listed in Table 2. The molecular weight was measured by Liquid Chromatography-Mass Spectrometry (LC-MS). The results showed that the calculated value of the sense strand was 7468.3, and the measured value of the sense strand was 7467.2; and the calculated value of the antisense strand was 7063.7, and the measured value of the antisense strand was 7062.7. Since the measured values were in conformity with the calculated values, it was confirmed that the synthesized Conjugate 2 was the designed double stranded nucleic acid sequence of interest with the group as shown by Formula (301). Conjugate 2 has a structure as shown by Formula (301), wherein Nu in Formula (301) is siRPTORc1-M1S as listed in Table 1 of the present disclosure; the conjugation group is linked to the 3'-position on the ribose ring at 3' terminal of the sense strand in the siRPTORc1-M1S; and the conjugate is in the form of a sodium salt.

Experimental Example 1: Inhibitory activities *in vitro* of the siRNAs of the present disclosure

In this experimental example, the inhibitory activities *in vitro* of siRPTORa2-M1X, siRPTORb2-M1X and siRPTORc2-M1X of the present disclosure in HepG2 human hepatoma cells were investigated.

HepG2 human hepatoma cells (purchased from Nanjing Cobioer Biosciences Co., LTD) were cultured in DMEM media (Hyclone company) supplemented with 10% fetal bovine serum (FBS, RMBIO company) at 37°C in an incubator containing 5% $CO_2$/95% air.

The HepG2 cells were inoculated in a 12-well plate at $2.0 \times 10^5$ cells/well, 1 mL cell solution per well. After the plate was cultured for 24 hours, the media in the culture well were aspirated. 500 µl of Opti-MEM medium (GIBCO company) was further added into each well.

The siRNAs siRPTORa2-M1X, siRPTORb2-M1X and siRPTORc2-M1X obtained in Preparation Examples 1-3 were respectively formulated into siRNA working solutions at a concentration of 20 μM with phosphate buffered saline (PBS).

For each siRNA, a 1A1 solution was prepared. Each portion of the 1A1 solution contains 48.5 μl of Opti-MEM medium and 1.5 μl of the siRNA working solution at a concentration of 20 μM.

A 1B solution was prepared. Each portion of the 1B solution contains 49 μl of Opti-MEM medium and 1 μl of Lipofectamine™ 2000 (Invitrogen).

For each siRNA, one portion of the 1B solution and one portion of the 1A1 solution were incubated at room temperature for 20 minutes to give transfection complexes 1Xa, 1Xb and 1Xc.

One portion of 1B solution was mixed with 50 μl of Opti-MEM medium and incubated at room temperature for 20 minutes to give a transfection complex 1X.

The transfection complex 1Xa was respectively added into two culture wells in an amount of 100 μl/well (both culture wells are the above culture wells containing HepG2 cells and 500 μL of Opti-MEM medium; the same applies hereinafter), and then mixed well to give a transfection mixture at a final concentration of 50 nM (designated as test group 1).

The transfection complex 1Xb was respectively added into two culture wells in an amount of 100 μl/well (both culture wells are the above culture wells containing HepG2 cells and 500 μL of Opti-MEM medium; the same applies hereinafter), and then mixed well to give a transfection mixture at a final concentration of 50 nM (designated as test group 2).

The transfection complex 1Xc was respectively added into two culture wells in an amount of 100 μl/well (both culture wells are the above culture wells containing HepG2 cells and 500 μL of Opti-MEM medium; the same applies hereinafter), and then mixed well to give a transfection mixture at a final concentration of 50 nM (designated as test group 3).

The transfection complex 1X was respectively added into two culture wells in an amount of 100 μl/well to give a transfection mixture without siRNA (designated as blank control group).

The test groups 1-3 and the blank control group were cultured in the culture wells for 4 hours, and then the supernatant in each culture well was aspirated. 1 mL of Opti-MEM medium was added into each well. The 12-well plate was placed in a $CO_2$ incubator and cultured at 37°C for another 24 hours.

The total RNA of the cells in each well was extracted by Sangon UN1Q-10 column Trizol total RNA isolation kit (purchased from Sangon Biotech Co., Cat No.: TC13KA4109) according to the method described in the instruction.

For the cells in each well, 1 μg of the total RNA was taken and was formulated into a 20 μL reverse transcription reaction system by using the reagents provided in the reverse transcription kit Goldenstar™ RT6 cDNA Synthesis Kit (purchased from Beijing Qingke Xinye Biotechnology Co., Ltd.) according to the precedures for reverse transcription in the instruction of the kit, in which Goldenstar™ Oligo $(dT)_{17}$ was selected as a primer, to reverse-transcribe the total RNA of the cells in each well. The conditions of the reverse transcription were as follows: for each reverse transcription reaction system, the reverse transcription reaction system was incubated at 50°C for 50 minutes, then incubated at 85°C for 5 minutes, and finally incubated at 4°C for 30 seconds. After the reaction was completed, 80 μl of DEPC water was added into the reverse transcription reaction system to give a cDNA-containing solution.

For each reverse transcription reaction system, 5 μl of the above cDNA-containing solution was taken as the template, and was formulated into a 20 μl of a qPCR reaction system by using the reagent provided in the NovoStart® SYBR qPCR SuperMix Plus kit (purchased from Novoprotein Scientific Co., Ltd., Cat No. E096-01B), wherein the sequences of PCR primers used for amplifying the target gene *RPTOR* and the internal control gene GAPDH were shown in Table 3, and the final concentration of each primer was 0.25 μM. Each qPCR reaction system was placed in an ABI StepOnePlus Real-Time PCR Thermal Cycler, and was amplified using a three-step method. The amplification procedures were as follows: pre-denaturation at 95°C for 10 minutes, denaturation at 95°C for 30 s, annealing at 60°C for 30 s, and extension at 72°C for 30 s. After repeating the above process of denaturation, annealing and extension for 40 times, a product W1 comprising the amplified target gene *RPTOR* and the amplified internal control gene *GAPDH* was obtained. The product W1 was then sequentially incubated at 95°C for 15 s, 60°C for 1 min, and 95°C for 15 s. The melting curves of the target gene *RPTOR* and the internal control gene *GAPDH* in the product W1 were respectively collected using a real-time fluorescent quantitative PCR Thermal Cycler, and the Ct values of the target gene *RPTOR* and the internal control gene *GAPDH* were obtained.

Table 3. Information of primers

| Gene name | Primer type | Nucleotide sequence(5'→3') | SEQ ID NO: |
|---|---|---|---|
| Human *RPTOR* | Upstream Primers | CAGCTGTGTGACGAGTCTGT | 369 |
| | Downstream Primers | GGCATCCGGGGATCAAAGAT | 370 |

(continued)

| Gene name | Primer type | Nucleotide sequence(5'→3') | SEQ ID NO: |
|---|---|---|---|
| Human *GAPDH* | Upstream Primers | GGTCGGAGTCAACGGATTT | 371 |
| | Downstream Primers | CCAGCATCGCCCCACTTGA | 372 |

The relative quantitative calculation of the target gene *RPTOR* in each test group was carried out using the comparative Ct (ΔΔCt) method, and the calculation method was as follows:

ΔCt (the test group) = Ct (target gene in the test group) - Ct (internal reference gene in the test group);

ΔCt (the control group) = Ct (target gene in the control group) - Ct (internal reference gene in the control group);

$$\Delta\Delta Ct \text{ (the test group)} = \Delta Ct \text{ (the test group)} - \Delta Ct \text{ (mean value in the control group)};$$

$$\Delta\Delta Ct \text{ (the control group)} = \Delta Ct \text{ (the control group)} - \Delta Ct \text{ (mean value in the control group)},$$

wherein the ΔCt (mean value in the control group) is the arithmetic mean value of the ΔCt (the control group) of the two culture wells in the control group. Thus, each culture well in the test group and the control group corresponds to one ΔΔCt value.

The expression level of *RPTOR* mRNA in the test group was normalized based on the mean value of the control group, and the mean value of the expression level of *RPTOR* mRNA in the blank control group was defined as 100%,

$$\text{Relative expression level of } \textit{RPTOR} \text{ mRNA in the test group} = 2^{-\Delta\Delta Ct \text{ (the test group)}} \times 100\%.$$

Inhibition rate of *RPTOR* mRNA in the test group = (1 - relative expression level of *RPTOR* mRNA in the test group) × 100%.

The experimental results are shown in Fig. 1.

Comparative Experimental Example 1: Inhibitory activity *in vitro* of comparative siRNA NC

The inhibitory activity *in intro* of the negative comparative siRNA NC in HepG2 human hepatoma cells was investigated by the same method as that described in Experimental Example 1, except that siRPTORa2-M1X, siRPTORb2-M1X or siRPTORc2-M1X was replaced by the comparative siRNA NC obtained in Comparative Preparation Example to formulate a siRNA working solution at a concentration of 20 μM for testing. The results are shown in Fig. 1.

Figure 1 is a histogram showing relative expression levels of *RPTOR* mRNA in HepG2 human hepatoma cells *in vitro* after transfection with the siRNA of the present disclosure at a concentration of 50 nM and with the comparative siRNA NC, whenrein NC in Fig.1 represents the comparative siRNA NC. The results of Figure 1 show that in HepG2 human hepatoma cells *in vitro,* siRPTORa2-M1X shows an inhibition rate of 76.8% against *RPTOR* mRNA at a concentration of 50 nM; siRPTORb2-M1X shows an inhibition rate of 86.8% against *RPTOR* mRNA at a concentration of 50 nM; and siRPTORc2-M1X shows an inhibition rate of 78.8% against *RPTOR* mRNA at a concentration of 50 nM; clearly, the siRNAs exhibit excellent inhibitory activities against *RPTOR* mRNA and show excellent inhibitory effects on the expression of *RPTOR* gene.

Experimental Example 2: Inhibitory activity *in vitro* of the siRNAs of the present disclosure against the target mRNA

In this experimental example, the inhibitory activities *in vitro* of siRPTORa2-M1X, siRPTORb2-M1X and siRPTORc2-M1X of the present disclosure at different concentrations in HepG2 human hepatoma cells were investigated.

HepG2 human hepatoma cells (purchased from Nanjing Cobioer Biosciences Co., LTD) were cultured in DMEM media (Hyclone company) supplemented with 10% fetal bovine serum (FBS, RMBIO company) at 37°C in an incubator containing 5% $CO_2$/95% air.

The HepG2 cells were inoculated in a 12-well plate at $2.0 \times 10^5$ cells/well, 1 mL cell solution per well. After the plate was cultured for 24 hours, the media in the culture wells were aspirated. 500 μl of Opti-MEM medium (GIBCO company) was further added into each well.

For each siRNA, the siRNAs siRPTORa2-M1X, siRPTORb2-M1X and siRPTORc2-M1X obtained in Preparation Examples 1-3 were respectively formulated into siRNA working solutions at a concentration of 20 μM, 2 μM or 0.2 μM with phosphate buffered saline (PBS).

For each siRNA, a 2A1 solution was prepared. Each portion of the 2A1 solution contains 48.5 μl of Opti-MEM medium and 1.5 μl of the siRNA working solution at a concentration of 20 μM; and thus, the siRNA working solutions 2A1a, 2A1b and 2A1c were respectively obtained.

For each siRNA, a 2A2 solution was prepared. Each portion of the 2A2 solution contains 48.5 μl of Opti-MEM medium and 1.5 μl of the siRNA working solution at a concentration of 2 μM; and thus, the siRNA working solutions 2A2a, 2A2b or 2A2c were respectively obtained.

For each siRNA, a 2A3 solution was prepared. Each portion of the 2A3 solution contains 48.5 μl of Opti-MEM medium and 1.5 μl of the siRNA working solution at a concentration of 0.2 μM; and thus, the siRNA working solutions 2A3a, 2A3b and 2A3c were respectively obtained.

A 2B solution was prepared. Each portion of the 2B solution contains 49 μl of Opti-MEM medium and 1 μl of Lipofectamine™ 2000 (Invitrogen).

A 2X0 solution was prepared. One portion of 2B solution was mixed with 50 μl of Opti-MEM medium and incubated at room temperature for 20 minutes to give a blank transfection complex 2X0.

A 2X1 solution was prepared. One portion of 2B solution was respectively mixed with one portion of 2A1a solution, one portion of 2A2a solution or one portion of 2A3a solution and incubated at room temperature for 20 minutes to give transfection complexes 2Xa1, 2Xa2 and 2Xa3, respectively.

A 2X2 solution was prepared. One portion of 2B solution was respectively mixed with one portion of 2A1b solution, one portion of 2A2b solution or one portion of 2A3b solution and incubated at room temperature for 20 minutes to give transfection complexes 2Xb1, 2Xb2 and 2Xb3, respectively.

A 2X3 solution was prepared. One portion of 2B solution was respectively mixed with one portion of 2A1c solution, one portion of 2A2c solution or one portion of 2A3c solution and incubated at room temperature for 20 minutes to form transfection complexes 2Xc1, 2Xc2 and 2Xc3, respectively.

For each siRNA, the transfection complexes 2Xa1, 2Xa2 and 2Xa3 were respectively added into two culture wells in an amount of 100 μl/well (both culture wells are the above culture wells containing HepG2 cells and 500 μL Opti-MEM medium; the same applies hereinafter), and then mixed well to give transfection mixtures comprising siRPTORa2-M1X at a final concentration of 50 nM, 5 nM or 0.5 nM (recorded as test group 1).

For each siRNA, the transfection complexes 2Xb1, 2Xb2 or 2Xb3 were respectively added into two culture wells in an amount of 100 μl/well (both culture wells are the above culture wells containing HepG2 cells and 500 μL Opti-MEM medium; the same applies hereinafter), and then mixed well to give transfection mixtures comprising siRPTORb2-M1X at a final concentration of 50 nM, 5 nM or 0.5 nM (recorded as test group 2).

For each siRNA, the transfection complexes 2Xc1, 2Xc2 and 2Xc3 were respectively added into two culture wells in an amount of 100 μl/well (both culture wells are the above culture wells containing HepG2 cells and 500 μL of Opti-MEM medium; the same applies hereinafter), and then mixed well to give transfection mixtures comprising siRPTORc2-M1X at a final concentration of 50 nM, 5 nM or 0.5 nM (recorded as test group 3).

The blank transfection complex 2X0 was respectively added into two culture wells in an amount of 100 μl/well to give a transfection mixture without siRNA (designated as blank control group).

The test groups 1-3 and the blank control group were cultured in the culture wells for 4 hours, and then the supernatant in each culture well was aspirated. 1 mL of Opti-MEM medium was added into each well. The 12-well plate was placed in a $CO_2$ incubator and cultured at 37°C for another 24 hours.

The total RNA in the cells of each well was extracted by using the same method as that in Experimental Example 1 and reverse-transcribed. The relative quantitative calculation of the target gene *RPTOR* mRNA in each test group was carried out. The results are shown in Fig. 2.

Figure 2 is a histogram showing relative expression levels of *RPTOR* mRNA in HepG2 human hepatoma cells *in vitro* after transfection with the siRNAs of the present disclosure at different concentrations. The results of Figure 2 show that in HepG2 human hepatoma cells *in vitro,* siRPTORc2-M1X shows an inhibition rate of at least 40.5% against

*RPTOR* mRNA at a low concentration of 0.5 nM; siRPTORa2-M1X shows an inhibition rate of at least 68.3% against *RPTOR* mRNA at a concentration of 5 nM and an inhibition rate of at least 71.1% against *RPTOR* mRNA at a concentration of 50 nM; and siRPTORb2-M1X shows an inhibition rate of even up to 86.4% against *RPTOR* mRNA at a concentration of 50 nM; clearly, the siRNAs exhibit excellent inhibitory effects on the expression of *RPTOR* gene.

Experimental example 3: Inhibitory activity *in vivo* of the siRNA conjugates in mice

Conjugates 1 and 2 prepared in Preparation Examples 6-7 were respectively dissolved in phosphate buffered saline (PBS) to give solutions of the siRNA conjugates with a concentration of 3 mg/ml (calculated based on the siRNA). C57BL/6 mice (female, 6 to 8 weeks old, 16 to 18 g body weight, and purchased from SPF biotechnology Co., LTD.) were randomly divided into three groups (6 mice per group) and respectively numbered. The above solution of the siRNA Conjugate 1 was respectively administered to each of the mice in the first group by subcutaneous injection on the nape of the neck. The mice were weighed and recorded before administration. Subsequently, the mice were administered according to body weights a dosing volume of 5 mL/kg, and recorded as test group 1. The above solution of the siRNA Conjugate 2 was respectively administered to each of the mice in the second group. The mice were weighed and recorded before administration. Subsequently, the mice were administered according to body weights with a dosing volume of 5 mL/kg, and recorded as test group 2. Each of the mice in the thied group was administered with PBS with a dosing volume of 5 mL/kg, and recorded as blank control group.

The time point of administration was recorded as day 1. On day 8, the liver tissue of each of the mice in the test groups and the blank control group was collected and stored in RNAlater.

The total RNA in the cells of each well was extracted by using the same method as that in Experimental Example 1 and reverse-transcribed. The relative quantitative calculation of the target gene *RPTOR* in each test group was carried out. Information of the primers used was shown in Table 3 above; and the results were normalized based on that of the blank control group. The results are shown in Table 4.

Table 4. Inhibitory activity *in vivo* of the siRNA conjugates in mice

| Group | Test group 1 | Test group 2 |
|---|---|---|
| Inhibition rate against *RPTOR* mRNA (%) | 52.9% | 51.3% |

As can be seen from the above results, at a concentration of 3 mg/kg, the siRNA conjugates in different modified embodiments of the present disclosure still show an inhibition rate of 50% or higher against *RPTOR* mRNA in C57BL/6j mice within one week after administration as compared with the results of the blank control group, and exhibit excellent *in vivo* inhibitory activity against *RPTOR* mRNA and longer *in vivo* stability.

The above experimental results have shown that the siRNAs of the present disclosure can effectively inhibit the expression of *RPTOR* gene in cells, and thus exhibit a promising potential in the preparation of a medicament for treating and/or preventing diseases or symptoms associated with abnormal activation of mTORC1 and cellular autophagy, such as NASH or neurodegenerative diseases.

The preferred embodiments of the present disclosure are described above in detail, but the present disclosure is not limited to the specific details of the above embodiments. Various simple variations of the technical solutions of the present disclosure can be made within the scope of the technical concept of the present disclosure, and these simple variations are within the scope of the present disclosure.

It is to be noted that each of the specific technical features described in the above embodiments can be combined in any suitable manner as long as no contradiction is caused. In order to avoid unnecessary repetition, various possible combinations are no longer described in the present disclosure.

In addition, various different embodiments of the present disclosure may also be arbitrarily combined as long as it does not deviate from the idea of the present disclosure, which should also be regarded as the disclosure of the present disclosure.

## Claims

1. A siRNA, comprising a sense strand and an antisense strand, each nucleotide in the siRNA being independently a modified or unmodified nucleotide, wherein the sense strand comprises a nucleotide sequence I, and the antisense strand comprises a nucleotide sequence II; the nucleotide sequence I and the nucleotide sequence II are at least partly reverse complementary to form a double-stranded region; wherein the nucleotide sequence I and the nucleotide sequence II are selected from a group of the sequences as shown in i) to iii):

i) the nucleotide sequence I has an equal length to the nucleotide sequence shown in SEQ ID NO: 1 with no more than 3 nucleotide differences; and the nucleotide sequence II has an equal length to the nucleotide sequence shown in SEQ ID NO: 2 with no more than 3 nucleotide differences:

5'-CUGCCAUGGAGUAUCUGA$Z_{a1}$-3' (SEQ ID NO:1);
5'-$Z_{a2}$UCAGAUACUCCAUGGCAG-3' (SEQ ID NO:2),

wherein $Z_{a1}$ is A and $Z_{a2}$ is U; the nucleotide sequence I comprises a nucleotide $Z_{a3}$ at the position corresponding to $Z_{a1}$; the nucleotide sequence II comprises a nucleotide $Z_{a4}$ at the position corresponding to $Z_{a2}$; the nucleotide $Z_{a4}$ is the first nucleotide at 5' terminal of the antisense strand;
ii) the nucleotide sequence I has an equal length to the nucleotide sequence shown in SEQ ID NO: 123 with no more than 3 nucleotide differences, and the nucleotide sequence II has an equal length to the nucleotide sequence shown in SEQ ID NO: 124 with no more than 3 nucleotide differences:

5'-ACAACAUCAAGUACUACG$Z_{b1}$-3' (SEQ ID NO: 123);
5'-$Z_{b2}$CGUAGUACUUGAUGUUGU-3' (SEQ ID NO: 124),
wherein $Z_{b1}$ is A and $Z_{b2}$ is U; the nucleotide sequence I comprises a nucleotide $Z_{b3}$ at the position corresponding to $Z_{b1}$; the nucleotide sequence II comprises a nucleotide $Z_{b4}$ at the position corresponding to $Z_{b2}$; the nucleotide $Z_{b4}$ is the first nucleotide at 5' terminal of the antisense strand; and

iii) the nucleotide sequence I has an equal length to the nucleotide sequence shown in SEQ ID NO: 245 with no more than 3 nucleotide differences; and the nucleotide sequence II has an equal length to the nucleotide sequence shown in SEQ ID NO: 246 with no more than 3 nucleotide differences:

5'-CGACUACUACAUCUCCGU$Z_{c1}$-3' (SEQ ID NO: 245);
5'-$Z_{c2}$ACGGAGAUGUAGUAGUCG-3' (SEQ ID NO: 246),

wherein $Z_{c1}$ is G and $Z_{c2}$ is C; the nucleotide sequence I comprises a nucleotide $Z_{c3}$ at the position corresponding to $Z_{c1}$; the nucleotide sequence II comprises a nucleotide $Z_{c4}$ at the position corresponding to $Z_{c2}$; the nucleotide $Z_{c4}$ is the first nucleotide at 5' terminal of the antisense strand.

**2.** The siRNA according to claim 1, wherein the nucleotide sequence I and the nucleotide sequence shown in SEQ ID NO: 1 have no more than 1 nucleotide difference, and/or the nucleotide sequence II and the nucleotide sequence shown in SEQ ID NO: 2 have no more than 1 nucleotide difference; or

the nucleotide sequence I and the nucleotide sequence shown in SEQ ID NO: 123 have no more than 1 nucleotide difference, and/or the nucleotide sequence II and the nucleotide sequence shown in SEQ ID NO: 124 have no more than 1 nucleotide difference; or
the nucleotide sequence I and the nucleotide sequence shown in SEQ ID NO: 245 have no more than 1 nucleotide difference, and/or the nucleotide sequence II and the nucleotide sequence shown in SEQ ID NO: 246 have no more than 1 nucleotide difference.

**3.** The siRNA according to claim 1 or 2, wherein the nucleotide difference between the nucleotide sequence II and the nucleotide sequence shown in SEQ ID NO: 2 includes a difference at the position $Z_{a4}$, where $Z_{a4}$ is selected from A, C or G; or

wherein the nucleotide difference between the nucleotide sequence II and the nucleotide sequence shown in SEQ ID NO: 124 includes a difference at the position $Z_{b4}$, where $Z_{b4}$ is selected from A, C or G; or
wherein the nucleotide difference between the nucleotide sequence II and the nucleotide sequence shown in SEQ ID NO: 246 includes a difference at the position $Z_{c4}$, where $Z_{c4}$ is selected from A, U or G.

**4.** The siRNA according to any one of claims 1-3, wherein $Z_{a3}$ is a nucleotide complementary to $Z_{a4}$; or $Z_{b3}$ is a nucleotide complementary to $Z_{b4}$; or $Z_{c3}$ is a nucleotide complementary to $Z_{c4}$.

**5.** The siRNA according to any one of claims 1-4, wherein the nucleotide sequence I and the nucleotide sequence II are basically reverse complementary, substantially reverse complementary, or completely reverse complementary to each other; the "basically reverse complementary" means that there are no more than 3 base mispairings between two nucleotide sequences; the "substantially reverse complementary" means that there is no more than 1 base

mispairing between two nucleotide sequences; and the "completely reverse complementary" means that there is no base mispairing between two nucleotide sequences.

**6.** The siRNA according to any one of claims 1-5, wherein the sense strand and the antisense strand have the same or different lengths; the sense strand has a length of 19 to 23 nucleotides, and the antisense strand has a length of 19 to 26 nucleotides; and wherein the nucleotide sequence I is the nucleotide sequence shown in SEQ ID NO: 3, and the nucleotide sequence II is the nucleotide sequence shown in SEQ ID NO: 4:

5'-CUGCCAUGGAGUAUCUGA$Z_{a3}$-3' (SEQ ID NO: 3);
5'-$Z_{a4}$UCAGAUACUCCAUGGCAG-3' (SEQ ID NO:4),
wherein $Z_{a3}$ is selected from A, U, G or C, and $Z_{a4}$ is a nucleotide complementary to $Z_{a3}$; or
the nucleotide sequence I is the nucleotide sequence shown in SEQ ID NO: 125, and the nucleotide sequence II is the nucleotide sequence shown in SEQ ID NO: 126:

5'-ACAACAUCAAGUACUACG$Z_{b3}$-3' (SEQ ID NO: 125);
5'-$Z_{b4}$CGUAGUACUUGAUGUUGU-3' (SEQ ID NO: 126),

wherein $Z_{b3}$ is selected from A, U, G or C, and $Z_{b4}$ is a nucleotide complementary to $Z_{b3}$; or
the nucleotide sequence I is the nucleotide sequence shown in SEQ ID NO: 247, and the nucleotide sequence II is the nucleotide sequence as shown by SEQ ID NO: 248:

5'-CGACUACUACAUCUCCGU$Z_{c3}$-3' (SEQ ID NO: 247);
5'-$Z_{c4}$ACGGAGAUGUAGUAGUCG-3' (SEQ ID NO: 248),

wherein $Z_{c3}$ is selected from A, U, G or C, and $Z_{c4}$ is a nucleotide complementary to $Z_{c3}$;

**7.** The siRNA according to claim 6, wherein $Z_{a3}$ is A, and $Z_{a4}$ is U; or, $Z_{b3}$ is A, and $Z_{b4}$ is U; or, $Z_{c3}$ is G, and $Z_{c4}$ is C.

**8.** The siRNA according to any one of claims 1-7, wherein the sense strand further comprises a nucleotide sequence III, and the antisense strand further comprises a nucleotide sequence IV; the nucleotide sequence III and the nucleotide sequence IV independently of each other have a length of 1 to 4 nucleotides; the nucleotide sequence III is linked to 5' terminal of the nucleotide sequence I; the nucleotide sequence IV is linked to 3' terminal of the nucleotide sequence II; the nucleotide sequence III and the nucleotide sequence IV have the same length and are substantially reverse complementary or completely reverse complementary to each other; the "substantially reverse complementary" means that there is no more than 1 base mispairing between two nucleotide sequences; and the "completely reverse complementary" means that there is no mispairing between two nucleotide sequences.

**9.** The siRNA according to claim 8, wherein the nucleotide sequence I has an equal length to the nucleotide sequence shown in SEQ ID NO: 1 with no more than 3 nucleotide differences; and the nucleotide sequence III and the nucleotide sequence IV both have a length of 1 nucleotide, and the base of the nucleotide sequence III is A; or

the nucleotide sequence III and the nucleotide sequence IV both have a length of 2 nucleotides, and in the direction from 5' terminal to 3' terminal, the base composition of the nucleotide sequence III is CA; or
the nucleotide sequence III and the nucleotide sequence IV both have a length of 3 nucleotides, and in the direction from 5' terminal to 3' terminal, the base composition of the nucleotide sequence III is UCA; or
the nucleotide sequence III and the nucleotide sequence IV both have a length of 4 nucleotides, and in the direction from 5' terminal to 3' terminal, the base composition of the nucleotide sequence III is GUCA; or
the nucleotide sequence I has an equal length to the nucleotide sequence shown in SEQ ID NO: 123 with no more than 3 nucleotide differences; and the nucleotide sequence III and the nucleotide sequence IV both have a length of 1 nucleotide, and the base of the nucleotide sequence III is A; or
the nucleotide sequence III and the nucleotide sequence IV both have a length of 2 nucleotides, and in the direction from 5' terminal to 3' terminal, the base composition of the nucleotide sequence III is CA; or
the nucleotide sequence III and the nucleotide sequence IV both have a length of 3 nucleotides, and in the direction from 5' terminal to 3' terminal, the base composition of the nucleotide sequence III is UCA; or
the nucleotide sequence III and the nucleotide sequence IV both have a length of 4 nucleotides, and in the direction from 5' terminal to 3' terminal, the base composition of the nucleotide sequence III is AUCA; or
the nucleotide sequence I has an equal length to the nucleotide sequence shown in SEQ ID NO:245 with no more than 3 nucleotide differences; and the nucleotide sequence III and the nucleotide sequence IV both have

a length of 1 nucleotide, and the base of the nucleotide sequence III is A; or
the nucleotide sequence III and the nucleotide sequence IV both have a length of 2 nucleotides, and in the direction from 5' terminal to 3' terminal, the base composition of the nucleotide sequence III is AA; or
the nucleotide sequence III and the nucleotide sequence IV both have a length of 3 nucleotides, and in the direction from 5' terminal to 3' terminal, the base composition of the nucleotide sequence III is CAA; or
the nucleotide sequence III and the nucleotide sequence IV both have a length of 4 nucleotides, and in the direction from 5' terminal to 3' terminal, the base composition of the nucleotide sequence III is GCAA.

**10.** The siRNA according to any one of claims 1-9, wherein the antisense strand further comprises a nucleotide sequence V, and the nucleotide sequence V has a length of 1 to 3 nucleotides and is linked to 3' terminal of the antisense strand, thereby forming a 3' overhang terminal of the antisense strand; and/or
the sense strand further comprises a nucleotide sequence VI, and the nucleotide sequence VI has a length of 1 to 3 nucleotides and is linked to 3' terminal of the sense strand, thereby forming a 3' overhang terminal of the sense strand.

**11.** The siRNA according to claim 10, wherein the nucleotide sequence V and/or the nucleotide sequence VI have a length of 2 nucleotides.

**12.** The siRNA according to claim 10 or 11, wherein the nucleotide sequence V and/or the nucleotide sequence VI is/are 2 consecutive thymine deoxyribonucleotides or 2 consecutive uracil ribonucleotides; or the nucleotide sequence V is complementary to the nucleotides at the corresponding positions of the target mRNA, and/or the nucleotide sequence VI is identical with the nucleotides at the corresponding positions of the target mRNA.

**13.** The siRNA according to any one of claims 1-12, wherein the sense strand of the siRNA comprises the nucleotide sequence shown in SEQ ID NO: 5, and the antisense strand comprises the nucleotide sequence shown in SEQ ID NO: 6:

5'-CUGCCAUGGAGUAUCUGA$Z_{a3}$-3' (SEQ ID NO: 5);
5'-$Z_{a4}$UCAGAUACUCCAUGGCAGUG-3' (SEQ ID NO: 6),
wherein $Z_{a4}$ is the first nucleotide at 5' terminal of the antisense strand; $Z_{a3}$ is selected from A, U, G or C, and $Z_{a4}$ is a nucleotide complementary to $Z_{a3}$; or
the sense strand of the siRNA comprises the nucleotide sequence shown in SEQ ID NO: 7, and the antisense strand of the siRNA comprises the nucleotide sequence shown in SEQ ID NO: 8:

5'-CACUGCCAUGGAGUAUCUGA$Z_{a3}$-3' (SEQ ID NO: 7);
5'-$Z_{a4}$UCAGAUACUCCAUGGCAGUGAC-3' (SEQ ID NO: 8),

wherein $Z_{a4}$ is the first nucleotide at 5' terminal of the antisense strand; $Z_{a3}$ is selected from A, U, G or C, and $Z_{a4}$ is a nucleotide complementary to $Z_{a3}$; or
the sense strand of the siRNA comprises the nucleotide sequence shown in SEQ ID NO: 127, and the antisense strand of the siRNA comprises the nucleotide sequence shown in SEQ ID NO: 128:

5'-ACAACAUCAAGUACUACG$Z_{b3}$-3' (SEQ ID NO: 127);
5'-$Z_{b4}$CGUAGUACUUGAUGUUGUUG-3' (SEQ ID NO: 128), or

the sense strand of the siRNA comprises the nucleotide sequence shown in SEQ ID NO: 129, and the antisense strand of the siRNA comprises the nucleotide sequence shown in SEQ ID NO: 130:

5'-CAACAACAUCAAGUACUACGZb3-3'(SEQ ID NO: 129);
5'-Zb4CGUAGUACUUGAUGUUGUUGAU-3'(SEQ ID NO: 130),

wherein $Z_{b4}$ is the first nucleotide at 5' terminal of the antisense strand; $Z_{b3}$ is selected from A, U, G or C, and $Z_{b4}$ is a nucleotide complementary to $Z_{b3}$; or
the sense strand of the siRNA comprises the nucleotide sequence shown in SEQ ID NO: 249, and the antisense strand of the siRNA comprises the nucleotide sequence shown in SEQ ID NO: 250:

5'-CGACUACUACAUCUCCGU$Z_{c3}$-3'(SEQ ID NO: 249);
5'-$Z_{c4}$ACGGAGAUGUAGUAGUCGUU-3'(SEQ ID NO:250), or

the sense strand of the siRNA comprises the nucleotide sequence shown in SEQ ID NO: 251, and the antisense strand of the siRNA comprises the nucleotide sequence shown in SEQ ID NO: 252:

5'-AACGACUACUACAUCUCCGUZ$_{c3}$-3'(SEQ ID NO: 251);
5'-Z$_{c4}$ACGGAGAUGUAGUAGUCGUUGC-3' (SEQ ID NO: 252),

wherein $Z_{c4}$ is the first nucleotide at 5' terminal of the antisense strand; $Z_{c3}$ is selected from A, U, G or C, and $Z_{c4}$ is a nucleotide complementary to $Z_{c3}$.

**14.** The siRNA according to any one of claims 1-13, wherein the siRNA is any one of siRPTORa1, siRPTORa2, siRPTORa3, siRPTORb1, siRPTORb2, siRPTORb3, siRPTORc1, siRPTORc2, and siRPTORc3.

**15.** The siRNA according to any one of claims 1-14, wherein at least one nucleotide in the sense strand or the antisense strand is a modified nucleotide, and/or at least one phosphate group is a phosphate group with modification group(s).

**16.** The siRNA according to any one of claims 1-15, wherein each nucleotide in the sense strand and the antisense strand is independently a fluoro modified nucleotide or a non-fluoro modified nucleotide.

**17.** The siRNA according to claim 16, wherein the fluoro modified nucleotides are in the nucleotide sequence I and the nucleotide sequence II; and in the direction from 5' terminal to 3' terminal, at least the nucleotides at positions 7, 8 and 9 of the nucleotide sequence I are fluoro modified nucleotides; and in the direction from 5' terminal to 3' terminal, at least the nucleotides at positions 2, 6, 14, and 16 of the nucleotide sequence II are fluoro modified nucleotides.

**18.** The siRNA according to claim 16 or 17, wherein the non-fluoro modified nucleotide is a 2'-O-methoxy modified nucleotide or a 2'-O-methoxyethyl modified nucleotide.

**19.** The siRNA according to claim 18, wherein in the direction from 5' terminal to 3' terminal, at least one nucleotide of the nucleotides at positions 3-6 of the nucleotide sequence II is a 2'-O-methoxyethyl modified nucleotide.

**20.** The siRNA according to claim 19, wherein the nucleotide at position 3 or 5 of the nucleotide sequence II is a 2'-O-methoxyethyl modified nucleotide.

**21.** The siRNA according to claim 19, wherein in the direction from 5' terminal to 3' terminal, no more than 2 nucleotides of the nucleotides at positions 3-9 of the nucleotide sequence II are 2'-O-methoxyethyl modified nucleotides.

**22.** The siRNA according to any one of claims 17-21, wherein the other nucleotides in the sense strand and the antisense strand are 2'-O-methoxy modified nucleotides.

**23.** The siRNA according to any one of claims 1-22, wherein the siRNA is any one of siRPTORa1-M1, siRPTORa1-M2, siRPTORa1-M3, siRPTORa2-M1, siRPTORa2-M2, siRPTORa2-M3, siRPTORa3-M1, siRPTORa3-M2, siS*RPTOR*a3-M3, siRPTORb1-M1, siRPTORb1-M2, siRPTORb1-M3, siRPTORb2-M1, siRPTORb2-M2, siRPTORb2-M3, siRPTORb3-M1, siRPTORb3-M2, siS*RPTOR*b3-M3, siRPTORc1-M1, siRPTORc1-M2, siRPTORc1-M3, siRPTORc2-M1, siRPTORc2-M2, siRPTORc2-M3, siRPTORc3-M1, siRPTORc3-M2, and siSRPTORc3-M3.

**24.** The siRNA according to claim 15, wherein the phosphate group with modification group(s) is a phosphorothioate group formed by substituting at least one oxygen atom in a phosphodiester bond in the phosphate group with a sulfur atom.

**25.** The siRNA according to any one of claims 1-22, wherein the siRNA is any one of siRPTORa1-M1S, siRPTORa1-M1X, siRPTORa1-M2S, siRPTORa1-M2X, siRPTORa1-M3S, siRPTORa1-M3X, siRPTORa2-M1S, siRPTORa2-M1X, siRPTORa2-M2S, siRPTORa2-M2X, siRPTORa2-M3S, siRPTORa2-M3X, siRPTORa3-M1S, siRPTORa3-M1X, siRPTORa3-M2S, siRPTORa3-M2X, siRPTORa3-M3S, siRPTORa3-M3X, siRPTORa1-T1S, siRPTORa1-T2S, siRPTORb1-M1S, siRPTORb1-M1X, siRPTORb1-M2S, siRPTORb1-M2X, siRPTORb1-M3S, siRPTORb1-M3X, siRPTORb2-M1S, siRPTORb2-M1X, siRPTORb2-M2S, siRPTORb2-M2X, siRPTORb2-M3S, siRPTORb2-M3X, siRPTORb3-M1S, siRPTORb3-M1X, siRPTORb3-M2S, siRPTORb3-M2X, siRPTORb3-M3S, siRPTORb3-M3X, siRPTORb1-T1S, siRPTORb1-T2S, siRPTORc1-M1S,siRPTORc1-M1X,siRPTORc1-M2S, siRPTORc1-M2X, siRPTORc1-M3S, siRPTORc1-M3X, siRPTORc2-M1S, siRPTORc2-M1X, siRPTORc2-M2S, siRPTORc2-M2X, siRPTORc2-M3S, siRPTORc2-M3X, siRPTORc3-M1S, siRPTORc3-M1X, siRPTORc3-M2S, siRPTORc3-M2X, siRPTORc3-M3S, siRPTORc3-M3X, siRPTORc1-T1S, and siRPTORc1-T2S.

**26.** The siRNA according to claim 15, wherein the nucleotide at 5'-terminal of the antisense strand is a 5'-phosphate nucleotide or a 5'-phosphate analogue modified nucleotide.

**27.** The siRNA conjugate according to claim 26, wherein the siRNA is any one of siRPTORa1-M1P1, siRPTORa1-M2P1, siRPTORa1-M3P1, siRPTORa2-M1P1, siRPTORa2-M2P1, siRPTORa2-M3P1, siRPTORa3-M1P1, siRPTORa3-M2P1, siRPTORa3-M3P1, siRPTORa1-M1SP1, siRPTORa1-M2SP1, siRPTORa1-M3SP1, siRPTORa2-M1SP1, siRPTORa2-M2SP1, siRPTORa2-M3SP1, siRPTORa3-M1SP1, siRPTORa3-M2SP1, siRPTORa3-M3 SP 1, siRPTORal-M1XP1, siRPTORal-M2XP1, siRPTORa1-M3XP1, siRPTORa2-M1XP1, siRPTORa2-M2XP1, siRPTORa2-M3XP1, siRPTORa3-M1XP1, siRPTORa3-M2XP1, siRPTORa3-M3XP1, siRPTORb1-M1P1, siRPTORb1-M2P1, siRPTORb1-M3P1, siRPTORb2-M1P1, siRPTORb2-M2P1, siRPTORb2-M3P1, siRPTORb3-M1P1, siRPTORb3-M2P1, siRPTORb3-M3P1, siRPTORb1-M1SP1, siRPTORb1-M2SP1, siRPTORb1-M3SP1, siRPTORb2-M1SP1, siRPTORb2-M2SP1, siRPTORb2-M3SPl, siRPTORb3-M1SP1, siRPTORb3-M2SP1, siRPTORb3-M3SP1, siRPTORb1-M1XP1, siRPTORb1-M2XP1, siRPTORb1-M3XP1, siRPTORb2-M1XP1, siRPTORb2-M2XP1, siRPTORb2-M3XP1, siRPTORb3-M1XP1, siRPTORb3-M2XP1, siRPTORb3-M3XP1, siRPTORc1-M1P1, siRPTORc1-M2P1, siRPTORc1-M3P1, siRPTORc2-M1P1, siRPTORc2-M2P1, siRPTORc2-M3P1, siRPTORc3-M1P1, siRPTORc3-M2P1, siRPTORc3-M3P1, siRPTORc1-M1SP1, siRPTORc1-M2SP1, siRPTORc1-M3SP1, siRPTORc2-M1SP1, siRPTORc2-M2SP1, siRPTORc2-M3SP1, siRPTORc3-M1SP1, siRPTORc3-M2SP1, siRPTORc3-M3 SP 1, siRPTORc1-M1XP1, siRPTORc1-M2XP1, siRPTORc1-M3XP1, siRPTORc2-M1XP1, siRPTORc2-M2XP1, siRPTORc2-M3XP1, siRPTORc3-M1XP1, siRPTORc3-M2XP1, and siRPTORc3-M3XP1.

**28.** A pharmaceutical composition, comprising the siRNA according to any one of claims 1-27 and a pharmaceutically acceptable carrier.

**29.** The pharmaceutical composition according to claim 28, wherein the weight ratio of the siRNA to the pharmaceutically acceptable carrier is 1: (1-500).

**30.** The pharmaceutical composition according to claim 28, wherein the weight ratio of the siRNA to the pharmaceutically acceptable carrier is 1: (1-50).

**31.** A siRNA conjugate, comprising the siRNA according to any one of claims 1-27 and a conjugation group conjugated to the siRNA, wherein the conjugation group comprises at least one pharmaceutically acceptable targeting group and/or delivery auxiliary group;

optionally, the conjugation group further comprises a linker, and the linker and/or the targeting group or the delivery auxiliary group are sequentially linked;
optionally, each of the targeting groups is selected from ligands capable of binding to a cell surface receptor, and/or each of the delivery auxiliary groups is selected from groups capable of increasing biocompatibility of the siRNA conjugate in the target organ or tissue to which it is delivered.

**32.** The siRNA conjugate according to claim 31, wherein the siRNA conjugate has a structure shown in formula (301), wherein Nu has a sequence corresponding to any one of siRPTORa2-M1S, siRPTORb2-M1S, siRPTORc2-M1S, siRPTORc1-T1S, and siRPTORc1-T2S; and the conjugation group is linked to the 3'-position on the ribose ring of the nucleotide at 3' terminal of the sense strand of the siRNA in the Nu, and the siRNA conjugate is in the form of a sodium salt.

**33.** Use of the siRNA according to any one of claims 1-27 and/or the pharmaceutical composition according to any one of claims 28-30 and/or the siRNA conjugate according to claim 31 or 32 in the manufacture of a medicament for inhibiting the expression of *RPTOR* genes in cells.

**34.** Use of the siRNA according to any one of claims 1-27 and/or the pharmaceutical composition according to any one of claims 28-30 and/or the siRNA conjugate according to claim 31 or 32 in the manufacture of a medicament for treating a disease or symptom associated with the regulation of *RPTOR* function.

**35.** The use according to claim 34, wherein the disease or symptom associated with the regulation of *RPTOR* function

is a disease caused by mTORC1 activation or a disease associated with cellular autophagy dysfunction.

**36.** The use according to claim 35, wherein the disease or symptom associated with the regulation of *RPTOR* function is a neurodegenerative disease or a non-alcoholic steatohepatitis.

**37.** The use according to claim 36, wherein the neurodegenerative disease is one or more of Alzheimer's disease, amyotrophic lateral sclerosis, frontotemporal dementia, Lewy body dementia, Huntington's disease, Reich's syndrome, and Parkinson's disease; preferably, the neurodegenerative disease is Alzheimer's disease.

**38.** A method for treating and/or preventing a disease or symptom associated with the regulation of *RPTOR* function, comprising administering an effective amount of the siRNA according to any one of claims 1-27 and/or the pharmaceutical composition according to any one of claims 28-30 and/or the siRNA conjugate according to claim 31 or 32 to a subject in need thereof.

**39.** A method for inhibiting the expression of *RPTOR* gene in cells, comprising contacting an effective amount of the siRNA according to any one of claims 1-27 and/or the pharmaceutical composition according to any one of claims 28-30 and/or the siRNA conjugate according to claim 31 or 32 with the cells.

**40.** A kit, comprising the siRNA according to any one of claims 1-27 and/or the pharmaceutical composition according to any one of claims 28-30 and/or the siRNA conjugate according to claim 31 or 32.

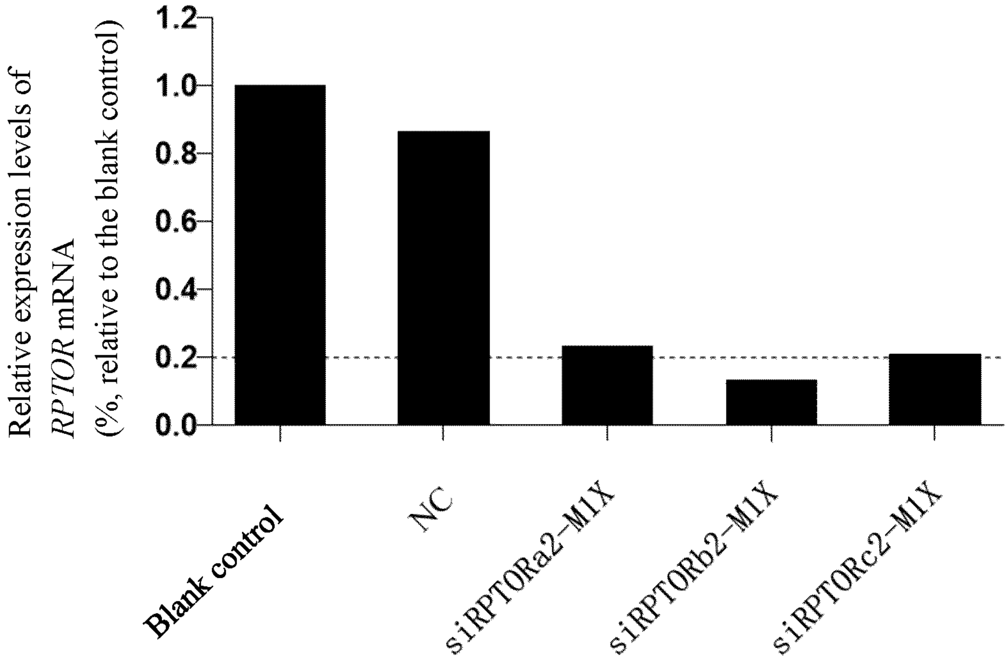


Figure 1

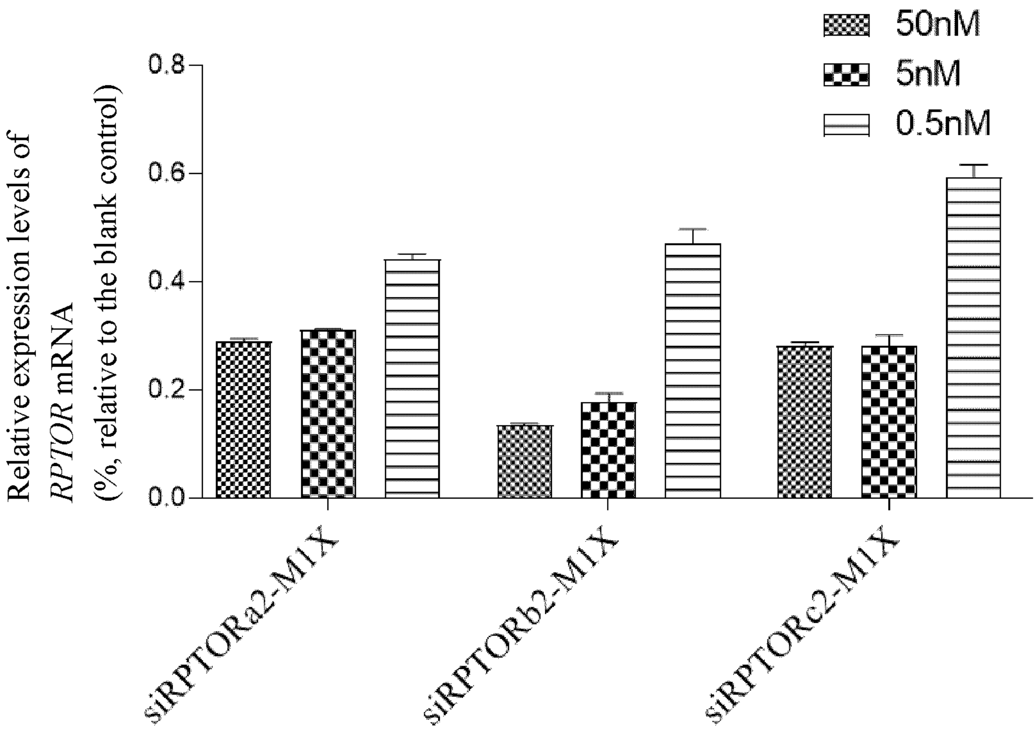


Figure 2

**INTERNATIONAL SEARCH REPORT**

International application No.
**PCT/CN2022/139942**

**A. CLASSIFICATION OF SUBJECT MATTER**

C12N15/113(2010.01)i;A61K31/713(2006.01)i

According to International Patent Classification (IPC) or to both national classification and IPC

**B. FIELDS SEARCHED**

Minimum documentation searched (classification system followed by classification symbols)

C12N A61K

Documentation searched other than minimum documentation to the extent that such documents are included in the fields searched

Electronic data base consulted during the international search (name of data base and, where practicable, search terms used)

CNABS, DWPI, CNTXT, USTXT, EPTXT, WOTXT, JPTXT, CNKI, Web of Science, 万方数据检索系统, WANFANG DATA RETRIEVAL SYSTEM, pubmed, patentics, 中国专利生物序列检索系统, China Patent Biological Sequence Search System, GENBANK, STN: SEQ ID NOs: 1-384, RPTOR, Regulatory Associated Protein Of MTOR Complex 1, regulatory associated protein of mTOR, siRNA, RNAi, shRNA, RNA干扰, 小干扰RNA, mTOR, 哺乳动物雷帕霉素靶蛋白, mammalian target of rapamycin

**C. DOCUMENTS CONSIDERED TO BE RELEVANT**

| Category* | Citation of document, with indication, where appropriate, of the relevant passages | Relevant to claim No. |
|---|---|---|
| A | CN 112423795 A (SUZHOU RIBO LIFE SCIENCE CO., LTD.) 26 February 2021 (2021-02-26)<br>entire document | 1-40 |
| A | US 2019091256 A1 (BETH ISRAEL DEACONESS MEDICAL CENTER et al.) 28 March 2019 (2019-03-28)<br>entire document | 1-40 |
| A | US 2019345497 A1 (CURIGIN CO., LTD.) 14 November 2019 (2019-11-14)<br>entire document | 1-40 |
| A | US 2021311076 A1 (UNIVERSITY OF VIRGINIA PATENT FOUNDATION) 07 October 2021 (2021-10-07)<br>entire document | 1-40 |
| A | WO 2009143371 A2 (INTRADIGM CORPORATION) 26 November 2009 (2009-11-26)<br>entire document | 1-40 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 March 2023** | **10 March 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No.
**PCT/CN2022/139942**

**C. DOCUMENTS CONSIDERED TO BE RELEVANT**

| Category* | Citation of document, with indication, where appropriate, of the relevant passages | Relevant to claim No. |
|---|---|---|
| A | WO 2010064851 A2 (UNIVERSITY OF ULSAN FOUNDATION FOR INDUSTRY COOPERATION) 10 June 2010 (2010-06-10)<br>entire document | 1-40 |
| X | KHOR, E.S. et al. "Endothelial Replicative Senescence Delayed by the Inhibition of Mtorc1 Signaling Involves Microrna-107"<br>*International Journal of Biochemistry and Cell Biology,*<br>Vol. vol. 101, 29 May 2018 (2018-05-29),<br>pp. 64-73 | 1-40 |

Form PCT/ISA/210 (second sheet) (July 2022)

**INTERNATIONAL SEARCH REPORT**

International application No.
**PCT/CN2022/139942**

**Box No. I    Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2022/139942**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **38, 39**
because they relate to subject matter not required to be searched by this Authority, namely:

Claims 38 and 39 respectively relate to a method for treating and/or preventing diseases or symptoms related to RPTOR function regulation, and a method for inhibiting the expression of a RPTOR gene in cells, which both belong to the subject matter for which a search is not required by the International Searching Authority as defined in PCT Rule 39.1(iv): (4) methods for treatment of the human or animal body by surgery or therapy, as well as diagnostic methods practiced on the human or animal body. The international search is provided on the basis of the use thereof in the preparation of corresponding drugs.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/139942**

| Patent document cited in search report | Publication date (day/month/year) | Patent family member(s) | Publication date (day/month/year) |
|---|---|---|---|
| CN 112423795 A | 26 February 2021 | None | |
| US 2019091256 A1 | 28 March 2019 | WO 2017151732 A1 | 08 September 2017 |
| US 2019345497 A1 | 14 November 2019 | EP 3578655 A1 | 11 December 2019 |
| | | EP 3578655 A4 | 23 December 2020 |
| | | CA 3052038 A1 | 09 August 2018 |
| | | AU 2018216509 A1 | 15 August 2019 |
| | | US 11149272 B2 | 19 October 2021 |
| | | US 2021054378 A1 | 25 February 2021 |
| | | WO 2018143626 A1 | 09 August 2018 |
| | | JP 2020509782 A | 02 April 2020 |
| | | JP 6962600 B2 | 05 November 2021 |
| US 2021311076 A1 | 07 October 2021 | WO 2020018461 A1 | 23 January 2020 |
| | | WO 2020018461 A8 | 20 February 2020 |
| WO 2009143371 A2 | 26 November 2009 | WO 2009143371 A3 | 14 January 2010 |
| WO 2010064851 A2 | 10 June 2010 | WO 2010064851 A3 | 07 October 2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2015013253 W
- CN 103380113 A
- CN 110959011 A
- CN 112400018 A
- WO 2019010274 A1
- WO 2019105437 A1


### Non-patent literature cited in the description


- **BEAUCAGE et al.** *Tetrahedron,* 1992, vol. 48, 2223-2311
- **GREENE ; WUTS.** Protective Groups in Organic Synthesis. John Wiley & Sons, 1991
- **J.K. WATTS ; G. F. DELEAVEY ; M. J.DAMHA.** Chemically Modified siRNA: tools and applications. *Drug Discov Today,* 2008, vol. 13 (19-20), 842-55
- **ANASTASIA KHVOROVA ; JONATHAN K. WATTS.** The chemical evolution of oligonucleotide therapies of clinical utility. *Nature Biotechnology,* 2017, vol. 35 (3), 238-48
- **MUTHIAH MANOHARAN.** siRNA conjugates carrying sequentially assembled trivalent N-acetylgalactosamine linked through nucleosides elicit robust gene silencing in vivo in hepatocytes. *ACS Chemical biology,* 2015, vol. 10 (5), 1181-7
- Molecular Cloning. Cold Spring Harbor Laboratory Press, 1989